(19) Europäisches Patentamt
European Patent Office
Office européen des brevets

(11) EP 3 842 540 A1

(12) **EUROPEAN PATENT APPLICATION**
published in accordance with Art. 153(4) EPC

(43) Date of publication:
**30.06.2021 Bulletin 2021/26**

(21) Application number: **19852485.2**

(22) Date of filing: **23.08.2019**

(51) Int Cl.:
***C12Q 1/68*** *(2018.01)* ***G01N 33/50*** *(2006.01)*
***G01N 33/68*** *(2006.01)*

(86) International application number:
**PCT/JP2019/033085**

(87) International publication number:
**WO 2020/040300 (27.02.2020 Gazette 2020/09)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
**KH MA MD TN**

(30) Priority: **24.08.2018 JP 2018157870**

(71) Applicant: **Kyushu University, National University Corporation**
**Nishi-ku**
**Fukuoka-shi**
**Fukuoka 819-0395 (JP)**

(72) Inventors:
- **NAKAYAMA, Keiichi**
  **Fukuoka-shi, Fukuoka 819-0395 (JP)**
- **SHIMIZU, Hideyuki**
  **Fukuoka-shi, Fukuoka 819-0395 (JP)**

(74) Representative: **Vos, Derk**
**Maiwald Patentanwalts- und Rechtsanwaltsgesellschaft mbH**
**Elisenhof**
**Elisenstraße 3**
**80335 München (DE)**

(54) **METHOD FOR PRODUCING CLASSIFIER FOR USE IN PREDICTION OF EVENT OCCURRING IN SUBJECT, AND METHOD FOR STRATIFYING SUBJECTS USING SAID CLASSIFIER**

(57) To stratify test subjects according to an event that will occur in the body of the test subject. Test subjects are stratified according to an event that will occur in the body of the test subject by a discriminator generated by a method for generating a discriminator, including step 1 of determining, for a parent population of biomarkers derived from a test subject, whether each biomarker varies in relation with an event that will occur in the body of the test subject by a statistical method based on a measured value of each biomarker, and extracting a group of biomarkers determined to vary as a first subpopulation, step 2 of validating each biomarker belonging to the first subpopulation, and extracting a group of biomarkers statistically predicted to be related more closely to the event that will occur in the body as a second subpopulation, and step 3 of calculating a weight of each biomarker belonging to the second subpopulation by deep learning, in which the discriminator calculates a weighed sum of scores of biomarkers belonging to the second subpopulation using a score obtained from the measured value of each biomarker belonging to the second subpopulation and the weight of each biomarker calculated in step 3.

[FIG. 18]

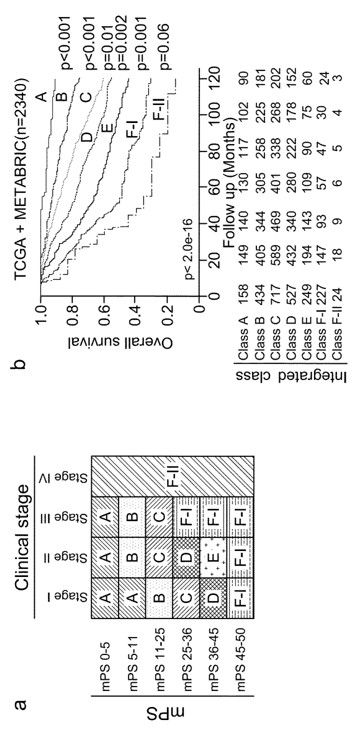


EP 3 842 540 A1

## Description

[Technical Field]

This disclosure relates to a method for generating a discriminator for predicting an event that will occur in a test subject, a method for stratifying test subjects using the discriminator, an apparatus equipped with the discriminator for stratifying test subjects, and a method for predicting a survival rate of a cancer patient.

[Background Art]

Cancer is the leading cause of deaths in developed countries, and methods for stratifying patients with high mortality or patients who benefit from anticancer treatments have been under study for a decade or more.
For example, Patent Document 1 discloses a method for predicting the risk for recurrence of breast cancer within 10 years after surgery from the expression of 95 genes. Non-patent Document 1 discloses a method for predicting the risk for recurrence of invasive (stages I, II, III [up to T3N1]) and estrogen-receptor (ER) positive breast cancer within 10 years after surgery from the expression of 21 genes. The method disclosed in Non-patent Document 1 is provided as an inspection service under the name of Oncotype DX. Non-patent Document 2 discloses a method for predicting the risk for recurrence after 10 years from the expression of 70 genes. The method disclosed in Non-patent Document 2 is provided as an inspection service under the name of MammaPrint. Non-patent Document 3 discloses a method for predicting the risk for distant recurrence after 10 years in young breast cancer patients based on the expression of PAM 50 genes. The method disclosed in Non-patent Document 3 is provided as an inspection service under the name of Prosigna. Non-patent Document 4 discloses a method for selecting ER-positive early-stage breast cancer patients to whom extended endocrine therapy will be applied. The method described in Non-patent Document 4 is provided as an inspection service under the name of Breast Cancer Index. Non-patent Document 5 discloses a method for predicting the risk for distant recurrence of ER-positive and HER2-negative early-stage breast cancer. The method disclosed in Non-patent Document 5 is provided as an inspection service under the name of EndoPredict.

[Related Art Document]

[Patent Document]

[Patent Document 1] Japanese Patent No. 5725274

[Non-Patent Document]

[Non-Patent Document 1] Sparano, J. A. et al. Prospective Validation of a 21-Gene Expression Assay in Breast Cancer. N. Engl. J. Med. 373, 2005-2014, doi:10.1056/NEJMoa1510764 (2015).
[Non-Patent Document 2] van 't Veer, L. J. et al. Nature 415, 530-536, doi:10.1038/415530a (2002).
[Non-Patent Document 3] Parker, J. S. et al. J. Clin. Oncol. 27, 1160-1167, doi:10.1200/jco.2008.18.1370 (2009).
[Non-Patent Document 4] Ma, X. J. et al. Cancer Cell 5, 607-616, doi:10.1016/j.ccr.2004.05.015 (2004).
[Non-Patent Document 5] Filipits, M. et al. Clin. Cancer Res. 17, 6012-6020,doi:10.1158/1078-0432.ccr-11-0926 (2011).

[Summary of the Invention]

[Problems to be Solved by the Invention]

The first question that those who have been notified of cancer ask the doctor is often how many more years they can expect to live.
The methods disclosed in Patent Document 1 and Non-patent Documents 1 to 5 are useful for predicting the risk for recurrence or the therapeutic effects but does not provide an answer to the above patients' question.
Also, the methods disclosed in Patent Document 1 and Non-patent Documents 1 to 5 are applied only to breast cancer that is ER-positive (that is, may benefit from hormone therapies) and has little lymph node metastasis.
In other words, at the present stage, a method for predicting an event that will occur in the body of a test subject such as life or death of the test subject over the entire cancer or entire diseases has not been established.
In the invention disclosed herein, it is an object to provide a method for stratifying test subjects according to an event that will occur in the body of the test subject.

[Means for Solving the Problem]

The present inventors have conducted diligent studies, and consequently found that test subjects can be stratified according to an event that will occur in the body of the test subject using a discriminator generated by a method described later.

The present disclosure includes the following embodiments.

Embodiment 1.

A method for generating a discriminator, comprising:

step 1 of determining, for a parent population of biomarkers derived from a test subject, whether each biomarker varies in relation with an event that will occur in the body of the test subject by a statistical method based on a measured value of each biomarker, and extracting a group of biomarkers determined to vary as a first subpopulation,
step 2 of validating each biomarker belonging to the first subpopulation, and extracting a group of biomarkers statistically predicted to be related more closely to the event that will occur in the body as a second subpopulation, and
step 3 of calculating a weight of each biomarker belonging to the second subpopulation by a deep learning method,

wherein the discriminator calculates a weighed sum of scores of biomarkers belonging to the second subpopulation using a score obtained from the measured value of each biomarker belonging to the second subpopulation and the weight of each biomarker calculated in step 3. Embodiment 2.

A method for generating a discriminator, comprising:

step A of determining, for a parent population of biomarkers derived from a test subject, whether each biomarker varies in relation with an event that will occur in the body of the test subject by a statistical method based on a measured value of each biomarker, and extracting a group of biomarkers determined to vary as a first subpopulation,
step B of validating each biomarker belonging to the first subpopulation, and extracting a group of biomarkers statistically predicted to be related more closely to the event that will occur in the body as a second subpopulation,
step C of extracting a group of biomarkers statistically predicted to be related much more closely to an event that will occur in the body from the biomarkers belonging to the second subpopulation as a third subpopulation by a machine learning method, and
step D of calculating a weight of each biomarker belonging to the third subpopulation by a deep learning method,

wherein the discriminator calculates a weighed sum of scores of biomarkers belonging to the third subpopulation using a score obtained from the measured value of each biomarker belonging to the third subpopulation and the weight of each biomarker calculated in step D. Embodiment 3.

The method for generating a discriminator according to Embodiment 1 or 2, wherein the machine learning method is a random forest.

Embodiment 4.

The method for generating a discriminator according to any one of Embodiments 1 to 3, wherein the deep learning method is a gradient descent method.

Embodiment 5.

The method for generating a discriminator according to any one of Embodiments 1 to 4, wherein the validation is a meta-analysis.

Embodiment 6.

The method for generating a discriminator according to any one of Embodiments 1 to 5, wherein the event that will occur in the body is the survival rate of the patient within or after a predetermined period of time.

Embodiment 7.

The method for generating a discriminator according to Embodiment 6, wherein the disease the patient is suffering from is cancer.

Embodiment 8.

The method for generating a discriminator according to any one of Embodiments 1 to 7, wherein the biomarkers are genes, and the measured value of the biomarker is the expression level of an mRNA or protein derived from the gene.

Embodiment 9.

A discriminator generation apparatus, comprising a processing unit, wherein the processing unit executes a method for generating a discriminator according to any one of Embodiments 1 to 8.

Embodiment 10.

A method of use in which a discriminator generated by a method for generating a discriminator according to any one of Embodiments 1 to 8 is used to predict the survival rate of a patient within or after a predetermined period of time.

Embodiment 11.

The method of use according to Embodiment 10, wherein the patient is a cancer patient. Embodiment 12.

The method of use according to Embodiment 11, wherein the cancer is breast cancer. Embodiment 13.

A method for predicting a survival rate of a patient, comprising the steps of:

acquiring, for a patient, a weighed sum calculated using a discriminator generated by a method for generating a discriminator according to any one of Embodiments 1 to 8, and
determining that the patient has a good survival rate when a weighed sum calculated using a discriminator generated by a method for generating a discriminator according to any one of Embodiments 1 to 8 takes a smaller value as the survival rate within or after a predetermined period of time is better and when the weighed sum of the patient is equal to or smaller than a reference value.

Embodiment 14.

A method for predicting a survival rate of a patient, comprising the steps of:

acquiring, for a patient, a weighed sum calculated using a discriminator generated by a method for generating a discriminator according to any one of Embodiments 1 to 8, and
determining that the patient has a poor survival rate when a weighed sum calculated using a discriminator generated by a method for generating a discriminator according to any one of Embodiments 1 to 8 takes a smaller value as the survival rate within or after a predetermined period of time is better and when the weighed sum of the patient is larger than a reference value. Embodiment 15.

A method for predicting a survival rate of a patient, comprising the steps of:

acquiring, for a patient, a weighed sum calculated using a discriminator generated by a method for generating a discriminator according to any one of Embodiments 1 to 8, and
determining that the patient has a good survival rate when a weighed sum calculated using a discriminator generated by a method for generating a discriminator according to any one of Embodiments 1 to 8 takes a larger value as the survival rate within or after a predetermined period of time is better and when the weighed sum of the patient is equal to or larger than a reference value.

Embodiment 16.

A method for predicting a survival rate of a patient, comprising the steps of:

acquiring, for a patient, a weighed sum calculated using a discriminator generated by a method for generating a discriminator according to any one of Embodiments 1 to 8, and
determining that the patient has a poor survival rate when a weighed sum calculated using a discriminator generated by a method for generating a discriminator according to any one of Embodiments 1 to 8 takes a larger value as the survival rate within or after a predetermined period of time is better and when the weighed sum of the patient is smaller than a reference value. Embodiment 17.

The prediction method according to any one of Embodiments 13 to 16, wherein the patient is a cancer patient.

Embodiment 18.

The prediction method according to Embodiment 17, wherein the cancer is breast cancer. Embodiment 19.
The prediction method according to Embodiment 18, wherein the biomarkers extracted as a second subpopulation when a discriminator is generated include the genes shown in Table 1-1 and Table 1-2 below, and the measured value of the biomarker is the expression level of an mRNA or protein derived from the gene.

Embodiment 20.

The prediction method according to Embodiment 18, wherein the biomarkers that are extracted as a third subpopulation when a discriminator is generated include FOXM1, CPT1A, GARS, MARS, UTP23, ANLN, HMGB3, ATP5B, APOOL, CYB561, GRHL2, ESRP1, EZR, RBBP8, CIRBP, PTGER3, LAMA3, OARD1, ANKRD29, EGR3, DIRAS3, MITD1 and LAMB3, and the measured value of the biomarker is the expression level of an mRNA or protein derived from the gene.

Embodiment 21.

A patient survival rate prediction apparatus comprising a processing unit, wherein the processing unit is for executing a prediction method according to any one of Embodiments 13 to 20.

Embodiment 22.

A method for assisting stratification of patients according to their survival rates, comprising the steps of:

comparing weighed sums calculated for the patients using a discriminator generated by a method for generating a discriminator according to any one of Embodiments 1 to 8 with corresponding reference ranges, and
determining bins of the reference ranges to which the weighed sums of the patients belong.

Embodiment 23.

The method according to Embodiment 22, wherein the patients are cancer patients. Embodiment 24.
The method according to Embodiment 22 or 23, wherein the reference ranges have been determined according to a category such as the clinical stage classification of cancer, the histological type, clinical stage or pathological tissue grade of cancer, or the age group, and
wherein the method further comprises a step of acquiring information on the clinical stage classification of the cancer of the test subject, the histological type, clinical stage or pathological tissue grade of the cancer, the age group of the test subject according to the category. Embodiment 25.
Use of at least one selected from the genes shown in Table 1-1 and Table 1-2 below (with the proviso that an aspect consisting only of ANLN, FOXM1, RBBP8 or a combination of these genes is not included) as a biomarker for predicting a survival rate of a breast cancer patient. Embodiment 26.
Use of at least one selected from the gene group consisting of FOXM1, CPT1A, GARS, MARS, UTP23, ANLN, HMGB3, ATP5B, APOOL, CYB561, GRHL2, ESRP1, EZR, RBBP8, CIRBP, PTGER3, LAMA3, OARD1, ANKRD29, EGR3, DIRAS3, MITD1 and LAMB3 (with the proviso that an aspect consisting only of ANLN, FOXM1, RBBP8 or a combination of these genes is not included) as a biomarker for predicting a survival rate of a breast cancer patient. Embodiment 27.
An inspection reagent for use in predicting a survival rate of a breast cancer patient, comprising a probe, primer or antibody for detecting the expression level of an mRNA or protein derived from at least one selected from the genes shown in Table 1-1 and Table 1-2 below (with the proviso that an aspect consisting only of ANLN, FOXM1, RBBP8 or a combination of these genes is not included).

Embodiment 28.

An inspection reagent for use in predicting a survival rate of a breast cancer patient, comprising a probe, primer, or antibody for detecting the expression level of an mRNA or protein derived from at least one selected from a gene group consisting of FOXM1, CPT1A, GARS, MARS, UTP23, ANLN, HMGB3, ATP5B, APOOL, CYB561, GRHL2, ESRP1, EZR, RBBP8, CIRBP, PTGER3, LAMA3, OARD1, ANKRD29, EGR3, DIRAS3, MITD1 and LAMB3 (with the proviso that an aspect consisting only of ANLN, FOXM1, RBBP8 or a combination of these genes is not included).

Embodiment 29.

A discriminator generated by a method for generating a discriminator according to any one of Embodiments 1 to 8.

Embodiment 30.

A computer readable storage medium in which a discriminator according to Embodiment 29 has been stored.

Embodiment 31.

A computer program for generating a discriminator, which executes a method for generating a discriminator according to any one of Embodiments 1 to 8 when executed by a computer.

Embodiment 32.

A computer program for predicting a survival rate of a patient, which executes a prediction method according to any one of Embodiments 13 to 20 when executed by a computer.

[Effects of the Invention]

It is possible to predict whether an event will occur in the body of a test subject. It is also possible to provide a method for generating a versatile discriminator for predicting whether an event will occur in the body of a test subject based on information from known databases.

[Brief Description of Drawings]

FIG. 1 is a flowchart showing the flow of a method for generating a discriminator.
FIG. 2 is a flowchart showing the flow of step S3 in FIG. 1.
FIG. 3 is a conceptual diagram of a discriminator generation apparatus, a prediction apparatus, and a stratification apparatus.
FIG. 4 is a block diagram of the discriminator generation apparatus, the prediction apparatus, and the stratification apparatus.
FIG. 5 is a flowchart showing the flow of a prediction method.
FIG. 6 is a flowchart showing the flow of a stratification method.
FIG. 7 shows a typical example of a method for generating a discriminator.
FIG. 8a shows Kaplan-Meier Plots for high and low PGK1 expression groups. FIG. 8b shows Kaplan-Meier Plots for high and low TMEM65 expression groups. FIG. 8c shows Kaplan-Meier Plots for high and low BEND5 expression groups. FIG. 8d shows Kaplan-Meier Plots for high and low ENOSF1 expression groups. FIG. 8e shows a result of a meta-analysis.
FIG. 9-1a shows the confidence intervals of genes that lead to a poor survival rate when expressed at a high level.
FIG. 9-1b shows the confidence intervals of genes that lead to a poor survival rate when expressed at a low level.
FIG. 9-2c shows Kaplan-Meier Plots for a $TMEM65^{high}$ and $DCTPP1^{high}$ group and a group of others. FIG. 9-2d shows Kaplan-Meier Plots for a $UBA7^{low}$ and $ENOSF1^{low}$ group and a group of others.
FIG. 10-1 shows the hazard ratios (HR) of the genes shown in Table 1-1 to Table 1-2 and their confidence intervals.
FIG. 10-2 shows the hazard ratios (HR) of the genes shown in Table 1-1 to Table 1-2 and their confidence intervals.
FIG. 10-3 shows the hazard ratios (HR) of the genes shown in Table 1-1 to Table 1-2 and their confidence intervals.
FIG. 10-4 shows the hazard ratios (HR) of the genes shown in Table 1-1 to Table 1-2 and their confidence intervals.
FIG. 11-1a shows Gene_Scores and Gene_Weights of 23 genes.

FIG. 11-2b shows the distribution of mPS in the METABRIC cohort. FIG. 11-2c shows the distribution of mPS in each stage.

FIG. 11-3d shows Kaplan-Meier Plots for every bin of mPS of the METABRIC cohort. FIG. 11-3e shows Kaplan-Meier Plots for every bin of mPS of the TCGA cohort.

FIG. 12 shows an outline of a method for predicting a survival rate according to the present disclosure.

FIG. 13-1a shows Kaplan-Meier Plots for every bin of mPS in HER2-enriched breast cancer patients from the METABRIC cohort. FIG. 13-1b shows Kaplan-Meier Plots for every bin of mPS in Claudin-low breast cancer patients from the METABRIC cohort.

FIG. 13-2c shows Kaplan-Meier Plots for every bin of mPS in Normal-like breast cancer patients from the METABRIC cohort. FIG. 13-2d shows Kaplan-Meier Plots for every bin of mPS in breast cancer patients younger than 50 years old from the METABRIC cohort.

FIG. 13-3e shows Kaplan-Meier Plots for every bin of mPS in ILC breast cancer patients from the METABRIC cohort. FIG. 13-3f shows Kaplan-Meier Plots for every bin of mPS in grade 2 breast cancer patients from the METABRIC cohort·(n=740).

FIG. 14a shows Kaplan-Meier Plots for every bin of mPS in breast cancer patients in their 50s and 60s from the METABRIC cohort. FIG. 14b shows Kaplan-Meier Plots for every bin of mPS in breast cancer patients not younger than 70 years old from the METABRIC cohort. FIG. 14c shows Kaplan-Meier Plots for every bin of mPS in breast cancer patients younger than 50 years old from the METABRIC cohort.

FIG. 15-1a shows Kaplan-Meier Plots for every bin of mPS in IDC breast cancer patients from the METABRIC cohort. FIG. 15-1b is a Kaplan-Meier Plot for every bin of mPS in MDLC breast cancer patients from the METABRIC cohort.

FIG. 15-2c shows Kaplan-Meier Plots for every bin of mPS in grade 1 breast cancer patients from the METABRIC cohort. FIG. 15-2d shows Kaplan-Meier Plots for every bin of mPS in grade 3 breast cancer patients from the METABRIC cohort.

FIG. 16-1a shows Kaplan-Meier Plot for Kaplan-Meier Plots for every bin of mPS in breast cancer patients categorized into the Nottingham Prognostic Index (NPI) Excellent group from the METABRIC cohort. FIG. 16-1b shows Kaplan-Meier Plots for every bin of mPS in breast cancer patients categorized into the Nottingham Prognostic Index (NPI) Good group from the METABRIC cohort.

FIG. 16-2c shows Kaplan-Meier Plots for every bin of mPS in breast cancer patients categorized into the Nottingham Prognostic Index (NPI) Moderate group from the METABRIC cohort. FIG. 16-2d shows Kaplan-Meier Plots for every bin of mPS in breast cancer patients categorized into the Nottingham Prognostic Index (NPI) Poor group from the METABRIC cohort.

FIG. 17-1a shows Kaplan-Meier Plots for every bin of mPS in breast cancer patients categorized into Stage I from the METABRIC cohort. FIG. 17-1b is Kaplan-Meier Plots for every bin of mPS in breast cancer patients categorized into Stage II from the METABRIC cohort.

FIG. 17-2c shows Kaplan-Meier Plots for every bin of mPS in breast cancer patients categorized into Stage III from the METABRIC cohort. FIG. 17-2d shows Kaplan-Meier Plots for every bin of mPS in breast cancer patients categorized into Stage II from the TCGA cohort.

FIG. 17-3e shows Kaplan-Meier Plots for every bin of mPS in breast cancer patients categorized into Stages I to III from the TCGA cohort. FIG. 17-3f shows Kaplan-Meier Plots for every bin of mPS in breast cancer patients categorized into Moderate II from the METABRIC cohort.

FIG. 18a is a diagram showing the relationship of classes in which clinical stages are integrated with mPS. FIG. 18b shows Kaplan-Meier Plots for every bin of classes in which clinical stages are integrated with mPS for the entire METABRIC and TCGA cohorts.

[Detailed Description of The Invention]

1. Method for generating discriminator

A discriminator is generated based on measured values of multiple biomarkers. First, for a parent population of biomarkers, multiple biomarkers that may vary in relation with an event that will occur in the body of a test subject are determined based on measured values of the biomarkers. The discriminator is generated from a score that is determined based on a measured value of each biomarker and a weight of each biomarker that is calculated by a statistical method for multiple extracted biomarkers.

More specifically, the discriminator is generated when a processing unit 101 of a discriminator generation apparatus, which is described later, executes each step shown in FIG. 1, for example.

First, in step S1, upon receiving a user's instruction to start processing from an input unit 111, the processing unit 101 extracts a group of biomarkers that vary in relation with an event that will occur in the body of a test subject from a parent population of biomarkers as a first subpopulation.

Next, in step S2, the processing unit 101 validates each biomarker belonging to the first subpopulation to extract a second subpopulation that is predicted to be related more closely to an event that will occur in the body.

Next, in step S3, the processing unit 101 extracts a third subpopulation that is predicted to be related much more closely to an event that will occur in the body from the biomarkers belonging to the second subpopulation by a machine learning method.

Next, in step S4, the processing unit 101 calculates a weight of each biomarker belonging to the third subpopulation by a deep learning method. The processing unit 101 generates a discriminator as described below from the weights calculated in step S4 and scores determined based on the measured value of each biomarker belonging to the third subpopulation;

[Mathematical Formula 1]

$$mPS = \sum_{i=1}^{n} (w_i * GS_i)$$

[wherein mPS indicates a molecular prognostic score, w indicates a weight of each biomarker, GS indicates a score of each biomarker, i indicates each biomarker, and n represents the total number of biomarkers. wi*Si represents the product of the weight of each biomarker and the score of the corresponding biomarker].

Finally, in step S5, the processing unit 101 may validate the generalization/versatile ability, application potentiality and/or the like of the generated discriminator.

Each step is described in more detail.

A typical example of each step is shown in FIG. 7, but the present disclosure should not be construed as being limited to FIG. 7.

(1) Step 1

Step 1 is a step of determining a group of biomarkers that may vary in relation with an event that will occur in the body of a test subject.

The test subject is not limited as long as it is a mammal. Examples include humans, monkeys, dogs, cats, mice, rats, rabbits and so on. Preferably, the test subject is a human. Preferably, the test subject is a test subject suffering from a disease or diagnosed as suffering from a disease. Preferably, the test subject is a patient.

The disease is not particularly limited. Preferably, the disease is a malignant tumor. More preferably, the disease is a malignant epithelial tumor (cancer). Examples of the malignant tumor include solid tumors including respiratory system malignant tumors arising from the trachea, bronchi, lung or the like; digestive system malignant tumors arising from the epipharynx, esophagus, stomach, duodenum, jejunum, ileum, cecum, appendix, ascending colon, transverse colon, sigmoid colon, rectum, anal region or the like; liver cancer; pancreatic cancer; urinary system malignant tumors arising from the bladder, ureter or kidney; female reproductive system malignant tumors arising from the ovary, oviduct, uterus and so on; breast cancer; prostatic cancer; skin cancer; malignant tumors in the endocrine system such as hypothalamus, pituitary gland, thyroid gland, parathyroid gland, adrenal gland or the like; central nervous system malignant tumors; malignant tumors arising from the bone-soft tissue, and so on. More preferred examples include epithelial malignant tumors in the respiratory system such as lung cancer (squamous cancer, small cell cancer, large cell cancer, adenocarcinoma) or the like; epithelial malignant tumors in the digestive system such as stomach cancer, duodenum cancer, large bowel cancer (sigmoid colon cancer, rectum cancer, etc.) or the like; liver cancer; pancreatic cancer; bladder cancer; thyroid gland cancer; ovary cancer; breast cancer; and prostate cancer. Most preferably, the disease is breast cancer.

When the disease is a malignant tumor, it is preferred that no metastasis of the malignant tumor is observed in the test subject.

The event that will occur in the body of a test subject is not limited. Examples include an onset of a disease (including, for example, an initial onset and recurrence), a biological response to treatment of a disease, the consequence of a disease (including, for example, cure and remission), the consequences of the test subject itself (including, for example, death and survival) and so on. Preferably, the event is an onset of a disease, the consequence of a disease, the consequences of the test subject itself, and so on. More preferably, the event is the consequence of the test subject itself within or after a predetermined period of time, and still more preferably the death or survival of the test subject itself after a predetermined period of time.

An event that will occur in the body of a test subject can be evaluated by whether or not the event will occur or by its probability. Examples of the probability include the rate of incidence of the disease, the rate at which the disease becomes severe, the treatment success rate for the disease, the treatment failure rate for the disease, the cure rate of the disease, the remission rate of the disease, the survival rate of the test subject, the mortality rate of the test subject and so on. Categorizing test subjects according to whether or not an event will occur or the probability of the event occurring is also referred to as stratifying the test subjects.

The predetermined period of time can be selected as appropriate from 1 year, 2 years, 3 years, 5 years, 6 years, 7 years, 8 years, 9 years, 10 years, 15 years, 20 years, and 30 years depending on the event that will occur in the body.

In this disclosure, the most typical example of the event that will occur in the body of a test subject is the survival of a breast cancer patient after 10 or 20 years.

The biomarkers are not limited as long as they are substances in the body. The substances in the body are at least one metabolic substance selected from the group consisting of nucleic acids (including genes); carbohydrates; lipids; glycoproteins; glycolipids; lipoproteins; amino acids, peptides; proteins; polyphenols; chemokines; and ultimate metabolites, intermediate metabolites and synthetic raw materials of the above substances; metal ions or the like, and more preferably are genes.

The measured values of biomarkers are not limited as long as they can be measured by a known method and are values that reflect the amounts or concentrations of the above biomarkers. The measured values may be represented as quantitative values, or may be represented in a seme-quantitative way such as "increase," "no change" or "decrease." For example, when the biomarkers are genes and values that reflect the abundance of proteins derived from the genes are measured, an immunological measurement method (such as an ELISA method, a western blotting method or the like) can be employed. When the biomarkers are genes and values that reflect the expression levels (such as the number of copies) of mRNAs derived from the genes are measured, a quantitative RT-PCR method, an RNA-Seq method or the like can be employed.

For example, when genes are used as the biomarkers, values that reflect the abundance of proteins or expression levels (such as the number of copies) of mRNAs derived from the genes can be used as the measured values of biomarkers. In FIG. 7, the parent population of biomarkers includes all the coding genes registered in HUGO Gene Nomenclature Committee (HGNC).

The measured values of biomarkers may be measured values in a specimen containing tissue or cells at the lesion site of the disease. Also, the measured values may be measured values in a specimen containing substances in the body that reflect the state of the disease such as blood samples (including whole blood, plasma, serum and so on), urine, cerebrospinal fluid, ascites, pleural effusion and so on. When the disease is cancer, the specimen is preferably cancer tissue or cancer cells.

As the measured values of biomarkers, information registered in a known database may be used. Examples of the known database include cBioPortal [Cerami, E. et al. Cancer Discov. 2, 401-404, doi:10.1158/2159-8290.cd-12-0095 (2012)], multicenter combined breast cancer cohorts [Abdel-Fatah, T. M. A. et al. The Lancet. Oncology 17, 1004-1018, doi:10.1016/s1470-2045(16)00174-1 (2016)], Whole METABRIC cohort [n=1904; Pereira, B. et al. Nature communications 7, 11479, doi:10.1038/ncomms11479 (2016) and Curtis, C. et al. Nature 486, 346-352, doi:10.1038/nature10983 (2012)] and so on. In the example shown in FIG. 7, the mRNA expression (RNA-seq) in the TCGA breast cancer dataset from cBioPortal is used as a first cohort for determining a group of biomarkers the measured values of which vary in relation with an event that will occur in the body of a test subject (also referred to as "discovery cohort").

Whether or not the measured value of each biomarker varies in relation with an event that will occur in the body of a test subject can be determined by a statistical method.

The statistical method is not limited as long as it can determine whether or not each biomarker is related to an event of interest. For example, each biomarker is classified into a high measured value or a low measured value group, and the relation with whether or not an event occurred in the body is evaluated for each biomarker belonging to each group. The relation with an event that will occur in the body is validated by a significant difference test, and biomarkers determined to have a "difference" by a significance test can be determined as candidate biomarkers that are related to an event that will occur in the body.

For each biomarker, whether its measured value is high or low can be determined on the basis of, for example, the median, average, interquartile range and so on. In the typical example in FIG. 7, with regard to the determination of whether the measured value of each biomarker is high or low, the median of measured values of the biomarkers in a population is used as a boundary, and the measured values are divided into two groups by determining measured values equal to or higher than the median as "high" and measured values lower than the median as "low." A median is characterized by the fact that no large difference is created between the numbers of biomarkers belonging to each group because the number of biomarkers belonging to each group after grouping is substantially the same. For this reason, it is preferred to use a median for grouping. Such a statistical analysis can be made using known statistical analysis software (for example, statistical analysis free software "R") or the like.

The significant difference test can be selected according to information that represents the probability of variations occurring depending on a target event for each biomarker. For example, when the event that will occur in the body is one represented by a survival rate, the information that represents the probability of variations occurring for each biomarker is a Kaplan-Meier Plot.

Because step S1 is intended to reduce the candidate biomarkers, a cutoff value is set for a significance level, p-value, calculated in a significant difference test. Biomarkers with a p-value smaller than the cutoff value can be determined as biomarkers "having a difference," and biomarkers with a p-value larger than the cutoff value can be separated as biomarkers "having no difference."

The significant difference test can be selected, depending on the distribution of the measured values of biomarkers, from, for example, a t-test, a chi-square test, a one-way analysis of variance (which may include a test such as a Kruskal-Wallis test, a Mann-Whitney test or the like), a Friedman's test, a Cochran's Q test, a Log-rank test and so on.

The cutoff value can be selected, for example, from 0.05, 0.01, 0.005, 0.001 and so on.

When the information that represents the probability for each biomarker is a Kaplan-Meier Plot, a Log-rank test, for example, can be employed as a method for the significant difference test. In the typical example shown in FIG. 7, the cutoff value is set to 0.01. In other words, genes determined to have a p-value of less than 0.01 in a Log-rank test were extracted as biomarkers belonging to a first subpopulation related to an event that will occur in the body.

Kaplan-Meier plots can be generated using statistical analysis software "R (survival package)" or the like. A Log-rank test can be conducted using the survival package, Python (lifelines package) of R or the like.

In this way, the processing unit 101 can determine the group of biomarkers determined to "have a difference" by a significant difference test as a first subpopulation.

(2) Step 2

In step S2, the processing unit 101 uses a validation cohort for the first subpopulation, which has determined to "have a difference" by a significant difference test in step S1, to validate the correctness as candidate biomarkers. The validation is performed exhaustively on all the biomarkers extracted as the first subpopulation, and biomarkers determined to be correct as candidate biomarkers are extracted as a second subpopulation.

The validation cohort is preferably selected from a cohort different from the discovery cohort. When there is only one cohort, the cohort may be divided into two groups and one of the groups may be used as a discovery cohort and the other as a validation cohort. To improve the generalization/versatile ability and/or application potentiality of the discriminator that will be finally generated, the validation cohort is preferably selected from a cohort different from the discovery cohort.

The validation method is not limited as long as the correctness as candidate biomarkers can be validated. As a validation method, a meta-analysis, a Log-rank test or the like can be employed. Preferably, a meta-analysis is performed using a validation cohort different from the discovery cohort. The meta-analysis performs validation from hazard ratios and 95% confidence intervals by a Cox regression analysis. These analyses can be made using software such as Python (3.6.2).

In the typical example shown in FIG. 7, multicenter combined breast cancer cohorts [Abdel-Fatah, T. M. A. et al. The Lancet. Oncology 17, 1004-1018, doi:10.1016/s1470-2045(16)00174-1 (2016)], which are different from the discovery cohort, are used as a validation cohort to perform validation by a meta-analysis.

In this way, the processing unit 101 can extract biomarkers validated to be correct as candidate biomarkers as a second subpopulation that is related more closely to an event that will occur in the body.

The second subpopulation of the typical example shown in FIG. 7 is as shown in Table 1-1 and Table 1-2.

[Table 1-1]

| | Gene Name | NCBI* Gene ID | | Gene Name | NCBI* Gene ID | | Gene Name | NCBI* Gene ID |
|---|---|---|---|---|---|---|---|---|
| 1 | ADHFE1 | 137872 | 41 | DCAF16 | 54876 | 81 | LEF1 | 51176 |
| 2 | AMD1 | 262 | 42 | DCTPP1 | 79077 | 82 | LEPROTL1 | 23484 |
| ***3*** | ***ANKRD29*** | ***147463*** | 43 | DEF6 | 50619 | 83 | LRP11 | 84918 |
| ***4*** | ***ANLN*** | ***54443*** | 44 | DERL1 | 79139 | 84 | LTF | 4057 |
| 5 | AP5S1 | 55317 | 45 | DHRS1 | 115817 | 85 | MAL2 | 114569 |
| 6 | APOBEC3F | 200316 | ***46*** | ***DIRAS3*** | ***9077*** | 86 | MAP4K1 | 11184 |
| 7 | APOBEC3G | 60489 | 47 | EEF1G | 1937 | 87 | MAPT | 4137 |
| 8 | APOL3 | 80833 | ***48*** | ***EGR3*** | ***1960*** | ***88*** | ***MARS*** | ***4141*** |

(continued)

| | Gene Name | NCBI* Gene ID | | Gene Name | NCBI* Gene ID | | Gene Name | NCBI* Gene ID |
|---|---|---|---|---|---|---|---|---|
| ***9*** | ***APOOL*** | ***139322*** | 49 | ENOSF1 | 55556 | 89 | MAX | 4149 |
| 10 | ARHGAP26 | 23092 | ***50*** | ***ESRP1*** | ***54845*** | ***90*** | ***MITD1*** | ***129531*** |
| 11 | ARMC1 | 55156 | 51 | EVA1C | 59271 | 91 | MMGT1 | 93380 |
| *12* | ***ATP5F1B**** | ***506*** | ***52*** | ***EZR*** | ***7430*** | 92 | MRPL13 | 28998 |
| 13 | ATP6V1C1 | 528 | 53 | FAXDC2 | 10826 | 93 | MTDH | 92140 |
| 14 | BEND5 | 79656 | 54 | FKBP5 | 2289 | 94 | MTFR1 | 9650 |
| 15 | BTF3 | 689 | ***55*** | ***FOXM1*** | ***2305*** | 95 | NDUFAF6 | 137682 |
| 16 | BTG1 | 694 | ***56*** | ***GARS*** | ***2617*** | 96 | NEFH | 4744 |
| 17 | C1RL | 51279 | ***57*** | ***GRHL2*** | ***79977*** | 97 | NFKBIA | 4792 |
| 18 | CARD10 | 29775 | 58 | GSTM4 | 2948 | 98 | NKAP | 79576 |
| 19 | CASP9 | 842 | 59 | GSTO1 | 9446 | 99 | NONO | 4841 |
| 20 | CCDC115 | 84317 | 60 | GTF2IRD2B | 389524 | 100 | NR1H3 | 10062 |
| 21 | CCDC24 | 149473 | 61 | H6PD | 9563 | 101 | NSA2 | 10412 |
| 22 | CCDC69 | 26112 | 62 | HCCS | 3052 | 102 | NSMCE2 | 286053 |
| 23 | CCNE1 | 898 | 63 | HILPDA | 29923 | ***103*** | ***OARD1*** | ***221443*** |
| 24 | CCT2 | 10576 | ***64*** | ***HMGB3*** | ***3149*** | 104 | PARG | 8505 |
| 25 | CCT4 | 10575 | 65 | HNRNPA1L2 | 144983 | 105 | PARP3 | 10039 |
| 26 | CCT6A | 908 | 66 | HSP90AA1 | 3320 | 106 | PCMT1 | 5110 |
| 27 | CD24 | 100133941 | 67 | HSPA2 | 3306 | 107 | PDCD2 | 5134 |
| 28 | CD48 | 962 | 68 | IARS | 3376 | 108 | PGK1 | 5230 |
| 29 | CD52 | 1043 | 69 | IGF2R | 3482 | 109 | PHF7 | 51533 |
| 30 | CD6 | 923 | 70 | IP6K2 | 51447 | 110 | PLAT | 5327 |
| 31 | CD74 | 972 | 71 | IRF2 | 3660 | 111 | PLEKHA4 | 57664 |
| 32 | CDK8 | 1024 | 72 | ITGA10 | 8515 | 112 | PLXNB3 | 5365 |
| 33 | CIR1 | 9541 | 73 | JAK1 | 3716 | 113 | PNRC2 | 55629 |
| ***34*** | ***CIRBP*** | ***1153*** | 74 | KANSL2 | 54934 | 114 | POP1 | 10940 |
| 35 | COG8 | 84342 | 75 | KDM4B | 23030 | 115 | PRKAB2 | 5565 |
| 36 | COPS6 | 10980 | 76 | KIF21A | 55605 | 116 | PSMD10 | 5716 |
| ***37*** | ***CPT1A*** | ***1374*** | 77 | KRCC1 | 51315 | 117 | PSME1 | 5720 |
| 38 | CRK | 1398 | ***78*** | ***LAMA3*** | ***3909*** | ***118*** | ***PTGER3*** | ***5733*** |
| ***39*** | ***CYB561*** | ***1534*** | ***79*** | ***LAMB3*** | ***3914*** | 119 | PTK2 | 5747 |
| 40 | CYP27A1 | 1593 | 80 | LARP4B | 23185 | 120 | PYCARD | 29108 |

*NCBI : National Center for Biotechnology Information
**The genes shown in bold italic letters belong to a third subpopulation.
***ATP5F1B is also referred to as ATP5B.

[Table 1-2]

| | Gene Name | NCBI* Gene ID | | Gene Name | NCBI* Gene ID |
|---|---|---|---|---|---|
| 121 | QPRT | 23475 | 161 | TPRG1 | 285386 |
| 122 | RAB2A | 5862 | 162 | TPT1 | 7178 |
| 123 | RAC2 | 5880 | 163 | TRAF5 | 7188 |
| 124 | RAD1 | 5810 | 164 | TRAPPC12 | 51112 |
| 125 | RAPGEFL1 | 51195 | 165 | TRIM34 | 53840 |
| 126 | RARRES3 | 5920 | 166 | TRIM35 | 23087 |
| ***127*** | ***RBBP8*** | ***5932*** | 167 | TRIM45 | 80263 |
| 128 | REPS1 | 85021 | 168 | TVP23C | 201158 |
| 129 | RILPL2 | 196383 | 169 | UBA7 | 7318 |
| 130 | RPF1 | 80135 | 170 | UBE2A | 7319 |
| 131 | RPL11 | 6135 | 171 | UBTF | 7343 |
| 132 | RPL14 | 9045 | 172 | UBXN1 | 51035 |
| 133 | RPS6KA1 | 6195 | 173 | UBXN11 | 91544 |
| 134 | RTN4IP1 | 84816 | ***174*** | ***UTP23*** | ***84294*** |
| 135 | SAV1 | 60485 | 175 | VBP1 | 7411 |
| 136 | SCUBE2 | 57758 | 176 | WLS | 79971 |
| 137 | SERPINA1 | 5265 | 177 | YTHDF3 | 253943 |
| 138 | SH3D21 | 79729 | 178 | ZBED6CL | 113763 |
| 139 | SHISA5 | 51246 | 179 | ZDHHC9 | 51114 |
| 140 | SKP1 | 6500 | 180 | ZFHX3 | 463 |
| 141 | SLC35A2 | 7355 | 181 | ZMYND10 | 51364 |
| 142 | SLC7A5 | 8140 | 182 | ZNF496 | 84838 |
| 143 | SPATA7 | 55812 | 183 | ZNF552 | 79818 |
| 144 | SPOCK2 | 9806 | 184 | ZNF671 | 79891 |
| 145 | STC2 | 8614 | | | |
| 146 | STEAP2 | 261729 | | | |
| 147 | STK3 | 6788 | | | |
| 148 | STK39 | 27347 | | | |
| 149 | STRAP | 11171 | | | |
| 150 | SURF4 | 6836 | | | |
| 151 | SUSD3 | 203328 | | | |
| 152 | TAPBPL | 55080 | | | |
| 153 | TARS | 6897 | | | |
| 154 | TBC1D10C | 374403 | | | |
| 155 | THG1L | 54974 | | | |
| 156 | TMEM229B | 161145 | | | |
| 157 | TMEM65 | 157378 | | | |

(continued)

| | Gene Name | NCBI* Gene ID | | Gene Name | NCBI* Gene ID |
|---|---|---|---|---|---|
| 158 | TMEM69 | 51249 | | | |
| 159 | TNFRSF14 | 8764 | | | |
| 160 | TNIP1 | 10318 | | | |
| *NCBI : National Center for Biotechnology Information<br>**The genes shown in bold italic letters belong to a third subpopulation. | | | | | |

(3) Step 3

The processing unit 101 extracts a third subpopulation from the second subpopulation by machine learning and validation. The machine learning method is not limited as long as feature values can be extracted. Examples of the machine learning method include a random forest, a decision tree, a support vector regression, a support vector machine, a logistic analysis, a sparse logistic analysis, a deep learning and so on. Preferred is a random forest. The machine learning can be performed using a Python-based scikit-learn library or the like.

As shown in FIG. 2, by extracting feature values from training data, for example, for the biomarkers belonging to the second subpopulation extracted in step S2 of FIG. 1, the machine learning method extracts, according to the feature values, biomarkers that are related more closely to an event that will occur in the body from the second subpopulation as feature values (step S31). The feature values are values that represent the intensity of relation between the variation of the measured value of each biomarker and an event that will occur in the body. For example, when the machine learning is a random forest, feature values are extracted based on feature importance values calculated in the validation. In other words, by extracting a plurality of biomarkers in the descending order of the feature importance values, biomarkers that are related more closely to an event that will occur in the body can be extracted. Here, the plurality is not limited as long as it is two or more. For example, the plurality is intended to mean 3, 5, 10, 15, 20, 23, 25, 30, 40, 50, 100 or the like.

The training data may be the cohort used as a discovery cohort or the cohort used as a validation cohort in step S2, or may be a different cohort. To improve the generalization/versatile ability and/or application potentiality of the discriminator that will be finally generated, it is preferred to use a cohort different from the discovery cohort or the validation cohort used in step S2 as the training data.

In the typical example shown in FIG. 7, the Whole METABRIC cohort [Pereira, B. et al. Nature communications 7, 11479, doi:10.1038/ncomms11479 (2016) and Curtis, C. et al. Nature 486, 346-352, doi:10.1038/nature10983 (2012)] is used as the training data.

The cohort used for validation is preferably selected from a cohort different from the training data. When there is only one cohort, the cohort may be divided into two groups and one of the groups may be used as the training data and the other as a cohort for validation. Selection may be made from different cohorts. Preferably, the cohort used for validation is selected from a cohort different from the training data.

Next, in step S32, as shown in FIG. 2, the processing unit 101 performs validation on the feature values extracted in step S31. The validation is repeated until feature values that are related most closely to an event that will occur in the body are found (step S33). In this way, feature values (biomarker group) that are related much more closely to an event that will occur in the body are extracted from the second subpopulation (step S34).

When a cohort selected from the same cohort as the training data is used for validation, a hold-out method, a cross-validation such as a leave-one-out cross-validation (LOOCV), a mixing matrix and so on can be employed as the validation method. Preferred is a cross-validation. As a cross-validation, for example, a 10-fold cross-validation or the like can be employed. These analyses can be conducted using Python (3.6.2) or the like.

In the typical example shown in FIG. 7, a 10-fold cross-validation is used for validation.

In this way, the processing unit 101 can extract a third subpopulation that is related more closely to an event that will occur in the body.

The genes extracted in the typical example shown in FIG. 7 are 23 genes shown in FIG. 11-1a.

(4) Step 4

In step 4, for the biomarkers belonging to the third subpopulation, the training data are input into a neural network to calculate a weight of each biomarker belonging to the third subpopulation (which may be represented by "Weight" or "$w_i$," wherein i represents each biomarker) by a deep learning method.

The deep learning method is not limited as long as a weight of each biomarker can be calculated. Examples include a gradient descent method, a stochastic gradient method, a feedforward neural network, an autoencoder, an error propagation method, a convolutional neural network, a recurrent neural network, a Boltzmann machine and so on. Preferred is a gradient descent method. The deep learning can be performed using a Python-based TensorFlow library or the like.

In the typical example shown in FIG. 7, a loss function by cross entropy of each gene is defined, and a weight "Gene_Weight" of each gene is calculated by a gradient descent method [Kourou, K., et al. Computational and structural biotechnology journal 13, 8-17,doi:10.1016/j.csbj.2014.11.005 (2015)].

The training data used in step 4 may be the same as or different from the training data used in step 3. Also, the training data used in step 4 may be the cohort used as a discovery cohort or the cohort used as a validation cohort in step S2.

As shown in Mathematical Formula 1, the discriminator is a function for multiplying the score of each biomarker belonging to the third subpopulation by the weight calculated by the deep learning and obtaining the sum of the products.

The score of each biomarker is a value that links the measured value of each biomarker belonging to the third subpopulation with an event that will occur in the body. The value to be linked is, for example, binary data such as "0" and "1." For example, when an event that will occur in the body is represented by a survival rate, for biomarkers that lead to a possibility that the prognosis will be poor when their measured values are higher than the median, "1" is given as a "Gene_Score" when the measured value of each biomarker is higher than the median and "0" is given as a "Gene_Score" when the measured value of each biomarker is lower than the median. For biomarkers that lead to a possibility that the prognosis will be poor when their measured values are lower than the median, "1" is given as a "Gene_Score" when the measured value of each biomarker is lower than the median, and "0" is given as a "Gene_Score" when the measured value of each biomarker is higher than the median. This aspect is hereinafter referred to as "score pattern 1."

By inputting these scores into the discriminator, a weighed sum can be calculated. The weighed sum using the discriminator is referred to as molecular prognostic score (mPS).

In the typical example shown in FIG. 7, the discriminator is the weighed sum of "Gene_Scores" of 23 genes shown in FIG. 11-1a in the breast cancer tissue of each patient. In other words, the discriminator is represented by the following formula.

[Mathematical Formula 2]

$$mPS = \sum_{i=1}^{n} (w_i * GS_i)$$

(wherein w represents a "Gene_Weight," GS indicates a "Gene_Score," n is 23, i indicates each biomarker, and wi*GSi indicates the product of the weight and score of each biomarker. 23 indicates the 23 genes shown in FIG. 11-1a.).

When scores based on the score pattern 1 are used, the mPS value exhibits a smaller value as the survival rate is better. Also, when scores based on the score pattern 1 are used, the mPS value exhibits a larger value as the survival rate is poorer.

Also, as another aspect, when the event that will occur in the body is represented by, for example, a survival rate, for biomarkers that lead to a possibility that the prognosis will be poor when their measured values are higher than the median, "0" is given as a "Gene_Score" when the measured value of each biomarker is higher than the median and "1" is given as a "Gene_Score" when the measured value of each biomarker is lower than the median. For biomarkers that lead to a possibility that the prognosis will be poor when their measured values are lower than the median, "0" is given as a "Gene_Score" when the measured value of each biomarker is lower than the median and "1" is given as a "Gene_Score" when the measured value of each biomarker is higher than the median. This aspect is hereinafter referred to as "score pattern 2."

When scores based on the score pattern 2 are used, the mPS value exhibits a larger value as the survival rate is better. Also, when scores based on the score pattern 2 are used, the mPS value exhibits a smaller value as the survival rate is poorer.

Here, the discriminator may be generated by performing step S4 on the measured values of the biomarkers in the second subpopulation without performing step S3 shown in FIG. 1.

The generated discriminator may be stored in a storage unit of each apparatus described later, or may be output from each output unit. Also, it may be stored in a storage medium described later, or may be network-transmitted via a communication I/F 105.

(5) Step 5

Step 5 is an optional step. Step 5 is a step of validating whether the discriminator generated in step 4 is applicable to any cohort divided into groups according to the type of the cohort, the subtypes of the disease, the age of the test subjects or the like.

The subtypes of the disease are, for example, clinical stage classification of cancer (A, B, C, D, E, F-I, and F-II shown in FIG. 18a), clinical stages (TNM classification for cancer; stages I, II, III, etc. for breast cancer), histopathological grades (poorly differentiated type, moderately-differentiated type, well-differentiated type, etc.), or histological types (invasive ductal carcinoma (IDC), invasive lobular carcinoma (ILC), mixed IDC and ILC (MDLC), etc. for breast cancer).

Grouping according to age or the like can divide the test subjects into, for example, a young group (younger than 50 years old), a mature group (not younger than 50 years old and younger than 70 years old) and a senior group (not younger than 70 years old).

The generalization/versatile ability, the application potentiality and/or the like of the discriminator can be validated by statistically validating the correlation between the measured value of each biomarker and an event that will occur in the body for each cohort divided into groups. The method for obtaining a correlation is known.

The generated discriminator may be stored in a storage medium such as a hard disk, a semiconductor memory device like a flash memory, an optical disc or the like. The format in which a program is stored in the storage medium is not limited as long as the processing unit 101, a processing unit 201 or a processing unit 301 can read the program. The program is preferably stored in the storage medium in a non-volatile manner.

The generated discriminator can be used to evaluate an event that will occur in the body of a test subject.

2. Apparatus for generating discriminator

This disclosure relates to an apparatus 10 for generating a discriminator (also referred to as "discriminator generation apparatus 10"). The discriminator generation apparatus 10 includes at least the processing unit 101, and the processing unit 101 generates a discriminator mentioned in the above section 1 according to the steps described in the above section 1., FIG. 1 or FIG. 2.

FIG. 3 shows the hardware configuration of the discriminator generation apparatus 10. FIG. 4 shows a block diagram of the discriminator generation apparatus 10. The discriminator generation apparatus 10 may be connected to an input unit 111, an output unit 112 and a storage medium 113.

In the discriminator generation apparatus 10, the processing unit 101, a memory 102, a ROM (read only memory) 103, a storage device 104, a communication interface (I/F) 105, an input interface (I/F) 106, an output interface (I/F) 107 and a media interface (I/F) 108 are data-communicably connected to one another by a bus 109. The memory 102 and the storage device 104 may be collectively referred to simply as "storage unit." The storage unit stores the generated discriminator, a reference values or reference ranges for mPS, measured values of biomarkers of each patient, and an mPS of each patient in a volatile or non-volatile manner.

The processing unit 101 is a CPU of the discriminator generation apparatus 10. The processing unit 101 may be a GPU. When the processing unit 101 executes a computer program stored in the storage device 104 or the ROM 103 and processes acquired data, the discriminator generation apparatus 10 functions.

The ROM 103 is constituted of a mask ROM, a PROM, an EPROM, an EEPROM or the like, and stores computer programs that are executed by the processing unit 101 and data used therefor. The processing unit 101 may be an MPU 101. The ROM 103 stores a boot program that is executed by the processing unit 101 when the discriminator generation apparatus 10 is activated and programs and settings related to the operation of the hardware of the discriminator generation apparatus 10.

The memory 102 is constituted of a RAM (Random access memory) such as an SRAM or DRAM. The memory 102 is used to read out computer programs stored in the ROM 103 and the storage device 104. Also, the memory 102 is utilized as a workspace when the processing unit 101 executes these computer programs.

The storage device 104 is constituted of a hard disk, a semiconductor memory device like a flash memory, an optical disc or the like. In the storage device 104, various computer programs to be executed by the processing unit 101 such as operating systems and application programs and various setting data used for execution of the computer programs are stored. Specifically, the storage device 104 stores the reference value and so on in a non-volatile manner.

The communication I/F 105 is constituted of a serial interface such as a USB, IEEE1394 or RS-232C, a parallel interface such as an SCSI, IDE or IEEE1284, an analog interface consisting of a D/A converter, A/D converter or the like, a network interface controller (NIC) and so on. Under the control of the processing unit 101, the communication I/F 105 receives data from a measurement unit 30 or other external devices and, when necessary, sends or displays information stored in or generated by the discriminator generation apparatus 10 to or on the measurement unit 30 or the outside. The communication I/F 105 may communicate with the measurement unit 30 or other external devices via a network.

The input I/F 106 is constituted, for example, of a serial interface such as a USB, IEEE1394 or RS-232C, a parallel interface such as an SCSI, IDE or IEEE1284, an analog interface consisting of a D/A converter, A/D converter or the like and so on. The input I/F 106 accepts text input, clicks, voice input or the like from the input unit 111. The accepted input is stored in the memory 102 or the storage device 104.

The input unit 111 is constituted of a touch panel, a keyboard, a mouse, a pen tablet, a microphone or the like, and performs text or voice input into the discriminator generation apparatus 10. The input unit 111 may be connected from the outside of the discriminator generation apparatus 10, or may be integrated with the discriminator generation apparatus 10.

The output I/F 107 is constituted, for example, of an interface similar to that for the input I/F 106. The output I/F 107 outputs information generated by the processing unit 101 to the output unit 112. The output I/F 107 outputs information generated by the processing unit 101 and stored in the storage device 104 to the output unit 112.

The output unit 112 is constituted, for example, of a display, a printer or the like, and displays measurement results transmitted from the measurement unit 30, various operation windows, results of analyses and so on in the discriminator generation apparatus 10.

The media I/F 108 reads out, for example, application software or the like stored in the storage medium 113. The read-out application software or the like is stored in the memory 102 or the storage device 104. Also, the media I/F 108 writes information generated by the processing unit 101 into the storage medium 113. The media I/F 108 writes information generated by the processing unit 101 and stored in the storage device 104 into the storage medium 113.

The storage medium 113 is constituted of a flexible disk, a CD-ROM, a DVD-ROM or the like. The storage medium 113 is connected to the media I/F 108 by a flexible disk drive, a CD-ROM drive, a DVD-ROM drive or the like. An application program that is used by a computer to execute an operation or the like may be stored in the storage medium 113.

The processing unit 101 may acquire application software or various settings necessary for control of the discriminator generation apparatus 10 via a network instead of reading those out of the ROM 103 or the storage device 104. When the application program is stored in a storage device of a server computer on a network, it is also possible for the discriminator generation apparatus 10 to access this server computer to download a computer program and store it into the ROM 103 or the storage device 104.

Also, an operation system that provides a graphical user interface environment such as Windows (trademark) manufactured and sold by Microsoft, USA or the like has been installed in the ROM 103 or the storage device 104. An application program according to a second embodiment shall operate on the operating system. In other words, the discriminator generation apparatus 10 may be a personal computer or the like.

3. Computer program for generating discriminator

A certain embodiment of this disclosure relates to a computer program for generating a discriminator. The computer program causes the processing unit 101 to execute step S1, step S2 and step S4, preferably steps S1 to S5, described in FIG. 1, and steps S31 to S34 described in FIG. 2.

In addition, a certain embodiment of this disclosure relates to a storage medium in which the computer program has been stored. In other words, the computer program is stored in a storage medium such as a hard disk, a semiconductor memory device like a flash memory, an optical disc or the like. The format in which the program is stored in the storage medium is not limited as long as the processing unit 101 can read the program. The program is preferably stored in the storage medium in a non-volatile manner.

4. Method for predicting survival rate of patient

A certain embodiment of this disclosure relates to a method for predicting a survival rate of a patient. In this embodiment, the discriminator generated by the method in the above section 1. is used to predict a survival rate of a patient after a predetermined period of time. The disease the patient is suffering from is not particularly limited, but the diseases listed in the above section 1.(1) can be incorporated here. Preferably, the disease is cancer, and more preferably breast cancer.

The survival rate of a patient after a predetermined period of time can be predicted by comparing the mPS value of the patient with a reference value. When the scores input into the discriminator are in the score pattern 1, specifically, the mPS value of each patient obtained by the discriminator is compared with its reference value. Then, when the mPS of the patient is larger than the reference value, it can be determined that the survival rate of the patient after a predetermined period of time, preferably after 10 or 20 years, will be poor. Also, when the mPS of the patient is equal to or smaller than the reference value, it can be determined that the survival rate of the patient after a predetermined period of time, preferably after 10 or 20 years, will be good. When the scores input into the discriminator are in the score pattern 2, specifically, the mPS value of each patient obtained by the discriminator is compared with its reference value.

Then, when the mPS of the patient is smaller than the reference value, it can be determined that the survival rate of the patient after a predetermined period of time, preferably after 10 or 20 years, will be poor. Also, when the mPS of the patient is equal to or larger than the reference value, it can be determined that the survival rate of the patient after a predetermined period of time, preferably after 10 or 20 years, will be good.

This embodiment may include a step of acquiring measured values of biomarkers from a specimen containing tissue or cells taken from a lesion site of the disease in the patient, or a specimen containing substances in the body that reflect the state of the disease such as blood samples (including whole blood, plasma, serum and so on), urine, cerebrospinal fluid, ascites, pleural effusion and so on. Measured values of biomarkers can be acquired by a known method. When the biomarkers are genes, measured values of the biomarkers can be acquired by, for example, a quantitative RT-PCR method, an RNA-Seq method or the like.

This embodiment may include a step of calculating an mPS value of each patient using a discriminator. The measured values of biomarkers of a group of genes in the second subpopulation or the third subpopulation obtained for each patient are binarized according to the method described in the above section 1. to determine the score of each gene. The processing unit 201 inputs the score of each gene into a discriminator stored in the storage unit to calculate the mPS of each patient.

The reference value is not limited as long as it is a value with which it can be determined that the survival rate of the patient after a predetermined period of time will be good and/or poor. For example, the reference value can be obtained from the mPS of a patient whose survival rate after a predetermined period of time is good or the mPS of a patient whose survival rate after a predetermined period of time is poor. Also, as another aspect, the reference value may be the upper limit of the mPSs in a group of patients whose survival rate after a predetermined period of time is good or the lower limit of the mPSs in a group of patients whose survival rate after a predetermined period of time is poor. Also, as a yet another aspect, the reference value may be the median, average, mode or the like of a plurality of mPSs in a population including both a group of patients whose survival rate after a predetermined period of time is good and a group of patients whose survival rate after a predetermined period of time is poor. Alternatively, the reference value may be calculated by an ROC curve (Receiver Operatorating Characteristic curve), a discriminant analysis method, a mode method, a Kittler method, a 3$\sigma$ method, a p-tile method or the like. It is preferred that the reference value has been determined in advance.

In this embodiment, for the terms used in the above section 1. such as discriminator, patient, cancer, survival rate, predetermined period, mPS and so on, the description in the above section 1. is incorporated here.

5. Apparatus for predicting survival rate of patient

This disclosure relates to an apparatus 20 for predicting a survival rate of a patient (also referred to as survival rate prediction apparatus 20). The survival rate prediction apparatus 20 includes at least the processing unit 201. Because the configuration of the survival rate prediction apparatus 20 is the same as that of the discriminator generation apparatus 10, the description in the above section 2. and FIG. 3 and FIG. 4 are incorporated here.

In FIG. 3 or FIG. 4, the discriminator generation apparatus 10, the processing unit 101, the memory 102, the ROM 103, the storage device 104, the communication interface (I/F) 105, the input interface (I/F) 106, the output interface (I/F) 107, the media interface (I/F) 108, the bus 109, the input unit 111, the output unit 112 and the storage medium 113 are replaced with a processing unit 201, a memory 202, a ROM 203, a storage device 204, a communication interface (I/F) 205, an input interface (I/F) 206, an output interface (I/F) 207, a media interface (I/F) 208, a bus 209, an input unit 211, an output unit 212 and a storage medium 213, respectively. Referring to FIG. 5, the operation of the survival rate prediction apparatus 20 is described. Here, a case where the scores that are input into the discriminator are in the score pattern 1 is described as an example.

First, in step S41, the processing unit 201 acquires measured values of biomarkers included in the second subpopulation or the third subpopulation input through the input unit 211 by a user for each cancer patient. Alternatively, the processing unit 201 acquires measured values of biomarkers included in the second subpopulation or the third subpopulation from a network through the communication I/F 205 for each patient according to an instruction to start processing input through the input unit 211 by a user. The processing unit 201 binarizes the measured value of each biomarker acquired according to the method described in the above section 1. to determine a score of each biomarker. The processing unit 201 inputs the score of each gene into a discriminator stored in the storage unit to calculate an mPS of each patient.

Next, in step S42, the processing unit 201 compares the mPS of each cancer patient with a reference value for mPS stored in the storage unit. In step S43, the processing unit 201 determines whether or not the mPS of each patient is larger than the reference value.

If the determination in step S43 is YES, it is determined that the survival rate of the patient after a predetermined period of time will be poor (step S44). If the determination in step S43 is NO, it is determined that the survival rate of the patient after a predetermined period of time will be good (step S45).

When the scores that are input into the discriminator are in the score pattern 2, if the determination in step S43 is YES, it is determined that the survival rate of the patient within or after a predetermined period of time will be good in step S44. If the determination in step S43 is NO, it is determined that the survival rate of the patient within or after a predetermined period of time will be poor in step S45.

Next, in step S46, the processing unit 201 outputs the determination result to the output unit 212. Although not shown, the processing unit 201 may store the determination result in the storage device 204.

In this embodiment, for the description of the terms in common with those in the above sections 1., 2. and 4., the description in the above sections 1., 2. and 4. is incorporated here.

6. Computer program for predicting survival rate of cancer patient

A certain embodiment of this disclosure relates to a computer program for predicting a survival rate of a cancer patient.

The computer program causes the processing unit 201 to execute steps S41 to S46 described in FIG. 5.

In addition, a certain embodiment of this disclosure relates to a storage medium in which the computer program has been stored. In other words, the computer program is stored in a storage medium such as a hard disk, a semiconductor memory device like a flash memory, an optical disc or the like. The format in which the program is stored in the storage medium is not limited as long as the processing unit 201 can read the program. The program is preferably stored in the storage medium in a non-volatile manner.

7. Method for assisting stratification of cancer patients

A certain embodiment of this disclosure relates to a method for assisting stratification of cancer patients based on their survival rates.

In this embodiment, mPSs calculated with a discriminator generated as described in the above section 1. are used to assist stratification of cancer patients based on survival rates of the cancer patients after a predetermined period of time. Specifically, cancer patients are stratified by comparing the mPS value of each cancer patient obtained by the discriminator with its reference ranges to determine the bin into which the mPS value of each cancer patient is categorized.

Specifically, this embodiment may include a step of acquiring measured values of biomarkers from a specimen containing tissue or cells taken from a lesion site of the disease in the cancer patient, or a specimen containing substances in the body that reflect the state of the disease such as blood samples (including whole blood, plasma, serum and so on), urine, cerebrospinal fluid, ascites, pleural effusion and so on. Measured values of biomarkers can be acquired by a known method. When the biomarkers are genes, measured values of the biomarkers can be acquired by, for example, a quantitative RT-PCR method, an RNA-Seq method or the like. Also, this embodiment may include a step of calculating an mPS value of each patient using a discriminator. The measured values of biomarkers in the second subpopulation or the third subpopulation obtained for each patient are binarized according to the method described in the above section 1. to determine the score of each gene. The processing unit 301 inputs the score of each gene into a discriminator stored in the storage unit to calculate the mPS of each patient.

For example, the reference ranges can be set to mPS=0 to 5 for a first bin, mPS=5 to 11 for a second bin, mPS=11 to 25 for a third bin, mPS=25 to 36 for a fourth bin, mPS=36 to 45 for a fifth bin, and mPS=45 to 50 for a sixth bin. In the case of the score pattern 1, because the survival rate after a predetermined period of time is poorer from the first bin to the sixth bin, the survival rate of each patient can be predicted in a stepwise fashion. In the case of the score pattern 2, because the survival rate after a predetermined period of time is better from the first bin to the sixth bin, the survival rate of each patient can be predicted in a stepwise fashion.

When the disease is cancer, the reference ranges may have been determined according to a category such as the histological type, clinical stage or pathological tissue grade, the age group or the like. In this case, the method for assisting stratification further includes acquiring information on the histological type, clinical stage or pathological tissue grade of the cancer and the age group according to the category. A processing unit 301 can acquire these pieces of information from an input through an input unit 311 by a user, linkage with an electronic health record or the like. It is preferred that the reference ranges have been determined in advance.

In this embodiment, for the terms used in the above section 1. such as discriminator, cancer, survival rate, predetermined period, mPS, histological type, subtype of cancer, clinical stage, pathological tissue grade, age group and so on, the description in the above section 1. is incorporated here.

8. Apparatus for stratifying patients

This disclosure relates to an apparatus 30 for stratifying patients (also referred to as stratification apparatus 30). The stratification apparatus 30 includes at least the processing unit 301. Because the configuration of the stratification apparatus 30 is the same as that of the discriminator generation apparatus 10, the description in the above section 2. and FIG. 3 and FIG. 4 are incorporated here.

In FIG. 3 or FIG. 4, the discriminator generation apparatus 10, the processing unit 101, the memory 102, the ROM 103, the storage device 104, the communication interface (I/F) 105, the input interface (I/F) 106, the output interface (I/F)107, the media interface (I/F) 108, the bus 109, the input unit 111, the output unit 112 and the storage medium 113 are replaced with a processing unit 301, a memory 302, a ROM 303, a storage device 304, a communication interface (I/F) 305, an input interface (I/F) 306, an output interface (I/F) 307, a media interface (I/F) 308, a bus 309, an input unit 311, an output unit 312 and a storage medium 313, respectively.

Referring to FIG. 6, the operation of the stratification apparatus 30 is described.

First, in step S51, the processing unit 301 acquires measured values of biomarkers included in the second subpopulation or the third subpopulation input through the input unit 311 by a user for each patient. Alternatively, the processing unit 301 acquires measured values of biomarkers included in the second subpopulation or the third subpopulation from a network through the communication I/F 305 for each patient according to an instruction to start processing input through the input unit 311 by a user. The processing unit 301 binarizes the measured value of each biomarker acquired according to the method described in the above section 1. to determine a score of each biomarker. The processing unit 301 inputs the score of each gene into a discriminator stored in the storage unit to calculate an mPS of each patient.

Next, in step S52, the processing unit 301 compares the mPS of each patient with reference ranges for mPS stored in a storage unit. In step S53, it is determined that the patient has a survival rate of the bin to which the mPS of the patient belongs.

Next, in step S54, the processing unit 301 outputs the determination result to the output unit 312. Although not shown, the processing unit 301 may store the determination result in the storage device 304.

In this embodiment, for the description of the terms in common with those in the above sections 1., 2. and 7., the description in the above sections 1., 2. and 7. is incorporated here.

## 9. Computer program for stratifying patients

A certain embodiment of this disclosure relates to a computer program for stratifying cancer patients.

The computer program causes the processing unit 301 to execute steps S51 to S54 described in FIG. 6.

In addition, a certain embodiment of this disclosure relates to a storage medium in which the computer program has been stored. In other words, the computer program is stored in a storage medium such as a hard disk, a semiconductor memory device like a flash memory, an optical disc or the like. The format in which the program is stored in the storage medium is not limited as long as the processing unit 301 can read the program. The program is preferably stored in the storage medium in a non-volatile manner.

## 10. Biomarker

A certain embodiment of this disclosure relates to the use of at least one gene selected from the genes described in Table 1-1 and Table 1-2 as a biomarker for predicting the survival rate of a breast cancer patient. Preferably, the biomarker does not include an aspect consisting only of ANLN, FOXM1, RBBP8 or a combination of these genes. More preferably, at least one selected from the group consisting of FOXM1, CPT1A, GARS, MARS, UTP23, ANLN, HMGB3, ATP5B, APOOL, CYB561, GRHL2, ESRP1, EZR, RBBP8, CIRBP, PTGER3, LAMA3, OARD1, ANKRD29, EGR3, DIRAS3, MITD1 and LAMB3 (with the proviso that an aspect consisting only of ANLN, FOXM1, RBBP8 or a combination of these genes is not included) is used as a biomarker for predicting a survival rate of a breast cancer patient. The expression level of an mRNA and/or a protein derived from at least one gene selected from the group of genes is used as a biomarker.

## 11. Inspection reagent

A certain embodiment of this disclosure relates to an inspection reagent used to predict a survival rate of a breast cancer patient that contains a probe, primer or antibody for detecting the expression level of an mRNA or protein derived from at least one gene selected from the genes described in Table 1-1 and Table 1-2. Preferably, an aspect consisting only of ANLN, FOXM1, RBBP8 or a combination of these genes is not included. More preferred is at least one selected from the group consisting of FOXM1, CPT1A, GARS, MARS, UTP23, ANLN, HMGB3, ATP5B, APOOL, CYB561, GRHL2, ESRP1, EZR, RBBP8, CIRBP, PTGER3, LAMA3, OARD1, ANKRD29, EGR3, DIRAS3, MITD1 and LAMB3 (with the proviso that an aspect consisting only of ANLN, FOXM1, RBBP8 or a combination of these genes is not included). This inspection reagent is not limited as long as it can be used in an RT-PCR method, a microarray method, an RNA-Seq method, an ELISA method, a western blotting method or the like.

The inspection reagent may constitute an inspection kit used to predict a survival rate of a breast cancer patient that includes the inspection reagent, an enzyme for reacting the inspection reagent, a reaction liquid in which a hybridization or antigen-antibody reaction is carried out, an instruction manual or a printed medium showing the URL or QR code (trademark) of a Web page that provides the instruction manual.

[Examples]

Examples are hereinafter shown to describe this disclosure in more detail. However, this disclosure should not be construed as being limited to the examples.

I. Generation of discriminator

<Cohort>

A plurality of known breast cancer cohorts was used to generate a discriminator. There were few patients with metastasis from the cohorts used in this study.

The TCGA cohort was used as a discovery cohort. As the TCGA cohort, the GISTEC-based copy number alterrarions (CNAs) cohort [n=958; Curtis, C. et al. Nature 486, 346-352, doi:10.1038/nature10983 (2012); hereinafter referred to as "TCGA cohort"] of the TCGA breast cancer dataset was downloaded from cBioPortal [Cerami, E. et al. Cancer Discov. 2, 401-404, doi:10.1158/2159-8290.cd-12-0095 (2012)]. The GISTEC can be referred to Mermel, C. H. et al. Genome Biol. 12, R41, doi:10.1186/gb-2011-12-4-r41(2011). In cBioPortal, the expression levels of mRNAs derived from each gene in breast cancer tissue (result of analysis by RNA-seq) are registered.

As a validation cohort for use in step 2, multicenter combined breast cancer cohorts [n=5844; Abdel-Fatah, T. M. A. et al. The Lancet. Oncology 17, 1004-1018, doi:10.1016/s1470-2045(16)00174-1 (2016); hereinafter referred to as "multicenter cohorts") were used.

As training data for use in step 3, the Whole METABRIC cohort [n=1904; Pereira, B. et al. Nature communications 7, 11479, doi:10.1038/ncomms11479 (2016) and Curtis, C. et al. Nature 486, 346-352, doi:10.1038/nature10983 (2012); hereinafter referred to as "METABRIC cohort"] was used. The METABRIC cohort is independent of the TCGA cohort.

The breakdown of each cohort is shown in Table 2.

[Table 2]

| | TCGA | METABRIC |
|---|---|---|
| Age<br>Median (IQR) | 59 (49-68) | 61.8 (51.4-70.6) |
| PAM50 | | |
| LumA | 415 | 679 |
| LumB | 176 | 461 |
| HER2 | 65 | 220 |
| Claudin-low | 0 | 199 |
| Normal-like | 25 | 140 |
| Basal | 136 | 199 |
| ONCOTREE | | |
| IDC | 643 | 1500 |
| ILC | 167 | 141 |
| MDLC | 26 | 87 |
| Clinical Stage | | |
| I | 157 | 475 |
| II | 545 | 800 |
| III | 220 | 115 |
| IV | 15 | 9 |
| NPI | | |
| Excellent | n.d. | 163 |
| Good | n.d. | 477 |

(continued)

| | | |
|---|---|---|
| NPI | | |
| Moderate I | n.d. | 662 |
| Moderate II | n.d. | 408 |
| Poor | n.d. | 194 |

<Gene list>

A list of human genes was acquired from HUGO Gene Nomenclature Committee (HGNC).

<Analysis method>

For download of data from cBioPortal, CGDS-R package and Web APIs provided by cBioPortal were used. For a random forest approach of machine learning, a Python-based scikit-learn library with parameters defaulted except n_estimators (= 500) and max_depth (= 10) was used. A Python-based TensorFlow library was also used for a neural network analysis. For other analyses, Python (3.6.2) established in Anaconda distribution and written in a custom script was used.

<Statistical analysis>

Kaplan-Meier plots were created using R (survival package), and validated using Python (lifelines package). As a cutoff value for dividing a low expression group and a high expression group for each gene, the median was used. In this example, the low expression may be represented by a superscript "low" and the high expression by a superscript "high." For validation of the molecular prognostic score (mPS), the data were truncated at 10 years unless otherwise specified. The survival period was from the date of diagnosis to the date of death except for the meta-analysis shown in FIG. 8e. A Log-rank test was conducted to compare the survival rates. For meta-analysis, the R survival package was used to estimate hazard ratios (HRs) and their 95% confidence intervals (CIs) with Cox regression. The presence or absence of a significant difference was determined at a two-sided p-value of 0.05 except for the discovery cohort (FIG. 8), and the cutoff was set to 0.01 in order to reduce the number of candidate genes.

<Discriminator generation steps>

FIG. 7 shows an outline of the generation of a discriminator for predicting the prognosis of cancer from multiple cohorts.

The method for generating a discriminator roughly includes the following five steps.

Step 1: Extraction of candidate genes
Step 2: Validation of candidate genes
Step 3: Extraction of feature values by machine learning
Step 4: Construction of a discriminator by deep learning
Step 5: Validation of discriminator

Each step is described.

1. Step 1

In step 1, the TCGA cohort was downloaded, and gene candidates that are related to the 10-year survival rate of breast cancer patients were explored based on the number of reads (expression levels) of these RNAs. The median age in this discovery cohort was 59 years old (IQR 49-68).

For each gene, the cohort was divided into two groups (a low expression group and a high expression group) at the median of the RNA read number. Then, survival curves for 10 years were generated for all genes by Kaplan-Meier Plots of the low expression group and the high expression group.

Next, a significant difference by a Log-rank test was obtained for the survival curves, and candidate genes possibly related to a poor survival rate were extracted based on the p-value. As a result, 286 genes were extracted as candidates. FIGs. 8a, 8b, 8c and 8d show Kaplan-Meier Plots of PGK1, TMEM65, BEND5, and ENOSF1, respectively, as examples of candidates genes. PGK1 and TMEM65 are examples in which the survival rate in breast cancer was worse in the high expression group than in the low expression group. BEND5 and ENOSF1 are examples in which the survival rate in breast cancer was worse in the low expression group than in the high expression group. In FIGs. 8a, 8b, 8c and 8d, the symbol H indicates the high expression group and the symbol L indicates the low expression group.

2. Step 2

In step 2, the 286 genes picked up by a Log-rank test were validated by a meta-analysis using the multicenter cohorts as validation cohorts, and 184 genes were further extracted as candidate genes (FIG. 8e). Among the extracted genes, TMEM65 and RILPL2 showed the highest HR expression level (FIG. 9-1a) and the lowest HR expression level (FIG. 9-1b), respectively, in the multicenter cohorts, and were genes that are considered to be most closely related to the prognosis of breast cancer. These genes have not been addressed neither in basic cancer studies nor in clinical cancer studies.

A combination of genes that are considered to be second most closely related to the prognosis of breast cancer is that of $TMEM65^{high}$ and $DCTPP1^{high}$, which was observed in 244 patients in the TCGA cohort. In the cohort having an expression pattern of this combination, the survival rate was poor (FIG. 9-2c: HR=2.971).

Also, the cohort having an expression pattern of a combination of $UBA7^{low}$ and $ENOSF1^{low}$, which was observed in 272 patients in the TCGA cohort, also had a poor survival rate (FIG. 9-2d: HR=3.109). The HRs of other genes are shown in FIG. 10-1 to FIG. 10-4.

3. Step 3

In step 3, feature values were extracted by a random forest for the 184 genes extracted in step 2 to extract genes that are related more closely to the survival rate of breast cancer. As training data, a cohort with n=952 extracted at random from the METABRIC cohort was used. The result obtained by a random forest was validated by a cross-validation. In this way, the 23 genes shown in FIG. 11-1a were selected as predictive genes for predicting a 10-year survival rate of breast cancer. 13 genes out of the 23 genes may lead to a poor prognosis when their expression is higher than the median and 10 genes may lead to a poor prognosis when their expression is lower than the median.

4. Step 4

In step 4, a neural network was used to calculate weights of the 23 genes extracted in step 3. As training data, a cohort with n=952 extracted at random from the METABRIC cohort, which was also used in step 3, was used. By deep learning with a neural network using the training data, a loss function of cross entropy of each gene was defined, and a weight of each gene "Gene_Weight" was calculated by a gradient descent method [FIG. 12 : Kourou, K., et al. Computational and structural biotechnology journal 13, 8-17,doi:10.1016/j.csbj.2014.11.005 (2015)].

To link the expression levels of the 23 genes shown in FIG. 11-1a with the prognosis and score it, "Gene Scores" were set. Specifically, for genes that may lead to a poor prognosis when their expression is higher than the median, "1" is given as a "Gene_Score" when the expression of each gene in the breast cancer tissue of the patient is higher than the median, and "0" is given as a "Gene_Score" when the expression of each gene in the breast cancer tissue of the patient is lower than the median. For genes that may lead to a poor prognosis when their expression is lower than the median, "1" is given as a "Gene_Score" when the expression of each gene in the breast cancer tissue of the patient is lower than the median, and "0" is given as a "Gene_Score" when the expression of each gene in the breast cancer tissue of the patient is higher than the median.

Next, a discriminator for scoring the prognosis of the breast cancer of each patient based on the expression levels of the 23 genes shown in FIG. 11-1a is established. In this specification, the score determined using the discriminator is referred to as "molecular prognostic score (mPS)."

The discriminator is a weighed sum of the "Gene_Scores" of the 23 genes shown in FIG. 11-1a in the breast cancer tissue of each patient. In other words, it is represented by the following formula.

[Mathematical formula 3]

$$mPS = \sum_{i=1}^{n} (w_i * GS_i)$$

(wherein w represents a "Gene_Weight," GS indicates a "Gene_Score," n is 23, i indicates each gene, and wi*GSi represents the product of the weight and score of each biomarker. 23 indicates the 23 genes shown in FIG. 11-1a.).

In this weighed sum, the "Gene_Weight" was set such that the mPS falls within the range of 0 to 50 (FIG.

11-1a). The distribution of mPSs in the METABRIC cohort is shown in FIG. 11-2b. The mPSs had an average of 24.973, an interquartile range of 15.915-34.030, and a standard deviation of 11.297.

<Validation of discriminator>

FIG. 11-2c shows the stage classification of breast cancer and the distribution of cohort in each of six bins into which the mPS range of 0 to 50 is divided. The bins include a first bin with an mPS=0 to 5 (hereinafter represented by a symbol "1st" in the drawings), a second bin with an mPS=5 to 11 (hereinafter represented by a symbol "2nd" in the drawing), a third bin with an mPS=11 to 25 (hereinafter represented by a symbol "3rd" in the drawings), a fourth bin with an mPS=25 to 36 (hereinafter represented by a symbol "4th" in the drawings), a fifth bin with a mPS=36 to 45 (hereinafter represented by a symbol "5th" in the drawings), and a sixth bin with an mPS=45 to 50 (hereinafter represented by a symbol "6th" in the drawings). In FIG. 11-2c, the symbol S1 indicates breast cancer stage I, the symbol S2 indicates breast cancer stage II, and the symbol S3 indicates breast cancer stage III.

As shown in FIG. 11-2c, the mPS exhibited a good correlation with the clinical stage.

FIG. 11-3d shows the survival rates of the cohorts belonging to each bin of the first to sixth bins of mPS of the METABRIC cohort. FIG. 11-3e shows the survival rates of the cohorts belonging to each bin of the first to sixth bins of mPS of the TCGA cohort. In both cohorts, the mPS can stratify the survival rates. It was shown that the mPS can stratify a breast cancer cohort according to the survival rate even without clinical information. In addition, because a cohort can be stratified regardless of the type of the cohort, it was shown that the discriminator has a high generalization/versatile ability.

Also, because the discriminator shown by Mathematical Formula 3 is generated using information from databases that are all known, it is believed that a discriminator can be generated for any cancer for which a database exists by a method similar to that used in this study.

II. Evaluation of application potentiality of mPS

In order to validate to which breast cancer subset the discriminator (Mathematical Formula 3) is applicable, the following examination was performed.

Breast cancer is categorized into Luminal A (LumA), Luminal B (LumB), HER2-enriched, Claudin-low, Normal-like, and Basal-like subtypes based on PAM50 intrinsic subtypes. Thus, in each group of a cohort categorized according to each breast cancer subtype, it was examined whether the discriminator can stratify the cohort according to the survival rate. The results are shown in FIG. 13-1a: METABRIC cohort·HER2-enriched (n=220), FIG. 13-1b: METABRIC cohort·Claudin-low (n=199), and FIG. 13-2c: METABRIC cohort·Normal-like (n=140). Even in HER2-enriched, Claudin-low, and Normal-like subtypes, the mPS exhibited a good correlation with the survival rate. This indicates that the discriminator has a possibility of being applicable to various breast cancer subtypes.

Also, in the treatment of breast cancer, the therapeutic choice for a pre-menopause cohort, e.g. younger than 50 years old and the therapeutic choice for an elder cohort, e.g. older than 70 years old may be different. Thus, a cohort is categorized according to the age group and it was examined whether the discriminator can stratify a cohort according to the survival rate using the METABRIC cohort. As shown in FIG. 13-2d: METABRIC cohort·< 50 years old, FIG. 14a: METABRIC cohort·50s and 60s, and FIG. 14b: METABRIC cohort·older than 70 years old, the mPS also exhibited a correlation with the survival rate in a cohort younger than 50 years old, a cohort in their 50s and 60s, and an elder cohort (not younger than 70 years old). In addition, the 20-year survival rate was tracked for the cohort younger than 50 years old to examine whether the discriminator can stratify a cohort according to the survival rate. As shown in FIG. 14c: METABRIC, younger than 50 years (n=411), a cohort with a high mPS value has a poorer prognosis than a cohort with a low mPS value in the 20-year survival rate. This indicates that the discriminator can precisely stratify cohorts of various age groups even when the follow-up period is extended.

In histological categorization of breast cancer subtypes, most breast cancer tissue is categorized as invasive ductal carcinoma (IDC). As shown in FIG. 15a: METABRIC, IDC (n=1,500), the mPS exhibited a good correlation with the survival rate even in a cohort of invasive ductal carcinoma. The second most frequent histological subtype of breast cancer is invasive lobular carcinoma (ILC) and mixed IDC and ILC (MDLC). ILC and MDLC are totally different from IDC in a histopathological sense. Thus, the application potentiality of the discriminator was examined for cohorts of these subtypes. As shown in FIG. 13-3e: METABRIC, ILC (n=141) and FIG. 15b: METABRIC, MDLC (n=87), the mPS also exhibited a good correlation with the survival rate in an ILC cohort and in an MDLC cohort. This indicates that the discriminator has a possibility of being applicable to various histopathological subtypes of breast cancer.

Histological subtypes of breast cancer are also categorized according to the malignancy diagnosed from histological appearance. The malignancy is divided into three grades depending on the degree of differentiation of cells constituting the cancer tissue: G1 (Grade 1: well differentiated), G2 (Grade 2: moderately differentiated) and G3 (Grade 3: poorly differentiated). Thus, a cohort was categorized according to these grades, and the correlation between the mPS and the survival rate was examined for the cohort of each grade after the categorization. The results are shown in FIG. 15-2c: METABRIC, Grade 1 (n=165), FIG. 13-3f: METABRIC, Grade 2 (n=740) and FIG. 15-2d: METABRIC, Grade 3 (n=927). The mPS was well-correlated with the survival rate in all the grades. This indicates that the discriminator has a possibility of being applicable to various grades of breast cancer.

In general, the TNM categorization is applied to categorization showing the degree of progression (stage) of cancer not only in the case of breast cancer. However, the TNM categorization is a method for categorizing the degree of progression of cancer based on information such as histological appearance, metastasis in lymph nodes and so on regardless of the various subtypes of cancer as described above. The TNM categorization categorizes breast cancer tissue into stages I to VI by an academically determined method. For example, even in a stage I patient whose degree of progression of cancer is determined to be lowest by the TNM categorization, the result may be different from the grade of malignancy of cancer tissue because the degree of progression is determined based on the TNM categorization criteria independently of each subtype as described above.

Thus, it is examined what relationship is there between the TNM categorization and the mPS.

In the METABRIC cohort, a cohort with n=475 was categorized into stage I. In each patient with a survival rate of more than 10 years among patients belonging to stage I, about 90% of patients showed an mPS of lower than 11 (a good 10-year survival rate). However, the other patients had an mPS of higher than 25 and, consequently, a low 10-year survival rate even though they were categorized into stage I by the TNM categorization [FIG. 17-1a: METABRIC, Stage I].

In the METABRIC cohorts, a cohort with n=800 was categorized into stage II. Among patients belonging to stage II, patients with an mPS below 5 showed a very good survival rate. On the other hand, patients with an mPS of above 45 had a poor survival rate [FIG. 17-1b: METABRIC, Stage II].

In the METABRIC cohorts, a cohort with n=115 was categorized into stage III. Among patients belonging to stage III, patients with an mPS below 25 showed a relatively good survival rate. On the other hand, patients with an mPS above 25 had a poor survival rate [FIG. 17-2c: METABRIC, Stage III].

Similar examination was performed on the TCGA cohort. In the TCGA cohort, a cohort with n=545 was categorized into stage II, and showed a similar tendency to that of the METABRIC cohort [FIG. 17-2d: TCGA, Stage II].

For the TCGA cohort, a cohort including all of stages I-III was divided into three bins according to the mPS (first bin: mPS 0-11, second bin: mPS 11-36, and third bin: mPS 36-50), and the relationship with each stage was examined. As shown in FIG. 17-3e: TCGA, Stage I-III, it was shown that the mPS increases and the survival rate becomes worse as the stage progressed.

Thus, it was proved that the mPS exhibits a correlation with the stage.

Next, the relation between the Nottingham Prognostic Index (NPI) as a prognosis predictive factor for breast cancer and the mPS was validated. The NPI is calculated based on the size of the primary tumor, the number of lymph nodes involved and the tumor grade. The METABRIC cohort is categorized into five NPI groups [Excellent (NPI <2.41), Good (2.41-3.4), Moderate I (3.41-4.4), Moderate II (4.41-5.4), and Poor (NPI>5.4)], and the relationship with the mPS was examined. As a result, it was proved that the NPI is heterogeneous (non-uniform) grouping in the survival rate. A cohort categorized into the NPI Moderate II is shown in FIG. 17-3f. Even taking a look only at the Moderate II (n=408) group, the mPS varied widely, and the survival rate also varied widely. The results of other groups are shown in FIG. 16-1a: METABRIC, Excellent, FIG. 16-1b: METABRIC, Good, FIG. 16-2c: METABRIC, Moderate I and FIG. 16-2d: METABRIC, Poor.

Finally, based on the mPS, a clinical stage categorization of breast cancer according to a new integrated score was created. FIG. 18a shows the result of categorizing the clinical stages I to IV of cancer, based on the first bin (mPS 0-5) to the sixth bin (mPS 45-50) of the mPS, into 7 classes: A, B, C, D, E, F-I and F-II. The distribution of the first to sixth bins of the mPS was in excellent agreement with the clinical stage categorization. FIG. 18b shows the survival rate in a cohort with n=2340 combining the TCGA cohort and the METABRIC cohort. The clinical stage categorization also exhibited a good correlation with the survival rate.

From above, it was concluded that the mPS precisely reflects the survival rate of cancer patients within and after a predetermined period of time.

In addition, it was shown that the mPS has a generalization/versatile ability because it exhibited a good correlation with the clinical stage categorization, clinical stage and pathological grade of cancer according to the integrated score.

## Claims

**1.** A method for generating a discriminator, comprising:

step 1 of determining, for a parent population of biomarkers derived from a test subject, whether each biomarker varies in relation with an event that will occur in a body of the test subject by a statistical method based on a measured value of each biomarker, and extracting a group of biomarkers determined to vary as a first subpopulation,
step 2 of validating each biomarker belonging to the first subpopulation, and extracting a group of biomarkers statistically predicted to be related more closely to the event that will occur in the body as a second subpopulation, and
step 3 of calculating a weight of each biomarker belonging to the second subpopulation by a deep learning method,

wherein the discriminator calculates a weighed sum of scores of biomarkers belonging to the second subpopulation using a score obtained from the measured value of each biomarker belonging to the second subpopulation and the weight of each biomarker calculated in step 3.

**2.** A method for generating a discriminator, comprising:

step A of determining, for a parent population of biomarkers derived from a test subject, whether each biomarker varies in relation with an event that will occur in a body of the test subject by a statistical method based on a measured value of each biomarker, and extracting a group of biomarkers determined to vary as a first subpopulation,
step B of validating each biomarker belonging to the first subpopulation, and extracting a group of biomarkers statistically predicted to be related more closely to the event that will occur in the body as a second subpopulation,
step C of extracting a group of biomarkers statistically predicted to be related much more closely to an event that will occur in the body from the biomarkers belonging to the second subpopulation as a third subpopulation by a machine learning method, and
step D of calculating a weight of each biomarker belonging to the third subpopulation by a deep learning method,

wherein the discriminator calculates a weighed sum of scores of biomarkers belonging to the third subpopulation using a score obtained from the measured value of each biomarker belonging to the third subpopulation and the weight of each biomarker calculated in step D.

**3.** The method for generating a discriminator according to Claim 1 or 2, wherein the machine learning method is a random forest.

**4.** The method for generating a discriminator according to any one of Claims 1 to 3, wherein the deep learning method is a gradient descent method.

**5.** The method for generating a discriminator according to any one of Claims 1 to 4, wherein the validation is a meta-analysis.

**6.** The method for generating a discriminator according to any one of Claims 1 to 5, wherein the event that will occur in the body is a survival rate of a patient within or after a predetermined period of time.

**7.** The method for generating a discriminator according to Claim 6, wherein a disease the patient is suffering from is cancer.

**8.** The method for generating a discriminator according to any one of Claims 1 to 7, wherein the biomarkers are genes, and the measured value of the biomarker is an expression level of an mRNA or protein derived from the gene.

**9.** A discriminator generation apparatus, comprising a processing unit, wherein the processing unit executes the method for generating a discriminator according to any one of Claims 1 to 8.

**10.** A method of use in which a discriminator generated by the method for generating a discriminator according to any one of Claims 1 to 8 is used to predict the survival rate of a patient within or after a predetermined period of time.

**11.** The method of use according to Claim 10, wherein the patient is a cancer patient.

**12.** The method of use according to Claim 11, wherein the cancer is breast cancer.

**13.** A method for predicting a survival rate of a patient, comprising the steps of:

acquiring, for a patient, a weighed sum calculated using a discriminator generated by the method for generating a discriminator according to any one of Claims 1 to 8, and
determining that the patient has a good survival rate when a weighed sum calculated using a discriminator generated by the method for generating a discriminator according to any one of Claims 1 to 8 takes a smaller value as the survival rate within or after a predetermined period of time is better and when the weighed sum of the patient is equal to or smaller than a reference value.

**14.** A method for predicting a survival rate of a patient, comprising the steps of:

acquiring, for a patient, a weighed sum calculated using a discriminator generated by the method for generating a discriminator according to any one of Claims 1 to 8, and
determining that the patient has a poor survival rate when a weighed sum calculated using a discriminator generated by the method for generating a discriminator according to any one of Claims 1 to 8 takes a smaller value as the survival rate within or after a predetermined period of time is better and when the weighed sum of the patient is larger than a reference value.

**15.** A method for predicting a survival rate of a patient, comprising the steps of:

acquiring, for a patient, a weighed sum calculated using a discriminator generated by the method for generating a discriminator according to any one of Claims 1 to 8, and
determining that the patient has a good survival rate when a weighed sum calculated using a discriminator generated by the method for generating a discriminator according to any one of Claims 1 to 8 takes a larger value as the survival rate within or after a predetermined period of time is better and when the weighed sum of the patient is equal to or larger than a reference value.

**16.** A method for predicting a survival rate of a patient, comprising the steps of:

acquiring, for a patient, a weighed sum calculated using a discriminator generated by the method for generating a discriminator according to any one of Claims 1 to 8, and
determining that the patient has a poor survival rate when a weighed sum calculated using a discriminator generated by the method for generating a discriminator according to any one of Claims 1 to 8 takes a larger value as the survival rate within or after a predetermined period of time is better and when the weighed sum of the patient is smaller than a reference value.

**17.** The prediction method according to any one of Claims 13 to 16, wherein the patient is a cancer patient.

**18.** The prediction method according to Claim 17, wherein the cancer is breast cancer.

**19.** The prediction method according to Claim 18, wherein the biomarkers extracted as a second subpopulation when a discriminator is generated include the genes shown in Table 1-1 and Table 1-2 below, and the measured value of the biomarker is the expression level of an mRNA or protein derived from the gene;

[Table 1-1]

| | Gene name | NCBI Gene ID | | Gene name | NCBI Gene ID | | Gene name | NCBI Gene ID |
|---|---|---|---|---|---|---|---|---|
| 1 | ADHFE1 | 137872 | 41 | DCAF16 | 54876 | 81 | LEF1 | 51176 |
| 2 | AMD1 | 262 | 42 | DCTPP1 | 79077 | 82 | LEPROTL1 | 23484 |
| 3 | ANKRD29 | 147463 | 43 | DEF6 | 50619 | 83 | LRP11 | 84918 |
| 4 | ANLN | 54443 | 44 | DERL1 | 79139 | 84 | LTF | 4057 |
| 5 | AP5S1 | 55317 | 45 | DHRS1 | 115817 | 85 | MAL2 | 114569 |
| 6 | APOBEC3F | 200316 | 46 | DIRAS3 | 9077 | 86 | MAP4K1 | 11184 |
| 7 | APOBEC3G | 60489 | 47 | EEF1G | 1937 | 87 | MAPT | 4137 |

(continued)

| | Gene name | NCBI Gene ID | | Gene name | NCBI Gene ID | | Gene name | NCBI Gene ID |
|---|---|---|---|---|---|---|---|---|
| 8 | APOL3 | 80833 | 48 | EGR3 | 1960 | 88 | MARS | 4141 |
| 9 | APOOL | 139322 | 49 | ENOSF1 | 55556 | 89 | MAX | 4149 |
| 10 | ARHGAP26 | 23092 | 50 | ESRP1 | 54845 | 90 | MITD1 | 129531 |
| 11 | ARMC1 | 55156 | 51 | EVA1C | 59271 | 91 | MMGT1 | 93380 |
| 12 | ATP5B | 506 | 52 | EZR | 7430 | 92 | MRPL13 | 28998 |
| 13 | ATP6V1C1 | 528 | 53 | FAXDC2 | 10826 | 93 | MTDH | 92140 |
| 14 | BEND5 | 79656 | 54 | FKBP5 | 2289 | 94 | MTFR1 | 9650 |
| 15 | BTF3 | 689 | 55 | FOXM1 | 2305 | 95 | NDUFAF6 | 137682 |
| 16 | BTG1 | 694 | 56 | GARS | 2617 | 96 | NEFH | 4744 |
| 17 | C1RL | 51279 | 57 | GRHL2 | 79977 | 97 | NFKBIA | 4792 |
| 18 | CARD10 | 29775 | 58 | GSTM4 | 2948 | 98 | NKAP | 79576 |
| 19 | CASP9 | 842 | 59 | GSTO1 | 9446 | 99 | NONO | 4841 |
| 20 | CCDC115 | 84317 | 60 | GTF2IRD2B | 389524 | 100 | NR1H3 | 10062 |
| 21 | CCDC24 | 149473 | 61 | H6PD | 9563 | 101 | NSA2 | 10412 |
| 22 | CCDC69 | 26112 | 62 | HCCS | 3052 | 102 | NSMCE2 | 286053 |
| 23 | CCNE1 | 898 | 63 | HILPDA | 29923 | 103 | OARD1 | 221443 |
| 24 | CCT2 | 10576 | 64 | HMGB3 | 3149 | 104 | PARG | 8505 |
| 25 | CCT4 | 10575 | 65 | HNRNPA1L2 | 144983 | 105 | PARP3 | 10039 |
| 26 | CCT6A | 908 | 66 | HSP90AA1 | 3320 | 106 | PCMT1 | 5110 |
| 27 | CD24 | 100133941 | 67 | HSPA2 | 3306 | 107 | PDCD2 | 5134 |
| 28 | CD48 | 962 | 68 | IARS | 3376 | 108 | PGK1 | 5230 |
| 29 | CD52 | 1043 | 69 | IGF2R | 3482 | 109 | PHF7 | 51533 |
| 30 | CD6 | 923 | 70 | IP6K2 | 51447 | 110 | PLAT | 5327 |
| 31 | CD74 | 972 | 71 | IRF2 | 3660 | 111 | PLEKHA4 | 57664 |
| 32 | CDK8 | 1024 | 72 | ITGA10 | 8515 | 112 | PLXNB3 | 5365 |
| 33 | CIR1 | 9541 | 73 | JAK1 | 3716 | 113 | PNRC2 | 55629 |
| 34 | CIRBP | 1153 | 74 | KANSL2 | 54934 | 114 | POP1 | 10940 |
| 35 | COG8 | 84342 | 75 | KDM4B | 23030 | 115 | PRKAB2 | 5565 |
| 36 | COPS6 | 10980 | 76 | KIF21A | 55605 | 116 | PSMD10 | 5716 |
| 37 | CPT1A | 1374 | 77 | KRCC1 | 51315 | 117 | PSME1 | 5720 |
| 38 | CRK | 1398 | 78 | LAMA3 | 3909 | 118 | PTGER3 | 5733 |
| 39 | CYB561 | 1534 | 79 | LAMB3 | 3914 | 119 | PTK2 | 5747 |
| 40 | CYP27A1 | 1593 | 80 | LARP4B | 23185 | 120 | PYCARD | 29108 |

[Table 1-2]

| | Gene name | NCBI Gene ID | | Gene name | NCBI Gene ID |
|---|---|---|---|---|---|
| 121 | QPRT | 23475 | 161 | TPRG1 | 285386 |

(continued)

| | Gene name | NCBI Gene ID | | Gene name | NCBI Gene ID |
|---|---|---|---|---|---|
| 122 | RAB2A | 5862 | 162 | TPT1 | 7178 |
| 123 | RAC2 | 5880 | 163 | TRAF5 | 7188 |
| 124 | RAD1 | 5810 | 164 | TRAPPC12 | 51112 |
| 125 | RAPGEFL1 | 51195 | 165 | TRIM34 | 53840 |
| 126 | RARRES3 | 5920 | 166 | TRIM35 | 23087 |
| 127 | RBBP8 | 5932 | 167 | TRIM45 | 80263 |
| 128 | REPS1 | 85021 | 168 | TVP23C | 201158 |
| 129 | RILPL2 | 196383 | 169 | UBA7 | 7318 |
| 130 | RPF1 | 80135 | 170 | UBE2A | 7319 |
| 131 | RPL11 | 6135 | 171 | UBTF | 7343 |
| 132 | RPL14 | 9045 | 172 | UBXN1 | 51035 |
| 133 | RPS6KA1 | 6195 | 173 | UBXN11 | 91544 |
| 134 | RTN4IP1 | 84816 | 174 | UTP23 | 84294 |
| 135 | SAV1 | 60485 | 175 | VBP1 | 7411 |
| 136 | SCUBE2 | 57758 | 176 | WLS | 79971 |
| 137 | SERFINA1 | 5265 | 177 | YTHDF3 | 253943 |
| 138 | SH3D21 | 79729 | 178 | ZBED6CL | 113763 |
| 139 | SHISA5 | 51246 | 179 | ZDHHC9 | 51114 |
| 140 | SKP1 | 6500 | 180 | ZFHX3 | 463 |
| 141 | SLC35A2 | 7355 | 181 | ZMYND10 | 51364 |
| 142 | SLC7A5 | 8140 | 182 | ZNF496 | 84838 |
| 143 | SPATA7 | 55812 | 183 | ZNF552 | 79818 |
| 144 | SPOCK2 | 9806 | 184 | ZNF671 | 79891 |

(continued)

| | Gene name | NCBI Gene ID | | Gene name | NCBI Gene ID |
|---|---|---|---|---|---|
| 145 | STC2 | 8614 | | | |
| 146 | STEAP2 | 261729 | | | |
| 147 | STK3 | 6788 | | | |
| 148 | STK39 | 27347 | | | |
| 149 | STRAP | 11171 | | | |
| 150 | SURF4 | 6836 | | | |
| 151 | SUSD3 | 203328 | | | |
| 152 | TAPBPL | 55080 | | | |
| 153 | TARS | 6897 | | | |
| 154 | TBC1D10C | 374403 | | | |
| 155 | THG1L | 54974 | | | |
| 156 | TMEM229B | 161145 | | | |
| 157 | TMEM65 | 157378 | | | |
| 158 | TMEM69 | 51249 | | | |
| 159 | TNFRSF14 | 8764 | | | |
| 160 | TNIP1 | 10318 | | | |

**20.** The prediction method according to Claim 18, wherein the biomarkers that are extracted as a third subpopulation when a discriminator is generated include FOXM1, CPT1A, GARS, MARS, UTP23, ANLN, HMGB3, ATP5B, APOOL, CYB561, GRHL2, ESRP1, EZR, RBBP8, CIRBP, PTGER3, LAMA3, OARD1, ANKRD29, EGR3, DIRAS3, MITD1 and LAMB3, and the measured value of the biomarker is the expression level of an mRNA or protein derived from the gene.

**21.** A patient survival rate prediction apparatus comprising a processing unit, wherein the processing unit is for executing the prediction method according to any one of Claims 13 to 20.

**22.** A method for assisting stratification of patients according to their survival rates, comprising the steps of:

comparing weighed sums calculated for the patients using a discriminator generated by the method for generating a discriminator according to any one of Claims 1 to 8 with corresponding reference ranges, and
determining bins of the reference ranges to which the weighed sums of the patients belong.

**23.** The method according to Claim 22, wherein the patients are cancer patients.

**24.** The method according to Claim 22 or 23, wherein the reference ranges have been determined according to a category such as the clinical stage classification of cancer, the histological type, clinical stage or pathological tissue grade of cancer, or the age group, and
wherein the method further comprises a step of acquiring information on the clinical stage classification of the cancer of the test subject, the histological type, clinical stage or pathological tissue grade of the cancer, and the age group of the test subject according to the category.

**25.** Use of at least one selected from the genes shown in Table 2-1 and Table 2-2 below (with the proviso that an aspect consisting only of ANLN, FOXM1, RBBP8 or a combination of these genes is not included) as a biomarker for predicting a survival rate of a breast cancer patient;

[Table 2-1]

| | Gene name | NCBI Gene ID | | Gene name | NCBI Gene ID | | Gene name | NCBI Gene ID |
|---|---|---|---|---|---|---|---|---|
| 1 | ADHFE1 | 137872 | 41 | DCAF16 | 54876 | 81 | LEF1 | 51176 |
| 2 | AMD1 | 262 | 42 | DCTPP1 | 79077 | 82 | LEPROTL1 | 23484 |
| 3 | ANKRD29 | 147463 | 43 | DEF6 | 50619 | 83 | LRP11 | 84918 |
| 4 | ANLN | 54443 | 44 | DERL1 | 79139 | 84 | LTF | 4057 |
| 5 | AP5S1 | 55317 | 45 | DHRS1 | 115817 | 85 | MAL2 | 114569 |
| 6 | APOBEC3F | 200316 | 46 | DIRAS3 | 9077 | 86 | MAP4K1 | 11184 |
| 7 | APOBEC3G | 60489 | 47 | EEF1G | 1937 | 87 | MAPT | 4137 |
| 8 | APOL3 | 80833 | 48 | EGR3 | 1960 | 88 | MARS | 4141 |
| 9 | APOOL | 139322 | 49 | ENOSF1 | 55556 | 89 | MAX | 4149 |
| 10 | ARHGAP26 | 23092 | 50 | ESRP1 | 54845 | 90 | MITD1 | 129531 |
| 11 | ARMC1 | 55156 | 51 | EVA1C | 59271 | 91 | MMGT1 | 93380 |
| 12 | ATP5B | 506 | 52 | EZR | 7430 | 92 | MRPL13 | 28998 |
| 13 | ATP6V1C1 | 528 | 53 | FAXDC2 | 10826 | 93 | MTDH | 92140 |
| 14 | BEND5 | 79656 | 54 | FKBP5 | 2289 | 94 | MTFR1 | 9650 |
| 15 | BTF3 | 689 | 55 | FOXM1 | 2305 | 95 | NDUFAF6 | 137682 |
| 16 | BTG1 | 694 | 56 | GARS | 2617 | 96 | NEFH | 4744 |
| 17 | C1RL | 51279 | 57 | GRHL2 | 79977 | 97 | NFKBIA | 4792 |
| 18 | CARD10 | 29775 | 58 | GSTM4 | 2948 | 98 | NKAP | 79576 |
| 19 | CASP9 | 842 | 59 | GSTO1 | 9446 | 99 | NONO | 4841 |
| 20 | CCDC115 | 84317 | 60 | GTF2IRD2B | 389524 | 100 | NR1H3 | 10062 |
| 21 | CCDC24 | 149473 | 61 | H6PD | 9563 | 101 | NSA2 | 10412 |
| 22 | CCDC69 | 26112 | 62 | HCCS | 3052 | 102 | NSMCE2 | 286053 |
| 23 | CCNE1 | 898 | 63 | HILPDA | 29923 | 103 | OARD1 | 221443 |
| 24 | CCT2 | 10576 | 64 | HMGB3 | 3149 | 104 | PARG | 8505 |
| 25 | CCT4 | 10575 | 65 | HNRNPA1L2 | 144983 | 105 | PARP3 | 10039 |
| 26 | CCT6A | 908 | 66 | HSP90AA1 | 3320 | 106 | PCMT1 | 5110 |
| 27 | CD24 | 100133941 | 67 | HSPA2 | 3306 | 107 | PDCD2 | 5134 |
| 28 | CD48 | 962 | 68 | IARS | 3376 | 108 | PGK1 | 5230 |
| 29 | CD52 | 1043 | 69 | IGF2R | 3482 | 109 | PHF7 | 51533 |
| 30 | CD6 | 923 | 70 | IP6K2 | 51447 | 110 | PLAT | 5327 |
| 31 | CD74 | 972 | 71 | IRF2 | 3660 | 111 | PLEKHA4 | 57664 |
| 32 | CDK8 | 1024 | 72 | ITGA10 | 8515 | 112 | PLXNB3 | 5365 |
| 33 | CIR1 | 9541 | 73 | JAK1 | 3716 | 113 | PNRC2 | 55629 |
| 34 | CIRBP | 1153 | 74 | KANSL2 | 54934 | 114 | POP1 | 10940 |
| 35 | COG8 | 84342 | 75 | KDM4B | 23030 | 115 | PRKAB2 | 5565 |
| 36 | COPS6 | 10980 | 76 | KIF21A | 55605 | 116 | PSMD10 | 5716 |
| 37 | CPT1A | 1374 | 77 | KRCC1 | 51315 | 117 | PSME1 | 5720 |
| 38 | CRK | 1398 | 78 | LAMA3 | 3909 | 118 | PTGER3 | 5733 |

(continued)

| | Gene name | NCBI Gene ID | | Gene name | NCBI Gene ID | | Gene name | NCBI Gene ID |
|---|---|---|---|---|---|---|---|---|
| 39 | CYB561 | 1534 | 79 | LAMB3 | 3914 | 119 | PTK2 | 5747 |
| 40 | CYP27A1 | 1593 | 80 | LARP4B | 23185 | 120 | PYCARD | 29108 |

[Table 2-2]

| | Gene name | NCBI Gene ID | | Gene name | NCBI Gene ID |
|---|---|---|---|---|---|
| 121 | QPRT | 23475 | 161 | TPRG1 | 285386 |
| 122 | RAB2A | 5862 | 162 | TPT1 | 7178 |
| 123 | RAC2 | 5880 | 163 | TRAF5 | 7188 |
| 124 | RAD1 | 5810 | 164 | TRAPPC12 | 51112 |
| 125 | RAPGEFL1 | 51195 | 165 | TRIM34 | 53840 |
| 126 | RARRES3 | 5920 | 166 | TRIM35 | 23087 |
| 127 | RBBP8 | 5932 | 167 | TRIM45 | 80263 |
| 128 | REPS1 | 85021 | 168 | TVP23C | 201158 |
| 129 | RILPL2 | 196383 | 169 | UBA7 | 7318 |
| 130 | RPF1 | 80135 | 170 | UBE2A | 7319 |
| 131 | RPL11 | 6135 | 171 | UBTF | 7343 |
| 132 | RPL14 | 9045 | 172 | UBXN1 | 51035 |
| 133 | RPS6KA1 | 6195 | 173 | UBXN11 | 91544 |
| 134 | RTN4IP1 | 84816 | 174 | UTP23 | 84294 |
| 135 | SAV1 | 60485 | 175 | VBP1 | 7411 |
| 136 | SCUBE2 | 57758 | 176 | WLS | 79971 |
| 137 | SERPINA1 | 5265 | 177 | YTH DF3 | 253943 |
| 138 | SH3D21 | 79729 | 178 | ZBED6CL | 113763 |
| 139 | SHISA5 | 51246 | 179 | ZDHHC9 | 51114 |
| 140 | SKP1 | 6500 | 180 | ZFHX3 | 463 |
| 141 | SLC35A2 | 7355 | 181 | ZMYND10 | 51364 |
| 142 | SLC7A5 | 8140 | 182 | ZNF496 | 84838 |
| 143 | SPATA7 | 55812 | 183 | ZNF552 | 79818 |
| 144 | SPOCK2 | 9806 | 184 | ZNF671 | 79891 |

(continued)

| | Gene name | NCBI Gene ID | | Gene name | NCBI Gene ID |
|---|---|---|---|---|---|
| 145 | STC2 | 8614 | | | |
| 146 | STEAP2 | 261729 | | | |
| 147 | STK3 | 6788 | | | |
| 148 | STK39 | 27347 | | | |
| 149 | STRAP | 11171 | | | |
| 150 | SURF4 | 6836 | | | |
| 151 | SUSD3 | 203328 | | | |
| 152 | TAPBPL | 55080 | | | |
| 153 | TARS | 6897 | | | |
| 154 | TBC1D10C | 374403 | | | |
| 155 | THG1L | 54974 | | | |
| 156 | TMEM229B | 161145 | | | |
| 157 | TMEM65 | 157378 | | | |
| 158 | TMEM69 | 51249 | | | |
| 159 | TNFRSF14 | 8764 | | | |
| 160 | TNIP1 | 10318 | | | |

**26.** Use of at least one selected from the gene group consisting of FOXM1, CPT1A, GARS, MARS, UTP23, ANLN, HMGB3, ATP5B, APOOL, CYB561, GRHL2, ESRP1, EZR, RBBP8, CIRBP, PTGER3, LAMA3, OARD1, ANKRD29, EGR3, DIRAS3, MITD1 and LAMB3 (with the proviso that an aspect consisting only of ANLN, FOXM1, RBBP8 or a combination of these genes is not included) as a biomarker for predicting a survival rate of a breast cancer patient.

**27.** An inspection reagent for use in predicting a survival rate of a breast cancer patient, comprising a probe, primer or antibody for detecting the expression level of an mRNA or protein derived from at least one selected from the genes shown in Table 3-1 and Table 3-2 below (with the proviso that an aspect consisting only of ANLN, FOXM1, RBBP8 or a combination of these genes is not included);

[Table 3-1]

| | Gene name | NCBI Gene ID | | Gene name | NCBI Gene ID | | Gene name | NCBI Gene ID |
|---|---|---|---|---|---|---|---|---|
| 1 | ADHFE1 | 137872 | 41 | DCAF16 | 54876 | 81 | LEF1 | 51176 |
| 2 | AMD1 | 262 | 42 | DCTPP1 | 79077 | 82 | LEPROTL1 | 23484 |
| 3 | ANKRD29 | 147463 | 43 | DEF6 | 50619 | 83 | LRP11 | 84918 |
| 4 | ANLN | 54443 | 44 | DERL1 | 79139 | 84 | LTF | 4057 |
| 5 | AP5S1 | 55317 | 45 | DHRS1 | 115817 | 85 | MAL2 | 114569 |
| 6 | APOBEC3F | 200316 | 46 | DIRAS3 | 9077 | 86 | MAP4K1 | 11184 |
| 7 | APOBEC3G | 60489 | 47 | EEF1G | 1937 | 87 | MAPT | 4137 |
| 8 | APOL3 | 80833 | 48 | EGR3 | 1960 | 88 | MARS | 4141 |
| 9 | APOOL | 139322 | 49 | ENOSF1 | 55556 | 89 | MAX | 4149 |
| 10 | ARHGAP26 | 23092 | 50 | ESRP1 | 54845 | 90 | MITD1 | 129531 |
| 11 | ARMC1 | 55156 | 51 | EVA1C | 59271 | 91 | MMGT1 | 93380 |
| 12 | ATP5B | 506 | 52 | EZR | 7430 | 92 | MRPL13 | 28998 |

(continued)

| | Gene name | NCBI Gene ID | | Gene name | NCBI Gene ID | | Gene name | NCBI Gene ID |
|---|---|---|---|---|---|---|---|---|
| 13 | ATP6V1C1 | 528 | 53 | FAXDC2 | 10826 | 93 | MTDH | 92140 |
| 14 | BEND5 | 79656 | 54 | FKBP5 | 2289 | 94 | MTFR1 | 9650 |
| 15 | BTF3 | 689 | 55 | FOXM1 | 2305 | 95 | NDUFAF6 | 137682 |
| 16 | BTG1 | 694 | 56 | GARS | 2617 | 96 | NEFH | 4744 |
| 17 | C1RL | 51279 | 57 | GRHL2 | 79977 | 97 | NFKBIA | 4792 |
| 18 | CARD10 | 29775 | 58 | GSTM4 | 2948 | 98 | NKAP | 79576 |
| 19 | CASP9 | 842 | 59 | GSTO1 | 9446 | 99 | NONO | 4841 |
| 20 | CCDC115 | 84317 | 60 | GTF2IRD2B | 389524 | 100 | NR1H3 | 10062 |
| 21 | CCDC24 | 149473 | 61 | H6PD | 9563 | 101 | NSA2 | 10412 |
| 22 | CCDC69 | 26112 | 62 | HCCS | 3052 | 102 | NSMCE2 | 286053 |
| 23 | CCNE1 | 898 | 63 | HILPDA | 29923 | 103 | OARD1 | 221443 |
| 24 | CCT2 | 10576 | 64 | HMGB3 | 3149 | 104 | PARG | 8505 |
| 25 | CCT4 | 10575 | 65 | HNRNPA1L2 | 144983 | 105 | PARP3 | 10039 |
| 26 | CCT6A | 908 | 66 | HSP90AA1 | 3320 | 106 | PCMT1 | 5110 |
| 27 | CD24 | 100133941 | 67 | HSPA2 | 3306 | 107 | PDCD2 | 5134 |
| 28 | CD48 | 962 | 68 | IARS | 3376 | 108 | PGK1 | 5230 |
| 29 | CD52 | 1043 | 69 | IGF2R | 3482 | 109 | PHF7 | 51533 |
| 30 | CD6 | 923 | 70 | IP6K2 | 51447 | 110 | PLAT | 5327 |
| 31 | CD74 | 972 | 71 | IRF2 | 3660 | 111 | PLEKHA4 | 57664 |
| 32 | CDK8 | 1024 | 72 | ITGA10 | 8515 | 112 | PLXNB3 | 5365 |
| 33 | CIR1 | 9541 | 73 | JAK1 | 3716 | 113 | PNRC2 | 55629 |
| 34 | CIRBP | 1153 | 74 | KANSL2 | 54934 | 114 | POP1 | 10940 |
| 35 | COG8 | 84342 | 75 | KDM4B | 23030 | 115 | PRKAB2 | 5565 |
| 36 | COPS6 | 10980 | 76 | KIF21A | 55605 | 116 | PSMD10 | 5716 |
| 37 | CPT1A | 1374 | 77 | KRCC1 | 51315 | 117 | PSME1 | 5720 |
| 38 | CRK | 1398 | 78 | LAMA3 | 3909 | 118 | PTGER3 | 5733 |
| 39 | CYB561 | 1534 | 79 | LAMB3 | 3914 | 119 | PTK2 | 5747 |
| 40 | CYP27A1 | 1593 | 80 | LARP4B | 23185 | 120 | PYCARD | 29108 |

[Table 3-2]

| | Gene name | NCBI Gene ID | | Gene name | NCBI Gene ID |
|---|---|---|---|---|---|
| 121 | QPRT | 23475 | 161 | TPRG1 | 285386 |
| 122 | RAB2A | 5862 | 162 | TPT1 | 7178 |
| 123 | RAC2 | 5880 | 163 | TRAF5 | 7188 |
| 124 | RAD1 | 5810 | 164 | TRAPPC12 | 51112 |
| 125 | RAPGEFL1 | 51195 | 165 | TRIM34 | 53840 |
| 126 | RARRES3 | 5920 | 166 | TRIM35 | 23087 |

(continued)

| | Gene name | NCBI Gene ID | | Gene name | NCBI Gene ID |
|---|---|---|---|---|---|
| 127 | RBBP8 | 5932 | 167 | TRIM45 | 80263 |
| 128 | REPS1 | 85021 | 168 | TVP23C | 201158 |
| 129 | RILPL2 | 196383 | 169 | UBA7 | 7318 |
| 130 | RPF1 | 80135 | 170 | UBE2A | 7319 |
| 131 | RPL11 | 6135 | 171 | UBTF | 7343 |
| 132 | RPL14 | 9045 | 172 | UBXN1 | 51035 |
| 133 | RPS6KA1 | 6195 | 173 | UBXN11 | 91544 |
| 134 | RTN4IP1 | 84816 | 174 | UTP23 | 84294 |
| 135 | SAV1 | 60485 | 175 | VBP1 | 7411 |
| 136 | SCUBE2 | 57758 | 176 | WLS | 79971 |
| 137 | SERPINA1 | 5265 | 177 | YTH DF3 | 253943 |
| 138 | SH3D21 | 79729 | 178 | ZBED6CL | 113763 |
| 139 | SHISA5 | 51246 | 179 | ZDHHC9 | 51114 |
| 140 | SKP1 | 6500 | 180 | ZFHX3 | 463 |
| 141 | SLC35A2 | 7355 | 181 | ZMYND10 | 51364 |
| 142 | SLC7A5 | 8140 | 182 | ZNF496 | 84838 |
| 143 | SPATA7 | 55812 | 183 | ZNF552 | 79818 |
| 144 | SPOCK2 | 9806 | 184 | ZNF671 | 79891 |
| 145 | STC2 | 8614 | | | |
| 146 | STEAP2 | 261729 | | | |
| 147 | STK3 | 6788 | | | |
| 148 | STK39 | 27347 | | | |
| 149 | STRAP | 11171 | | | |
| 150 | SURF4 | 6836 | | | |
| 151 | SUSD3 | 203328 | | | |
| 152 | TAPBPL | 55080 | | | |
| 153 | TARS | 6897 | | | |
| 154 | TBC1D10C | 374403 | | | |
| 155 | THG1L | 54974 | | | |
| 156 | TMEM229B | 161145 | | | |
| 157 | TMEM65 | 157378 | | | |
| 158 | TMEM69 | 51249 | | | |
| 159 | TNFRSF14 | 8764 | | | |
| 160 | TNIP1 | 10318 | | | |

**28.** An inspection reagent for use in predicting a survival rate of a breast cancer patient, comprising a probe, primer, or antibody for detecting the expression level of an mRNA or protein derived from at least one selected from a gene group consisting of FOXM1, CPT1A, GARS, MARS, UTP23, ANLN, HMGB3, ATP5B, APOOL, CYB561, GRHL2, ESRP1, EZR, RBBP8, CIRBP, PTGER3, LAMA3, OARD1, ANKRD29, EGR3, DIRAS3, MITD1 and LAMB3 (with the proviso that an aspect consisting only of ANLN, FOXM1, RBBP8 or a combination of these genes is not included).

**29.** A discriminator generated by the method for generating a discriminator according to any one of Claims 1 to 8.

**30.** A computer readable storage medium in which the discriminator according to Claim 29 has been stored.

**31.** A computer program for generating a discriminator, which executes the method for generating a discriminator according to any one of Claims 1 to 8 when executed by a computer.

**32.** A computer program for predicting a survival rate of a patient, which executes the prediction method according to any one of Claims 13 to 20 when executed by a computer.

FIG. 1

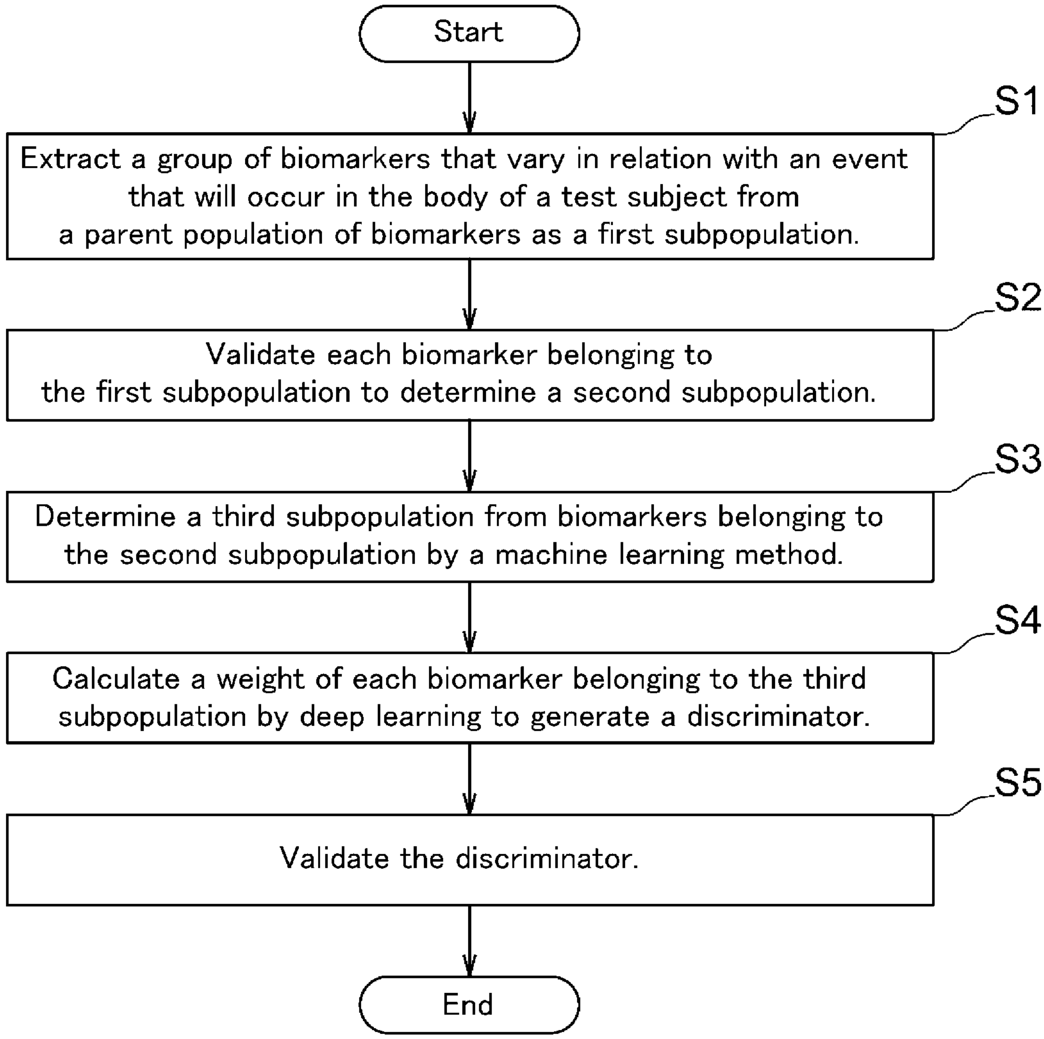
Start
S1
Extract a group of biomarkers that vary in relation with an event that will occur in the body of a test subject from a parent population of biomarkers as a first subpopulation.
S2
Validate each biomarker belonging to the first subpopulation to determine a second subpopulation.
S3
Determine a third subpopulation from biomarkers belonging to the second subpopulation by a machine learning method.
S4
Calculate a weight of each biomarker belonging to the third subpopulation by deep learning to generate a discriminator.
S5
Validate the discriminator.
End

FIG. 2

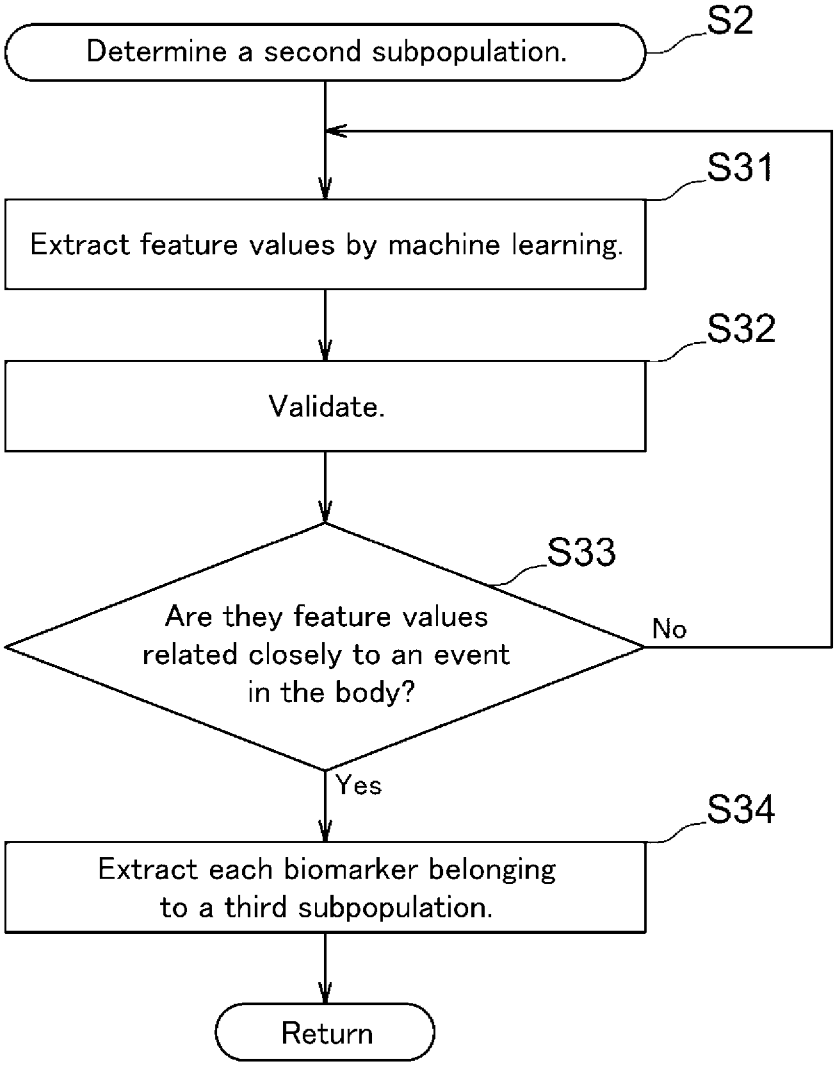
S2
Determine a second subpopulation.
S31
Extract feature values by machine learning.
S32
Validate.
S33
Are they feature values related closely to an event in the body?
No
Yes
S34
Extract each biomarker belonging to a third subpopulation.
Return

FIG. 3

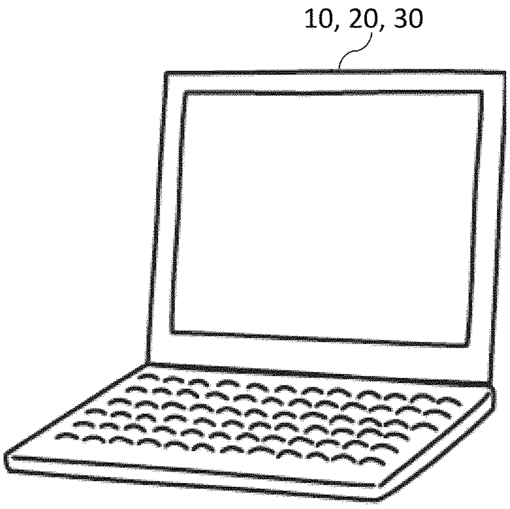
10, 20, 30

FIG. 4

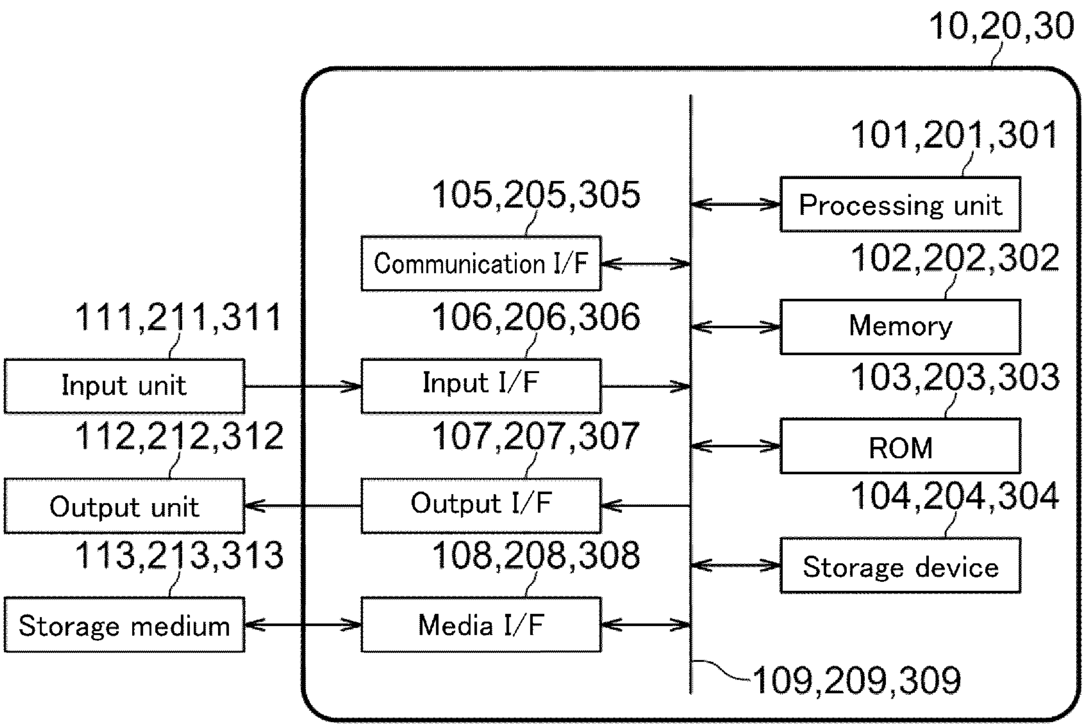
10,20,30
101,201,301
Processing unit
105,205,305
Communication I/F
102,202,302
Memory
111,211,311
106,206,306
Input unit
Input I/F
103,203,303
112,212,312
107,207,307
ROM
Output unit
Output I/F
104,204,304
113,213,313
108,208,308
Storage device
Storage medium
Media I/F
109,209,309

FIG. 5

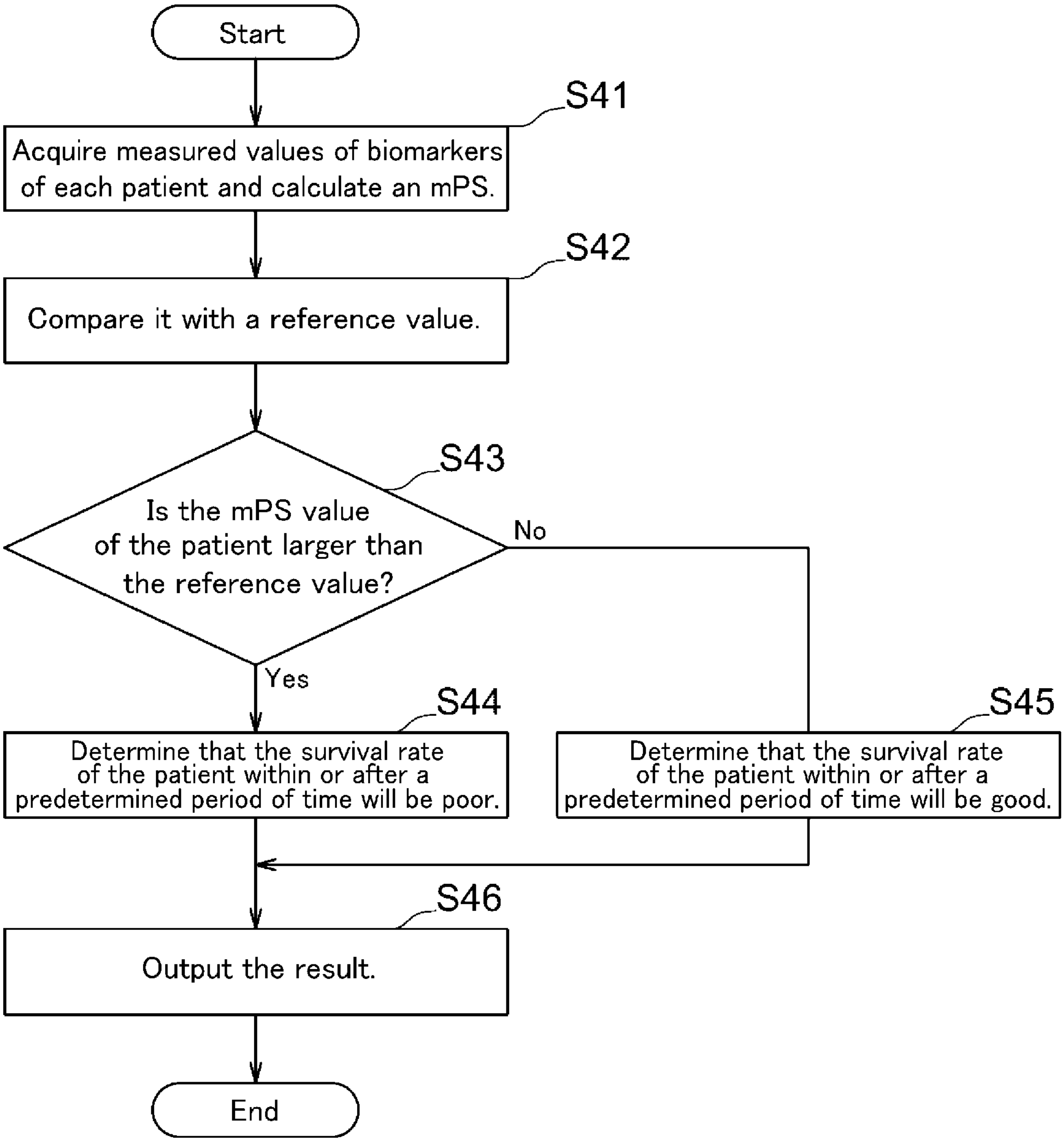
Start
S41
Acquire measured values of biomarkers of each patient and calculate an mPS.
S42
Compare it with a reference value.
S43
Is the mPS value of the patient larger than the reference value?
No
Yes
S44
Determine that the survival rate of the patient within or after a predetermined period of time will be poor.
S45
Determine that the survival rate of the patient within or after a predetermined period of time will be good.
S46
Output the result.
End

FIG. 6

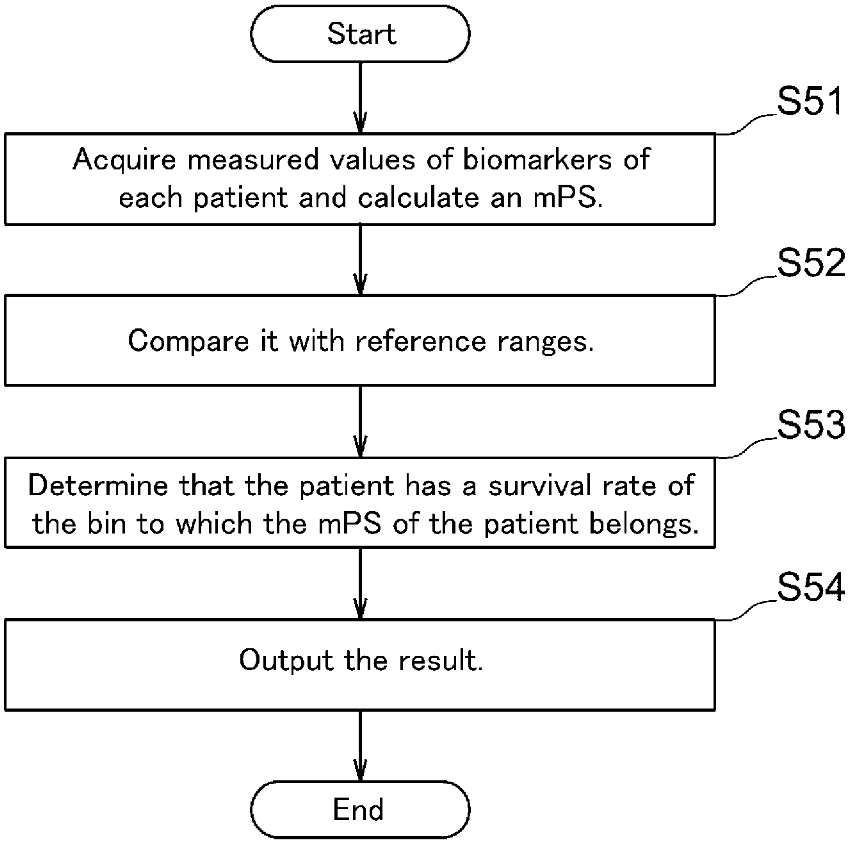
Start
S51
Acquire measured values of biomarkers of each patient and calculate an mPS.
S52
Compare it with reference ranges.
S53
Determine that the patient has a survival rate of the bin to which the mPS of the patient belongs.
S54
Output the result.
End

FIG. 7

| Processing | Processing details | Data set |
|---|---|---|
| 1. Extraction of candidate genes<br><br>2. Validation of candidate genes | 1. Plot a survival curve for 10 years for all coding genes by a Kaplan-Meier Plot.<br>2. Extract candidate genes possibly related to a poor survival rate based on the p-value of a Log-rank test (286 genes).<br>3. Validate each candidate gene by a meta-analysis.<br>4. Determine 184 genes as candidates for prognostic genes. | Discovery cohort<br>→TCGA CNAs cohort (n=958)<br><br>Validation cohort<br>→multicenter combined breast cancer cohorts(n=5844) |
| 3. Extraction of feature values by machine learning | Extract a combination of genes predicted to be related to each other in terms of a survival rate out of the 184 prognostic genes by a random forest and a cross-validation using training data (23 genes). | Training data<br>→A cohort extracted at random from the METABRIC cohort (n=952) |
| 4. Construction of a discriminator by deep learning | 1. Construct a discriminator (model) for obtaining a molecular prognostic score (mPS).<br>Define a Gene score as a binary value of expression.<br>mPS=a weighed sum of Gene scores of each gene<br>2. Define a loss function of each gene by a cross entropy error using training data.<br>3. Calculate a weight of each gene by a gradient descent method.<br>4. Construct a discriminator.<br>$mPS=w_1GS_1+w_2GS_2+.......w_{23}GS_{23}$ | Training data<br>→A cohort extracted at random from the METABRIC cohort (n=952) |
| 5. Validation of discriminator | Generalization ability<br><br>Versatile ability<br><br>Application potentiality | Whole METABRIC cohort (n=1904)& TCGA CNAs cohort (n=958)<br><br>METABRIC (age, intrinsic subtype, pathological tissue classification, disease stage)<br><br>Whole METABRIC cohort(n=1904)& TCGA CNAs cohort(n=958) |

FIG. 8

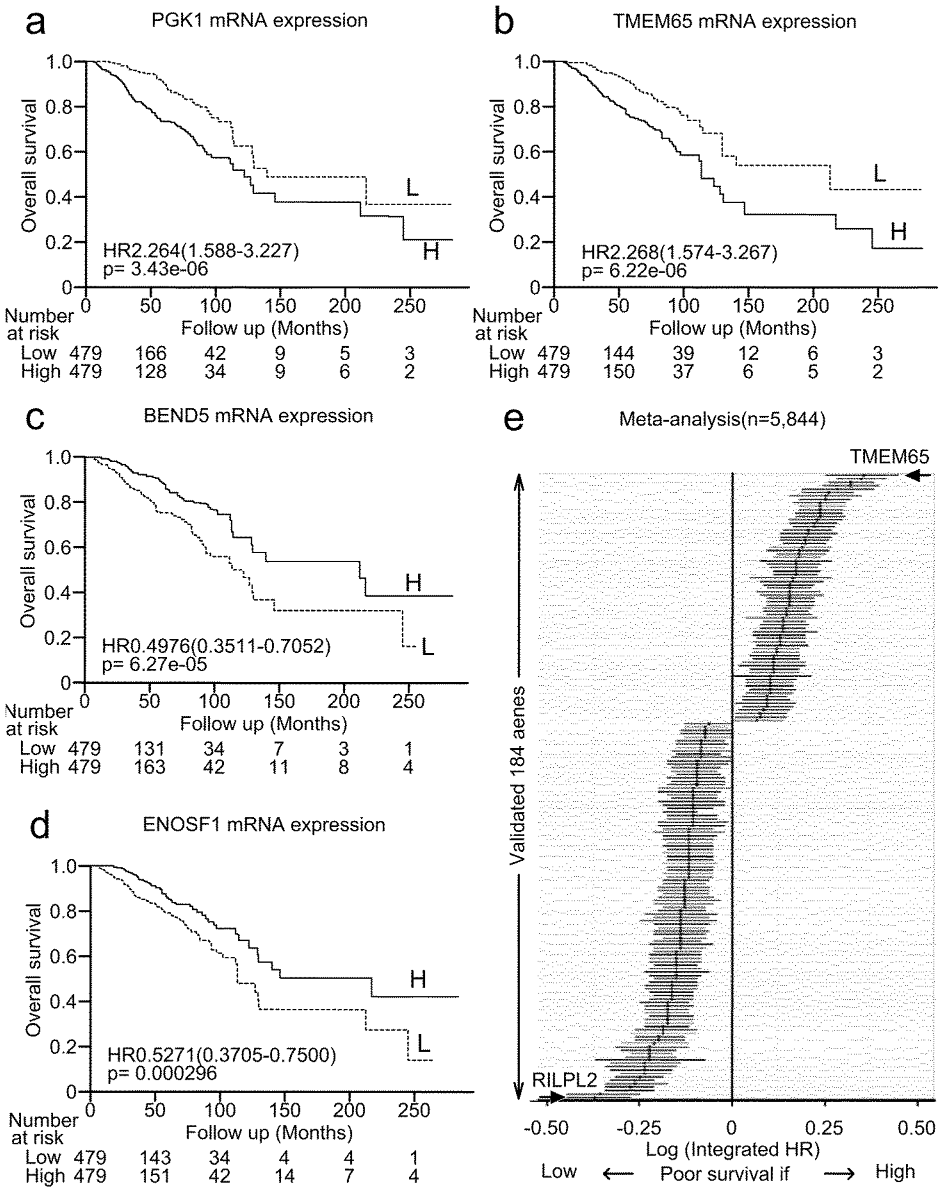
a
PGK1 mRNA expression
Overall survival
HR2.264(1.588-3.227)
p= 3.43e-06
L
H
Number at risk
Follow up (Months)
Low 479 166 42 9 5 3
High 479 128 34 9 6 2
b
TMEM65 mRNA expression
Overall survival
HR2.268(1.574-3.267)
p= 6.22e-06
L
H
Number at risk
Follow up (Months)
Low 479 144 39 12 6 3
High 479 150 37 6 5 2
c
BEND5 mRNA expression
Overall survival
HR0.4976(0.3511-0.7052)
p= 6.27e-05
H
L
Number at risk
Follow up (Months)
Low 479 131 34 7 3 1
High 479 163 42 11 8 4
d
ENOSF1 mRNA expression
Overall survival
HR0.5271(0.3705-0.7500)
p= 0.000296
H
L
Number at risk
Follow up (Months)
Low 479 143 34 4 4 1
High 479 151 42 14 7 4
e
Meta-analysis(n=5,844)
TMEM65
RILPL2
Validated 184 aenes
Log (Integrated HR)
Low ⟵ Poor survival if ⟶ High

[FIG. 9-1]

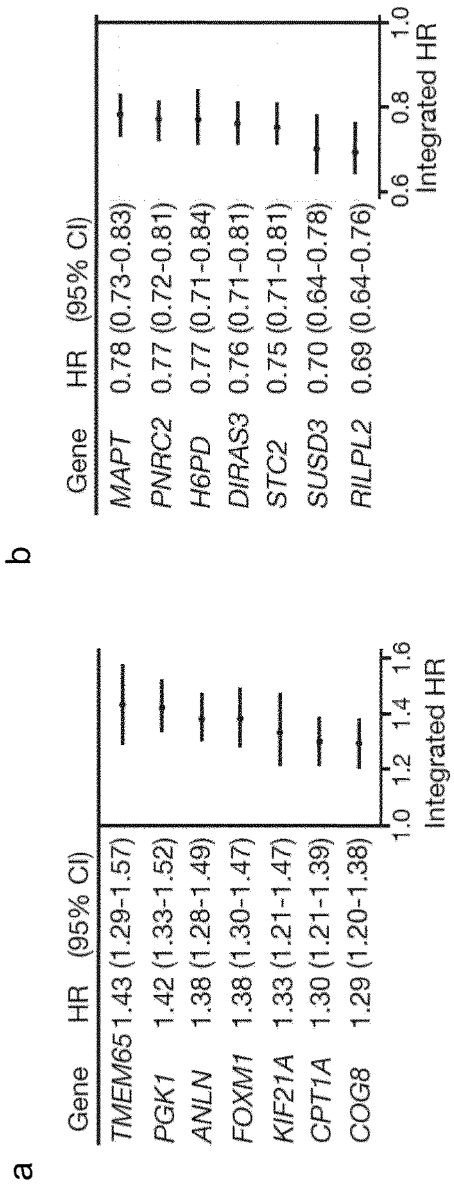
a
Gene HR (95% CI)
TMEM65 1.43 (1.29-1.57)
PGK1 1.42 (1.33-1.52)
ANLN 1.38 (1.28-1.49)
FOXM1 1.38 (1.30-1.47)
KIF21A 1.33 (1.21-1.47)
CPT1A 1.30 (1.21-1.39)
COG8 1.29 (1.20-1.38)
1.0 1.2 1.4 1.6
Integrated HR
b
Gene HR (95% CI)
MAPT 0.78 (0.73-0.83)
PNRC2 0.77 (0.72-0.81)
H6PD 0.77 (0.71-0.84)
DIRAS3 0.76 (0.71-0.81)
STC2 0.75 (0.71-0.81)
SUSD3 0.70 (0.64-0.78)
RILPL2 0.69 (0.64-0.76)
0.6 0.8 1.0
Integrated HR

[FIG. 9-2]

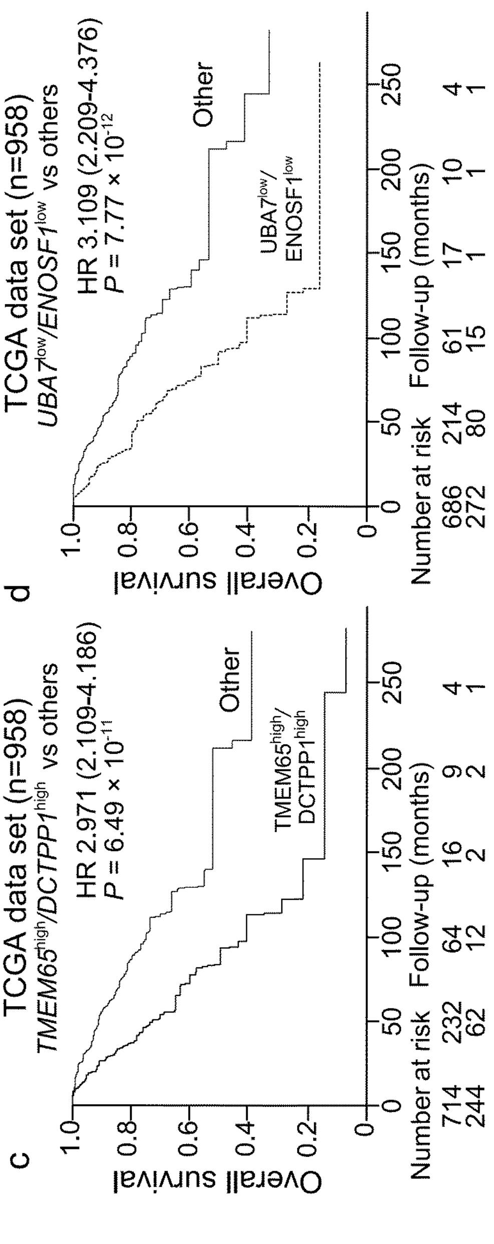
c
TCGA data set (n=958)
TMEM65high/DCTPP1high vs others
HR 2.971 (2.109-4.186)
P = 6.49 × 10-11
Other
TMEM65high/
DCTPP1high
Overall survival
1.0
0.8
0.6
0.4
0.2
0
0
50
100
150
200
250
Follow-up (months)
Number at risk
714 232 64 16 9 4
244 62 12 2 2 1
d
TCGA data set (n=958)
UBA7low/ENOSF1low vs others
HR 3.109 (2.209-4.376)
P = 7.77 × 10-12
Other
UBA7low/
ENOSF1low
Overall survival
1.0
0.8
0.6
0.4
0.2
0
0
50
100
150
200
250
Follow-up (months)
Number at risk
686 214 61 17 10 4
272 80 15 1 1 1

[FIG. 10-1]

| Gene | HR | 95% CI lower | 95% CI upper |
|---|---|---|---|
| PGK1 | 1.42 | 1.33 | 1.52 |
| UBA7 | 0.87 | 0.79 | 0.96 |
| TMEM65 | 1.43 | 1.29 | 1.57 |
| PCMT1 | 1.14 | 1.07 | 1.22 |
| BEND5 | 0.85 | 0.8 | 0.9 |
| QPRT | 1.22 | 1.14 | 1.3 |
| GRHL2 | 1.11 | 1.04 | 1.18 |
| TNIP1 | 0.88 | 0.83 | 0.93 |
| DERL1 | 1.23 | 1.14 | 1.33 |
| RTN4IP1 | 1.12 | 1.02 | 1.22 |
| SAV1 | 0.86 | 0.81 | 0.92 |
| ENOSF1 | 0.91 | 0.84 | 0.98 |
| HSP90AA1 | 1.19 | 1.12 | 1.27 |
| RARRES3 | 0.84 | 0.79 | 0.89 |
| CD24 | 1.1 | 1.03 | 1.18 |
| CCDC115 | 0.87 | 0.8 | 0.95 |
| COG8 | 1.29 | 1.2 | 1.38 |
| TNFRSF14 | 0.84 | 0.79 | 0.9 |
| IRF2 | 0.91 | 0.86 | 0.97 |
| CCNE1 | 1.13 | 1.06 | 1.2 |
| PLEKHA4 | 0.88 | 0.82 | 0.94 |
| TRIM45 | 0.84 | 0.78 | 0.89 |
| TAPBPL | 0.85 | 0.8 | 0.91 |
| EGR3 | 0.85 | 0.8 | 0.9 |
| PLAT | 0.88 | 0.83 | 0.94 |
| NFKBIA | 0.91 | 0.85 | 0.96 |
| UTP23 | 1.19 | 1.08 | 1.31 |
| MAPT | 0.78 | 0.73 | 0.83 |
| TRIM34 | 0.87 | 0.81 | 0.94 |
| GARS | 1.25 | 1.18 | 1.32 |
| PARP3 | 0.8 | 0.76 | 0.85 |
| ZFHX3 | 1.07 | 1 | 1.15 |
| SCUBE2 | 0.84 | 0.79 | 0.89 |
| LRP11 | 1.29 | 1.17 | 1.42 |
| HCCS | 1.27 | 1.19 | 1.36 |
| PYCARD | 0.87 | 0.82 | 0.93 |
| DCTPP1 | 1.12 | 1.05 | 1.2 |
| CCDC24 | 0.9 | 0.82 | 0.99 |
| RPL14 | 0.89 | 0.83 | 0.95 |
| ZDHHC9 | 1.15 | 1.04 | 1.26 |
| MARS | 1.27 | 1.2 | 1.35 |
| KDM4B | 0.85 | 0.79 | 0.91 |
| RAB2A | 1.19 | 1.1 | 1.27 |
| PSMD10 | 1.15 | 1.08 | 1.23 |
| RAC2 | 0.86 | 0.8 | 0.92 |
| CASP9 | 0.87 | 0.81 | 0.93 |
| UBXN1 | 0.89 | 0.83 | 0.96 |
| ZNF671 | 0.87 | 0.82 | 0.93 |
| APOBEC3G | 0.91 | 0.84 | 0.98 |
| NDUFAF6 | 1.18 | 1.05 | 1.31 |
| NKAP | 1.17 | 1.07 | 1.27 |
| ATP5F1B | 1.16 | 1.09 | 1.23 |
| CD52 | 0.93 | 0.88 | 1 |
| WLS | 0.92 | 0.86 | 0.98 |

[FIG. 10-2]

| Gene | HR | 95% CI lower | 95% CI upper |
|---|---|---|---|
| MTDH | 1.2 | 1.13 | 1.29 |
| SPOCK2 | 0.88 | 0.82 | 0.94 |
| UBE2A | 1.22 | 1.14 | 1.31 |
| HSPA2 | 0.8 | 0.75 | 0.86 |
| TPT1 | 0.79 | 0.71 | 0.86 |
| DIRAS3 | 0.76 | 0.71 | 0.81 |
| C1RL | 0.85 | 0.81 | 0.9 |
| SH3D21 | 0.93 | 0.88 | 0.99 |
| ARMC1 | 1.17 | 1.1 | 1.25 |
| RILPL2 | 0.69 | 0.64 | 0.76 |
| ATP6V1C1 | 1.12 | 1.05 | 1.2 |
| STC2 | 0.75 | 0.71 | 0.81 |
| PHF7 | 0.87 | 0.81 | 0.94 |
| GSTM4 | 0.84 | 0.8 | 0.9 |
| SLC35A2 | 1.14 | 1.06 | 1.22 |
| MAX | 0.89 | 0.83 | 0.95 |
| CCT6A | 1.19 | 1.12 | 1.26 |
| PLXNB3 | 1.1 | 1.03 | 1.17 |
| MMGT1 | 1.1 | 1.01 | 1.19 |
| SUSD3 | 0.7 | 0.64 | 0.78 |
| DCAF16 | 0.9 | 0.84 | 0.96 |
| MITD1 | 0.88 | 0.81 | 0.97 |
| NR1H3 | 0.9 | 0.84 | 0.95 |
| H6PD | 0.77 | 0.71 | 0.84 |
| AMD1 | 1.16 | 1.1 | 1.24 |
| PRKAB2 | 1.08 | 1.01 | 1.16 |
| MRPL13 | 1.19 | 1.11 | 1.26 |
| CPT1A | 1.3 | 1.21 | 1.39 |
| PTK2 | 1.11 | 1.04 | 1.19 |
| CIR1 | 0.87 | 0.83 | 0.93 |
| SERPINA1 | 0.8 | 0.74 | 0.86 |
| STK3 | 1.27 | 1.18 | 1.36 |
| KANSL2 | 1.17 | 1.1 | 1.25 |
| TPRG1 | 0.8 | 0.73 | 0.89 |
| CYB561 | 1.14 | 1.07 | 1.23 |
| CYP27A1 | 0.89 | 0.84 | 0.95 |
| ANKRD29 | 0.81 | 0.74 | 0.89 |
| HMGB3 | 1.27 | 1.19 | 1.35 |
| ITGA10 | 0.94 | 0.88 | 1 |
| HILPDA | 1.22 | 1.15 | 1.29 |
| KRCC1 | 0.89 | 0.84 | 0.95 |
| LTF | 0.86 | 0.81 | 0.91 |
| CD48 | 0.86 | 0.8 | 0.92 |
| STK39 | 0.9 | 0.85 | 0.96 |
| IARS | 1.2 | 1.12 | 1.27 |
| APOL3 | 0.84 | 0.79 | 0.9 |
| PARG | 1.11 | 1.04 | 1.18 |
| BTG1 | 0.84 | 0.79 | 0.9 |
| REPS1 | 0.92 | 0.85 | 1 |
| KIF21A | 1.33 | 1.21 | 1.47 |
| RAD1 | 1.21 | 1.11 | 1.31 |
| MAL2 | 1.17 | 1.08 | 1.28 |
| MTFR1 | 1.25 | 1.17 | 1.33 |
| TRAPPC12 | 0.88 | 0.83 | 0.94 |

[FIG. 10-3]

| Gene | HR | 95% CI lower | 95% CI upper |
|---|---|---|---|
| AP5S1 | 1.11 | 1.04 | 1.18 |
| RPF1 | 0.87 | 0.81 | 0.94 |
| CDK8 | 1.11 | 1.05 | 1.19 |
| HNRNPA1L2 | 0.8 | 0.69 | 0.93 |
| ZNF496 | 0.9 | 0.82 | 1 |
| SLC7A5 | 1.27 | 1.2 | 1.36 |
| PSME1 | 0.92 | 0.86 | 0.97 |
| APOBEC3F | 0.9 | 0.82 | 0.98 |
| MAP4K1 | 0.86 | 0.8 | 0.92 |
| PTGER3 | 0.79 | 0.72 | 0.85 |
| COPS6 | 1.16 | 1.09 | 1.24 |
| SURF4 | 1.26 | 1.17 | 1.35 |
| ESRP1 | 1.23 | 1.15 | 1.31 |
| CD74 | 0.88 | 0.83 | 0.93 |
| FKBP5 | 0.9 | 0.84 | 0.96 |
| ZNF552 | 0.89 | 0.83 | 0.95 |
| OARD1 | 0.93 | 0.87 | 1 |
| NONO | 1.08 | 1.01 | 1.16 |
| DHRS1 | 0.89 | 0.84 | 0.95 |
| EVA1C | 0.83 | 0.77 | 0.91 |
| SKP1 | 0.92 | 0.86 | 0.99 |
| TARS | 1.17 | 1.09 | 1.25 |
| TMEM69 | 0.91 | 0.84 | 0.99 |
| NEFH | 0.88 | 0.82 | 0.95 |
| CCT4 | 1.15 | 1.08 | 1.22 |
| NSA2 | 0.89 | 0.84 | 0.95 |
| ZBED6CL | 0.91 | 0.84 | 1 |
| RAPGEFL1 | 1.1 | 1.03 | 1.16 |
| CD6 | 0.86 | 0.79 | 0.94 |
| POP1 | 1.17 | 1.1 | 1.24 |
| RBBP8 | 0.86 | 0.8 | 0.92 |
| CARD10 | 0.91 | 0.85 | 0.98 |
| APOOL | 1.15 | 1.06 | 1.26 |
| CRK | 0.93 | 0.87 | 1 |
| ADHFE1 | 0.83 | 0.76 | 0.91 |
| CCT2 | 1.17 | 1.1 | 1.25 |
| UBXN11 | 0.89 | 0.81 | 0.98 |
| CCDC69 | 0.87 | 0.81 | 0.92 |
| EZR | 1.14 | 1.06 | 1.22 |
| EEF1G | 0.89 | 0.84 | 0.95 |
| NSMCE2 | 1.2 | 1.1 | 1.3 |
| LARP4B | 1.18 | 1.08 | 1.28 |
| GTF2IRD2B | 0.89 | 0.83 | 0.96 |
| STEAP2 | 0.9 | 0.82 | 0.98 |
| TVP23C | 0.79 | 0.72 | 0.87 |
| ANLN | 1.38 | 1.28 | 1.49 |
| PDCD2 | 0.9 | 0.83 | 0.98 |
| ARHGAP26 | 0.92 | 0.85 | 1 |
| FOXM1 | 1.38 | 1.3 | 1.47 |
| ZMYND10 | 0.82 | 0.77 | 0.88 |
| UBTF | 0.89 | 0.83 | 0.95 |
| BTF3 | 0.86 | 0.81 | 0.91 |
| LEF1 | 0.9 | 0.83 | 0.91 |
| PNRC2 | 0.77 | 0.72 | 0.81 |

[FIG. 10-4]

| Gene | HR | 95% CI lower | 95% CI upper |
|---|---|---|---|
| SHISA5 | 1.12 | 1.03 | 1.22 |
| IP6K2 | 0.87 | 0.81 | 0.93 |
| VBP1 | 1.15 | 1.09 | 1.22 |
| IGF2R | 1.09 | 1.02 | 1.17 |
| TRAF5 | 0.9 | 0.84 | 0.96 |
| FAXDC2 | 0.88 | 0.81 | 0.95 |
| YTHDF3 | 1.12 | 1.05 | 1.2 |
| THG1L | 0.91 | 0.85 | 0.97 |
| TMEM229B | 1.11 | 1 | 1.24 |
| CIRBP | 0.82 | 0.77 | 0.88 |
| GSTO1 | 1.13 | 1.06 | 1.2 |
| SPATA7 | 0.89 | 0.84 | 0.95 |
| TRIM35 | 0.9 | 0.82 | 0.97 |
| DEF6 | 0.89 | 0.83 | 0.95 |
| JAK1 | 0.79 | 0.74 | 0.85 |
| LEPROTL1 | 0.85 | 0.79 | 0.91 |
| STRAP | 1.12 | 1.06 | 1.2 |
| RPL11 | 0.83 | 0.79 | 0.89 |
| RPS6KA1 | 0.92 | 0.86 | 0.98 |
| LAMA3 | 0.86 | 0.8 | 0.92 |
| LAMB3 | 0.89 | 0.84 | 0.95 |
| TBC1D10C | 0.87 | 0.78 | 0.96 |

[FIG. 11-1]

a

| Symbol | Gene ID | Full name | Score (High) | Score (Low) | Weight |
|---|---|---|---|---|---|
| FOXM1 | 2305 | forkhead box M1 | 1 | 0 | 3.424 |
| CPT1A | 1374 | carnitine palmitoyltransferase 1A | 1 | 0 | 3.399 |
| GARS | 2617 | glycyl-tRNA synthetase | 1 | 0 | 2.539 |
| MARS | 4141 | methionyl-tRNA synthetase | 1 | 0 | 2.312 |
| UTP23 | 84294 | UTP23,small subunit processome component | 1 | 0 | 2.311 |
| ANLN | 54443 | anillin actin binding protein | 1 | 0 | 2.225 |
| HMGB3 | 3149 | high mobility group box 3 | 1 | 0 | 2.202 |
| ATP5B | 506 | ATP synthase,H+ transporting,mitochondrial F1 complex,beta polypeptid | 1 | 0 | 1.934 |
| APOOL | 139322 | apolipoprotein O like | 1 | 0 | 1.754 |
| CYB561 | 1534 | cytochrome b561 | 1 | 0 | 1.594 |
| GRHL2 | 79977 | grainyhead like transcription factor 2 | 1 | 0 | 1.526 |
| ESRP1 | 54845 | epithelial splicing regulatory protein 1 | 1 | 0 | 1.485 |
| EZR | 7430 | ezrin | 1 | 0 | 1.372 |
| RBBP8 | 5932 | RB binding protein 8, endonuclease | 0 | 1 | 3.095 |
| CIRBP | 1153 | cold inducible RNA binding protein | 0 | 1 | 3.083 |
| PTGER3 | 5733 | prostaglandin E receptor 3 | 0 | 1 | 2.802 |
| LAMA3 | 3909 | laminin subunit alpha 3 | 0 | 1 | 2.601 |
| OARD1 | 221443 | O-acyl-ADP-ribose deacylase 1 | 0 | 1 | 2.008 |
| ANKRD29 | 147463 | ankyrin repeat domain29 | 0 | 1 | 1.886 |
| EGR3 | 1960 | early growth response 3 | 0 | 1 | 1.836 |
| DIRAS3 | 9077 | DIRAS family GTPase 3 | 0 | 1 | 1.821 |
| MITD1 | 129531 | microtubule interacting and trafficking domain containing 1 | 0 | 1 | 1.425 |
| LAMB3 | 3914 | laminin subunit beta 3 | 0 | 1 | 1.366 |

mPS=Σ(Gene_Score x Gene_Weight)

[FIG. 11-2]

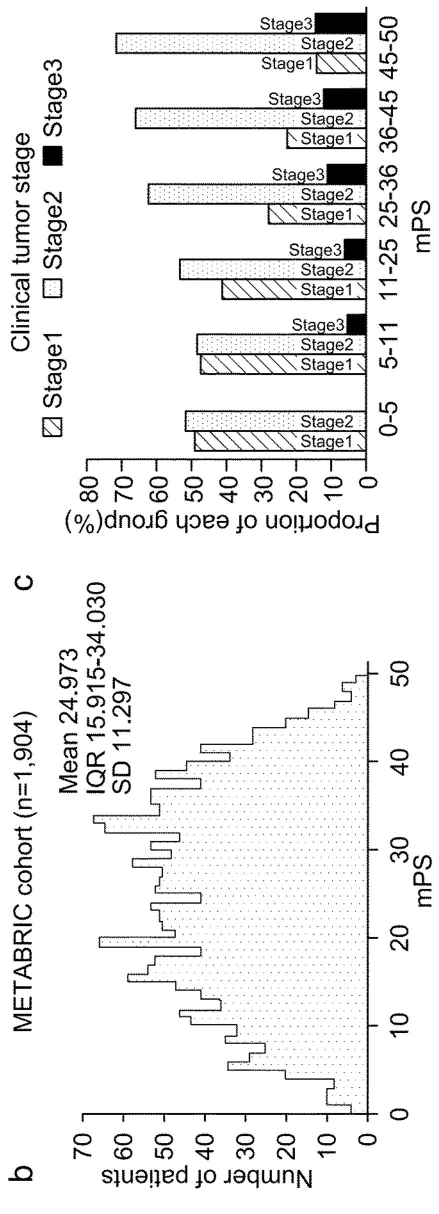
b
METABRIC cohort (n=1,904)
Mean 24.973
IQR 15.915-34.030
SD 11.297
Number of patients
mPS
c
Clinical tumor stage
Stage1
Stage2
Stage3
Proportion of each group(%)
0-5
5-11
11-25
25-36
36-45
45-50
mPS

[FIG. 11-3]

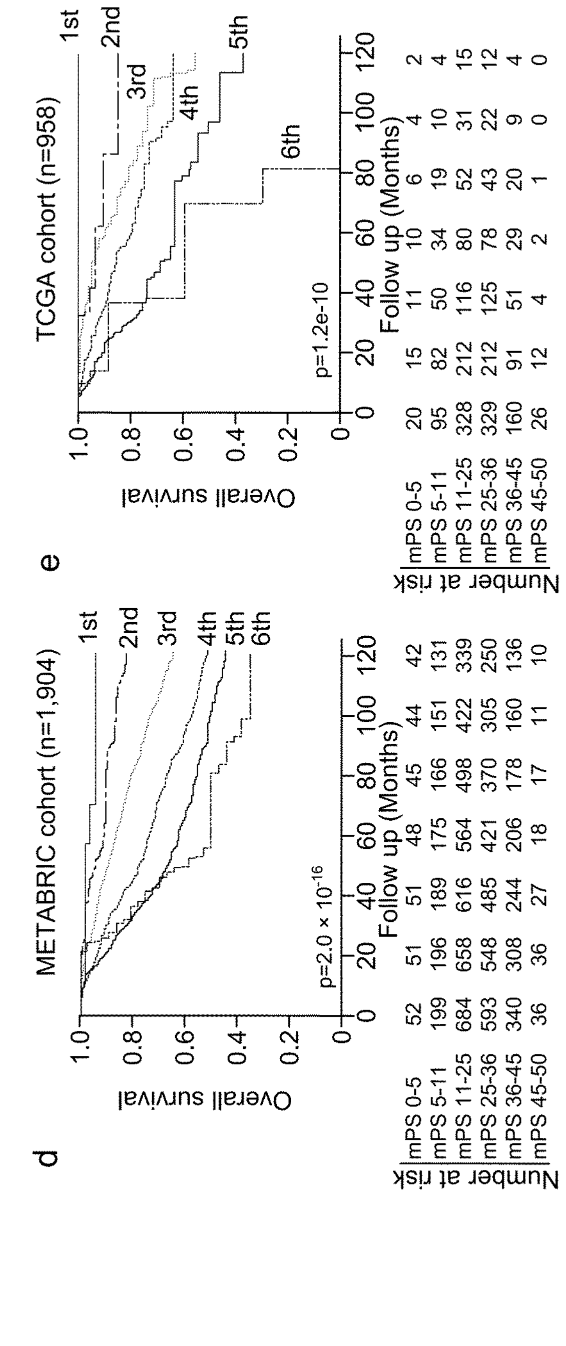
d
METABRIC cohort (n=1,904)
Overall survival
p=2.0 × 10^-16
Follow up (Months)
Number at risk
e
TCGA cohort (n=958)
p=1.2e-10

[FIG. 12]

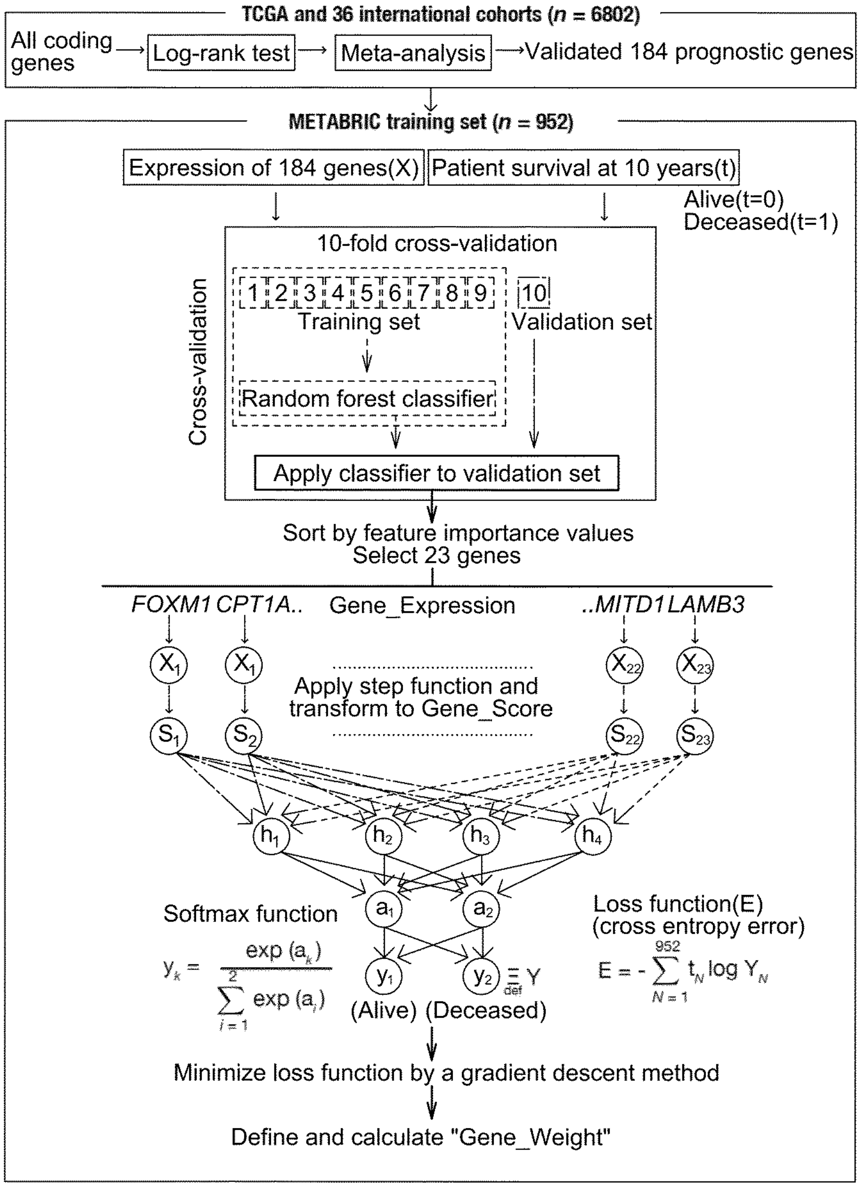
TCGA and 36 international cohorts (n = 6802)
All coding genes
Log-rank test
Meta-analysis
Validated 184 prognostic genes
METABRIC training set (n = 952)
Expression of 184 genes(X)
Patient survival at 10 years(t)
Alive(t=0)
Deceased(t=1)
10-fold cross-validation
Cross-validation
1 2 3 4 5 6 7 8 9
10
Training set
Validation set
Random forest classifier
Apply classifier to validation set
Sort by feature importance values
Select 23 genes
FOXM1 CPT1A..
Gene_Expression
..MITD1 LAMB3
Apply step function and transform to Gene_Score
Softmax function
Loss function(E)
(cross entropy error)
(Alive) (Deceased)
Minimize loss function by a gradient descent method
Define and calculate "Gene_Weight"

[FIG. 13-1]

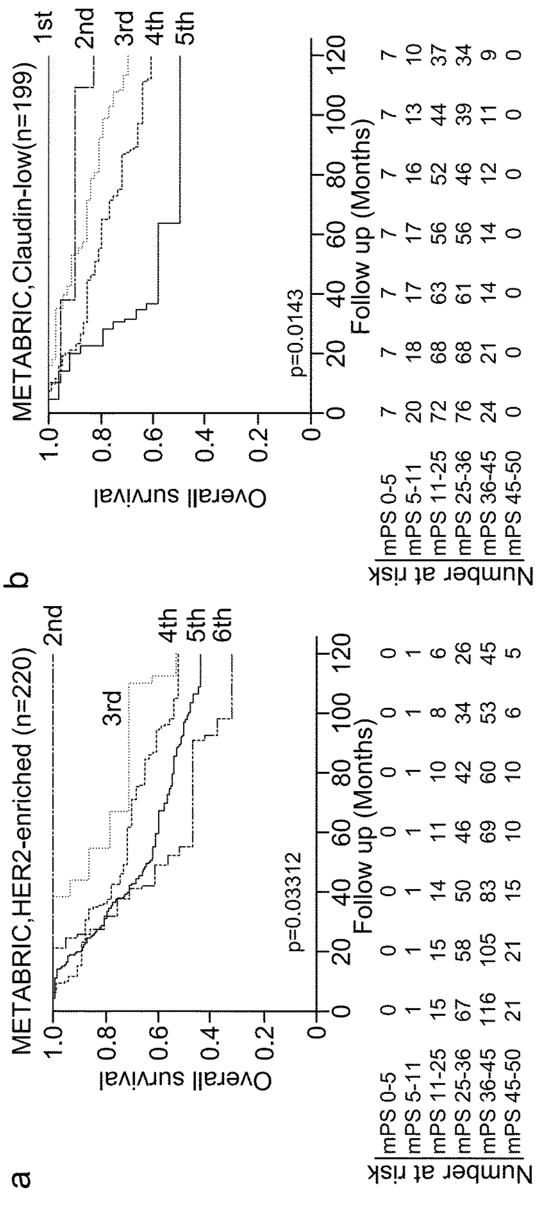
a
METABRIC,HER2-enriched (n=220)
p=0.03312
Overall survival
Follow up (Months)
2nd
3rd
4th
5th
6th
Number at risk
mPS 0-5 0 0 0 0 0 0 0
mPS 5-11 1 1 1 1 1 1 1
mPS 11-25 15 15 14 11 10 8 6
mPS 25-36 67 58 50 46 42 34 26
mPS 36-45 116 105 83 69 60 53 45
mPS 45-50 21 21 15 10 10 6 5
b
METABRIC,Claudin-low(n=199)
p=0.0143
Overall survival
Follow up (Months)
1st
2nd
3rd
4th
5th
Number at risk
mPS 0-5 7 7 7 7 7 7 7
mPS 5-11 20 18 17 17 16 13 10
mPS 11-25 72 68 63 56 52 44 37
mPS 25-36 76 68 61 56 46 39 34
mPS 36-45 24 21 14 14 12 11 9
mPS 45-50 0 0 0 0 0 0 0

[FIG. 13-2]

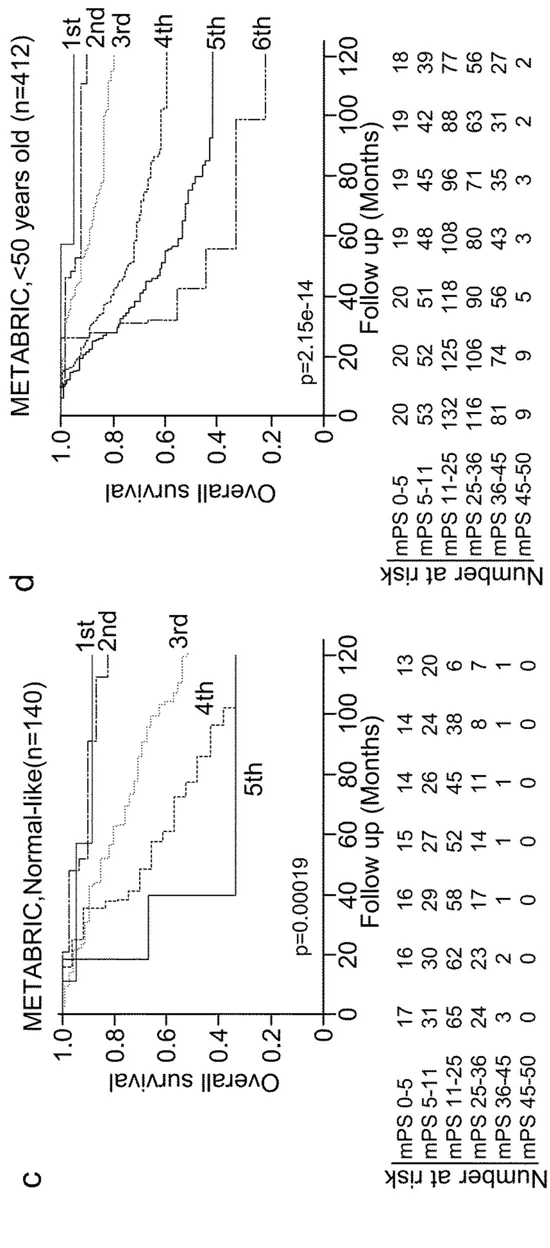
c
METABRIC,Normal-like(n=140)
Overall survival
1.0 0.8 0.6 0.4 0.2 0
p=0.00019
1st
2nd
3rd
4th
5th
0 20 40 60 80 100 120
Follow up (Months)
Number at risk
mPS 0-5 17 16 16 15 14 14 13
mPS 5-11 31 30 29 27 26 24 20
mPS 11-25 65 62 58 52 45 38 6
mPS 25-36 24 23 17 14 11 8 7
mPS 36-45 3 2 1 1 1 1 1
mPS 45-50 0 0 0 0 0 0 0
d
METABRIC,<50 years old (n=412)
Overall survival
1.0 0.8 0.6 0.4 0.2 0
p=2.15e-14
1st
2nd
3rd
4th
5th
6th
0 20 40 60 80 100 120
Follow up (Months)
Number at risk
mPS 0-5 20 20 20 19 19 19 18
mPS 5-11 53 52 51 48 45 42 39
mPS 11-25 132 125 118 108 96 88 77
mPS 25-36 116 106 90 80 71 63 56
mPS 36-45 81 74 56 43 35 31 27
mPS 45-50 9 9 5 3 3 2 2

[FIG. 13-3]

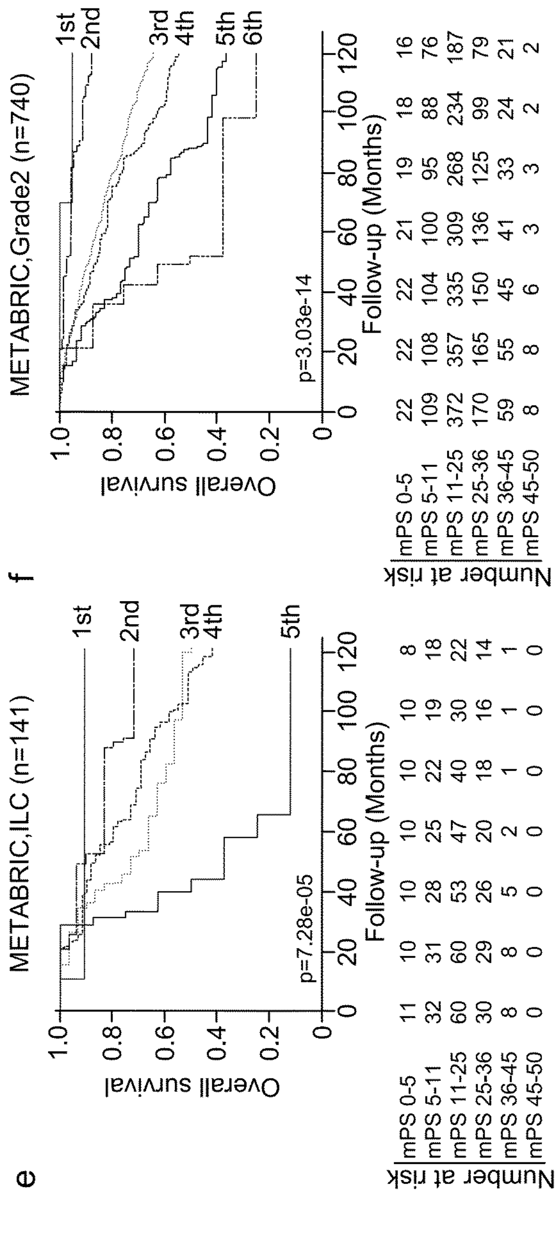
e
METABRIC,ILC (n=141)
p=7.28e-05
Overall survival
Follow-up (Months)
1st
2nd
3rd
4th
5th
Number at risk
mPS 0-5
mPS 5-11
mPS 11-25
mPS 25-36
mPS 36-45
mPS 45-50
f
METABRIC,Grade2 (n=740)
p=3.03e-14
6th

[FIG. 14]

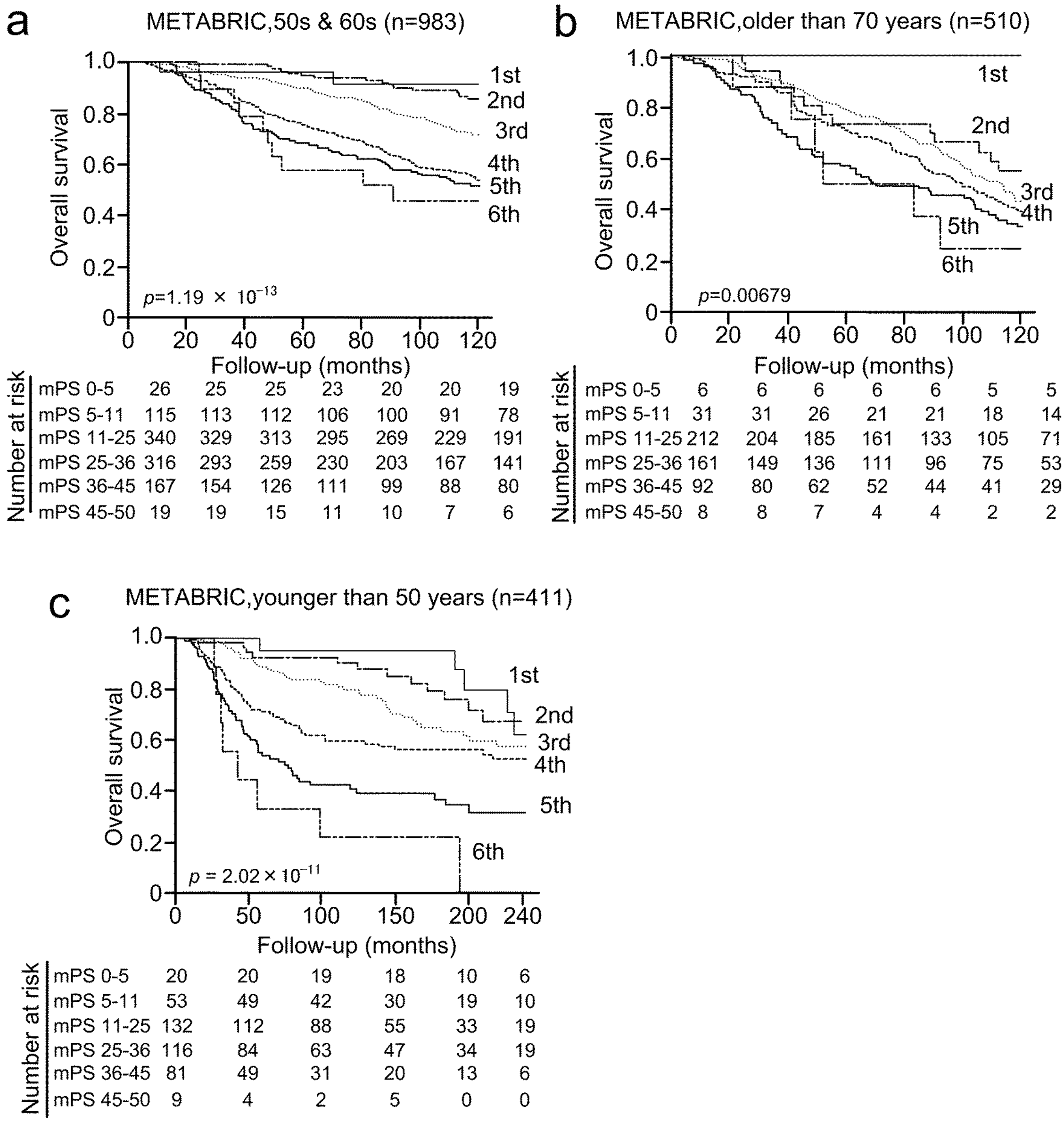
a
METABRIC,50s & 60s (n=983)
1st
2nd
3rd
4th
5th
6th
Overall survival
p=1.19 × 10^-13
Follow-up (months)
Number at risk
mPS 0-5 26 25 25 23 20 20 19
mPS 5-11 115 113 112 106 100 91 78
mPS 11-25 340 329 313 295 269 229 191
mPS 25-36 316 293 259 230 203 167 141
mPS 36-45 167 154 126 111 99 88 80
mPS 45-50 19 19 15 11 10 7 6
b
METABRIC,older than 70 years (n=510)
1st
2nd
3rd
4th
5th
6th
Overall survival
p=0.00679
Follow-up (months)
Number at risk
mPS 0-5 6 6 6 6 6 5 5
mPS 5-11 31 31 26 21 21 18 14
mPS 11-25 212 204 185 161 133 105 71
mPS 25-36 161 149 136 111 96 75 53
mPS 36-45 92 80 62 52 44 41 29
mPS 45-50 8 8 7 4 4 2 2
c
METABRIC,younger than 50 years (n=411)
1st
2nd
3rd
4th
5th
6th
Overall survival
p = 2.02 × 10^-11
Follow-up (months)
Number at risk
mPS 0-5 20 20 19 18 10 6
mPS 5-11 53 49 42 30 19 10
mPS 11-25 132 112 88 55 33 19
mPS 25-36 116 84 63 47 34 19
mPS 36-45 81 49 31 20 13 6
mPS 45-50 9 4 2 5 0 0

[FIG. 15-1]

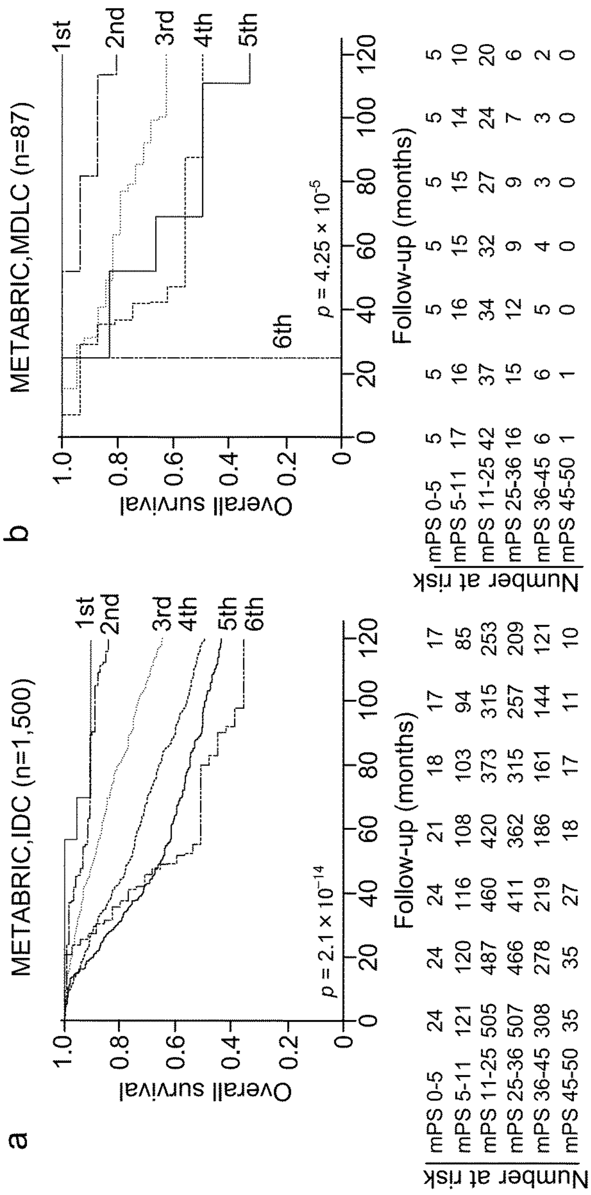
a
METABRIC,IDC (n=1,500)
Overall survival
1.0
0.8
0.6
0.4
0.2
0
0
20
40
60
80
100
120
Follow-up (months)
p = 2.1 × 10^-14
1st
2nd
3rd
4th
5th
6th
Number at risk
mPS 0-5 24 24 24 21 18 17 17
mPS 5-11 121 120 116 108 103 94 85
mPS 11-25 505 487 460 420 373 315 253
mPS 25-36 507 466 411 362 315 257 209
mPS 36-45 308 278 219 186 161 144 121
mPS 45-50 35 35 27 18 17 11 10
b
METABRIC,MDLC (n=87)
Overall survival
1.0
0.8
0.6
0.4
0.2
0
0
20
40
60
80
100
120
Follow-up (months)
p = 4.25 × 10^-5
1st
2nd
3rd
4th
5th
6th
Number at risk
mPS 0-5 5 5 5 5 5 5 5
mPS 5-11 17 16 16 15 15 14 10
mPS 11-25 42 37 34 32 27 24 20
mPS 25-36 16 15 12 9 9 7 6
mPS 36-45 6 6 5 4 3 3 2
mPS 45-50 1 1 0 0 0 0 0

[FIG. 15-2]

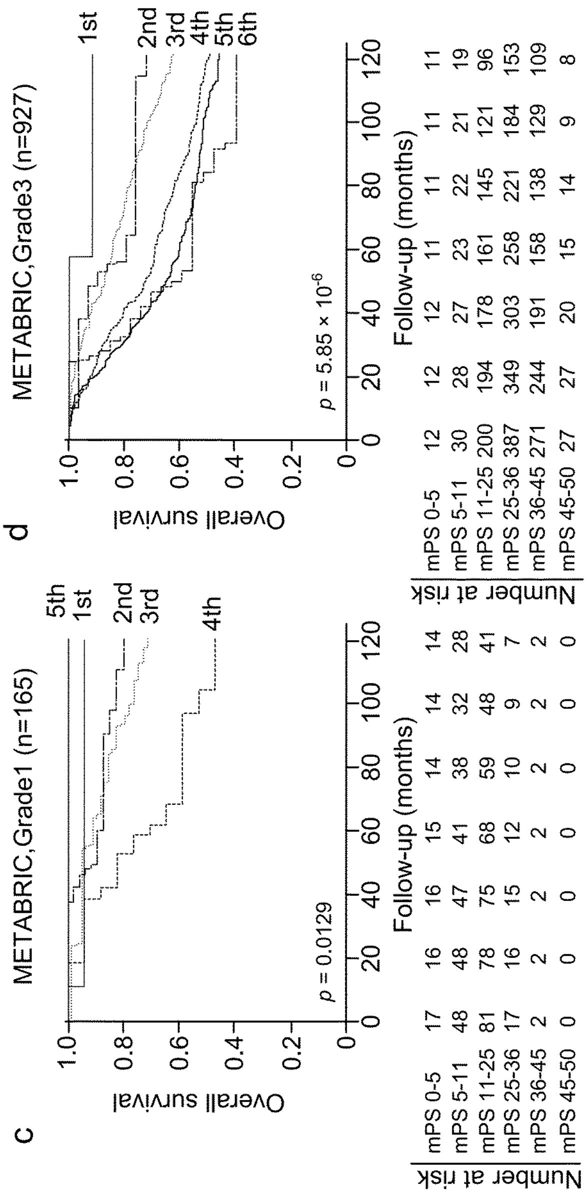
c
METABRIC,Grade1 (n=165)
Overall survival
1.0
0.8
0.6
0.4
0.2
0
p = 0.0129
5th
1st
2nd
3rd
4th
0
20
40
60
80
100
120
Follow-up (months)
Number at risk
mPS 0-5 17 16 16 15 14 14 14
mPS 5-11 48 48 47 41 38 32 28
mPS 11-25 81 78 75 68 59 48 41
mPS 25-36 17 16 15 12 10 9 7
mPS 36-45 2 2 2 2 2 2 2
mPS 45-50 0 0 0 0 0 0 0
d
METABRIC,Grade3 (n=927)
Overall survival
1.0
0.8
0.6
0.4
0.2
0
p = 5.85 × 10^-6
1st
2nd
3rd
4th
5th
6th
0
20
40
60
80
100
120
Follow-up (months)
Number at risk
mPS 0-5 12 12 12 11 11 11 11
mPS 5-11 30 28 27 23 22 21 19
mPS 11-25 200 194 178 161 145 121 96
mPS 25-36 387 349 303 258 221 184 153
mPS 36-45 271 244 191 158 138 129 109
mPS 45-50 27 27 20 15 14 9 8

[FIG. 16-1]

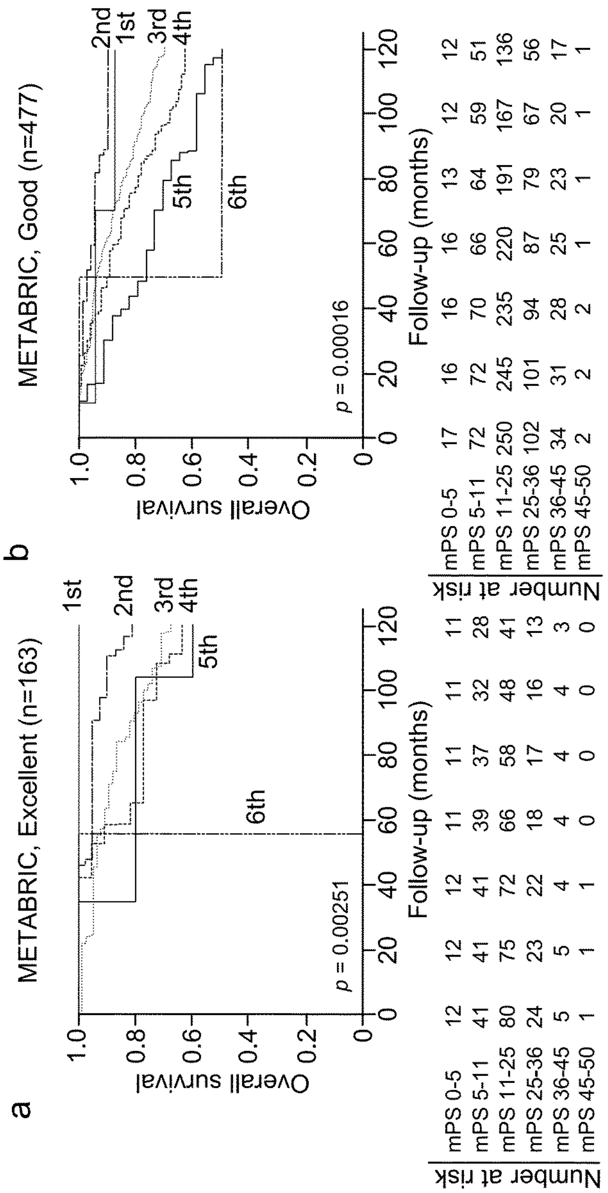
a
METABRIC, Excellent (n=163)
Overall survival
Follow-up (months)
p = 0.00251
1st
2nd
3rd
4th
5th
6th
Number at risk
mPS 0-5 12 12 12 11 11 11 11
mPS 5-11 41 41 41 39 37 32 28
mPS 11-25 80 75 72 66 58 48 41
mPS 25-36 24 23 22 18 17 16 13
mPS 36-45 5 5 4 4 4 4 3
mPS 45-50 1 1 1 0 0 0 0
b
METABRIC, Good (n=477)
Overall survival
Follow-up (months)
p = 0.00016
1st
2nd
3rd
4th
5th
6th
Number at risk
mPS 0-5 17 16 16 16 13 12 12
mPS 5-11 72 72 70 66 64 59 51
mPS 11-25 250 245 235 220 191 167 136
mPS 25-36 102 101 94 87 79 67 56
mPS 36-45 34 31 28 25 23 20 17
mPS 45-50 2 2 2 1 1 1 1

[FIG. 16-2]

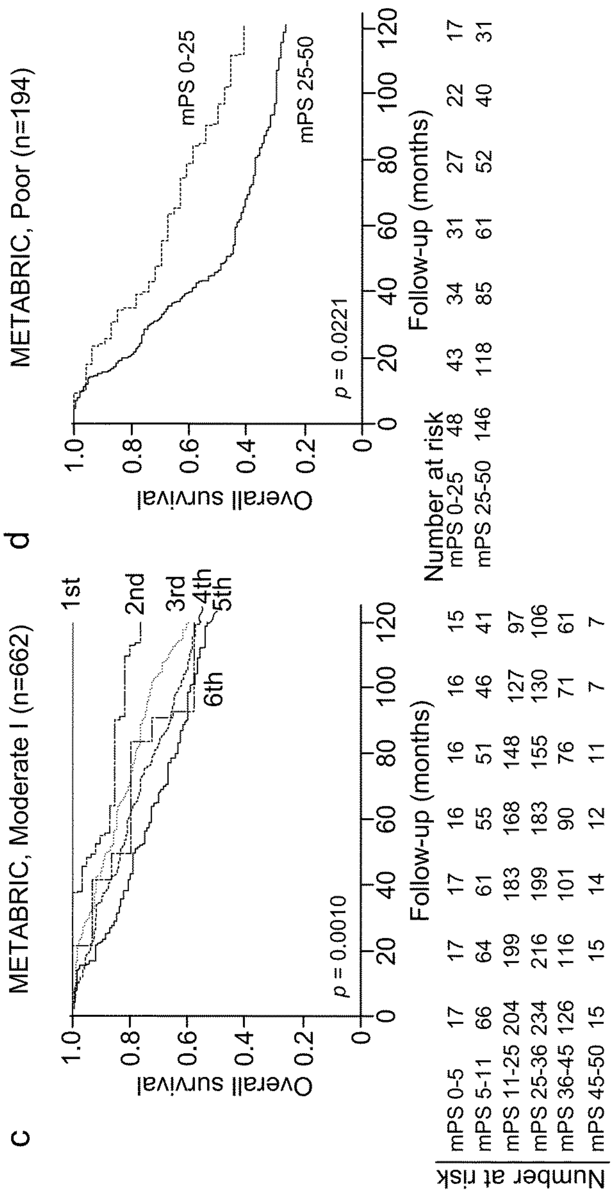
c
METABRIC, Moderate I (n=662)
Overall survival
1.0
0.8
0.6
0.4
0.2
0
p = 0.0010
1st
2nd
3rd
4th
5th
6th
0
20
40
60
80
100
120
Follow-up (months)
Number at risk
mPS 0-5 17 17 17 16 16 16 15
mPS 5-11 66 64 61 55 51 46 41
mPS 11-25 204 199 183 168 148 127 97
mPS 25-36 234 216 199 183 155 130 106
mPS 36-45 126 116 101 90 76 71 61
mPS 45-50 15 15 14 12 11 7 7
d
METABRIC, Poor (n=194)
Overall survival
1.0
0.8
0.6
0.4
0.2
0
p = 0.0221
mPS 0-25
mPS 25-50
0
20
40
60
80
100
120
Follow-up (months)
Number at risk
mPS 0-25 48 43 34 31 27 22 17
mPS 25-50 146 118 85 61 52 40 31

[FIG. 17-1]

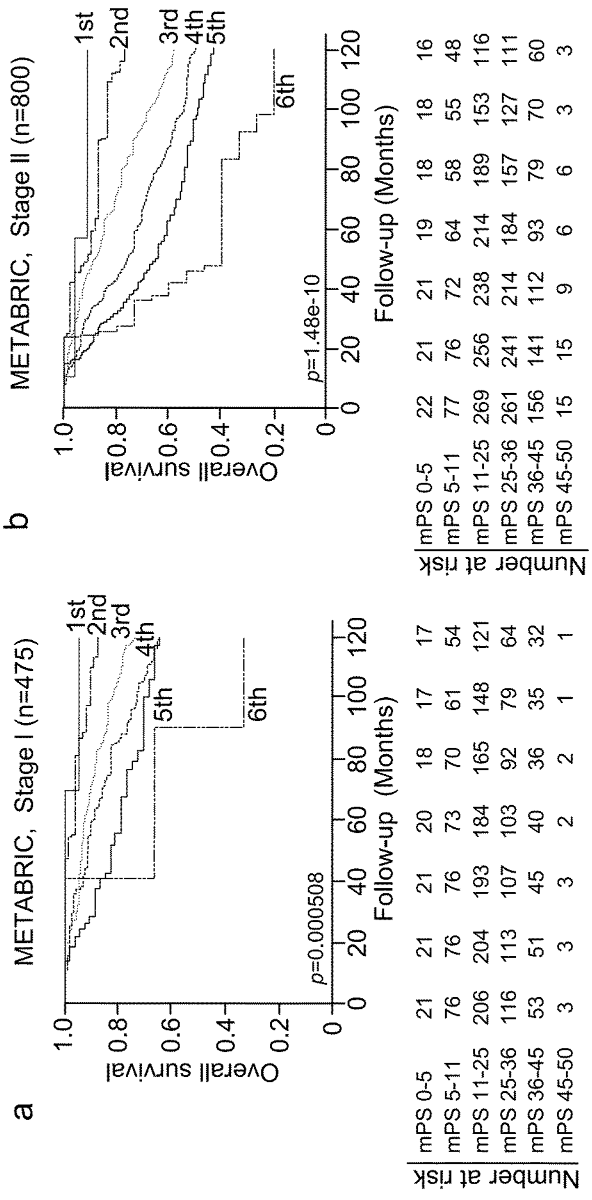
a
METABRIC, Stage I (n=475)
Overall survival
1.0
0.8
0.6
0.4
0.2
0
0
20
40
60
80
100
120
Follow-up (Months)
p=0.000508
1st
2nd
3rd
4th
5th
6th
Number at risk
mPS 0-5 21 21 21 20 18 17 17
mPS 5-11 76 76 76 73 70 61 54
mPS 11-25 206 204 193 184 165 148 121
mPS 25-36 116 113 107 103 92 79 64
mPS 36-45 53 51 45 40 36 35 32
mPS 45-50 3 3 3 2 2 1 1
b
METABRIC, Stage II (n=800)
Overall survival
1.0
0.8
0.6
0.4
0.2
0
0
20
40
60
80
100
120
Follow-up (Months)
p=1.48e-10
1st
2nd
3rd
4th
5th
6th
Number at risk
mPS 0-5 22 21 21 19 18 18 16
mPS 5-11 77 76 72 64 58 55 48
mPS 11-25 269 256 238 214 189 153 116
mPS 25-36 261 241 214 184 157 127 111
mPS 36-45 156 141 112 93 79 70 60
mPS 45-50 15 15 9 6 6 3 3

[FIG. 17-2]

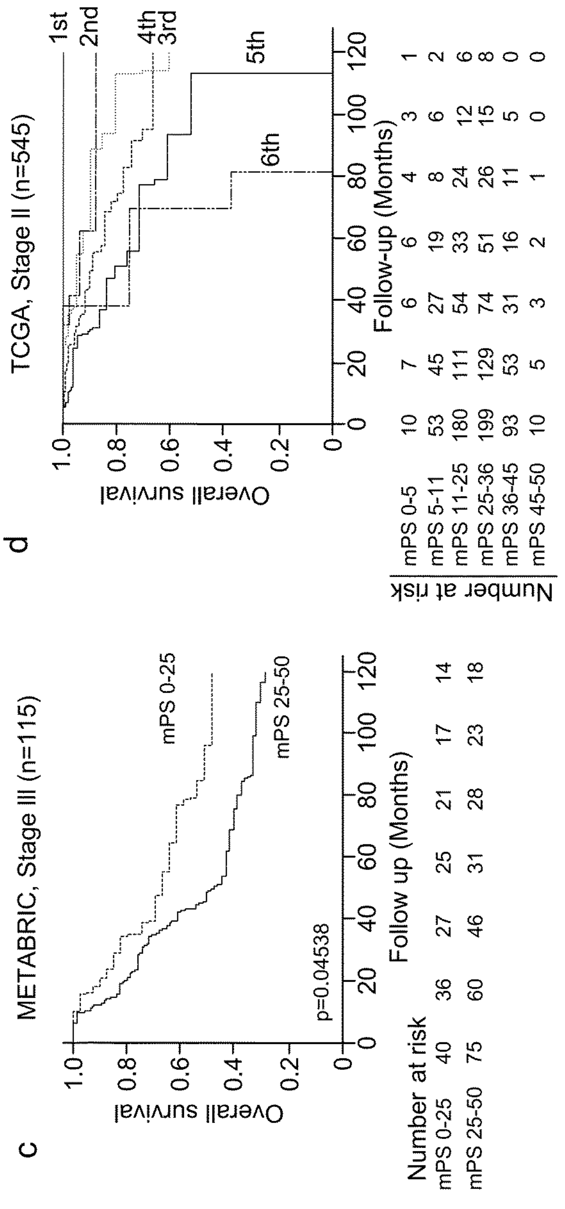
c
METABRIC, Stage III (n=115)
Overall survival
p=0.04538
mPS 0-25
mPS 25-50
Follow up (Months)
Number at risk
mPS 0-25 40 36 27 25 21 17 14
mPS 25-50 75 60 46 31 28 23 18
d
TCGA, Stage II (n=545)
Overall survival
1st
2nd
3rd
4th
5th
6th
Follow-up (Months)
Number at risk
mPS 0-5 10 7 6 6 4 3 1
mPS 5-11 53 45 27 19 8 6 2
mPS 11-25 180 111 54 33 24 12 6
mPS 25-36 199 129 74 51 26 15 8
mPS 36-45 93 53 31 16 11 5 0
mPS 45-50 10 5 3 2 1 0 0

[FIG. 17-3]

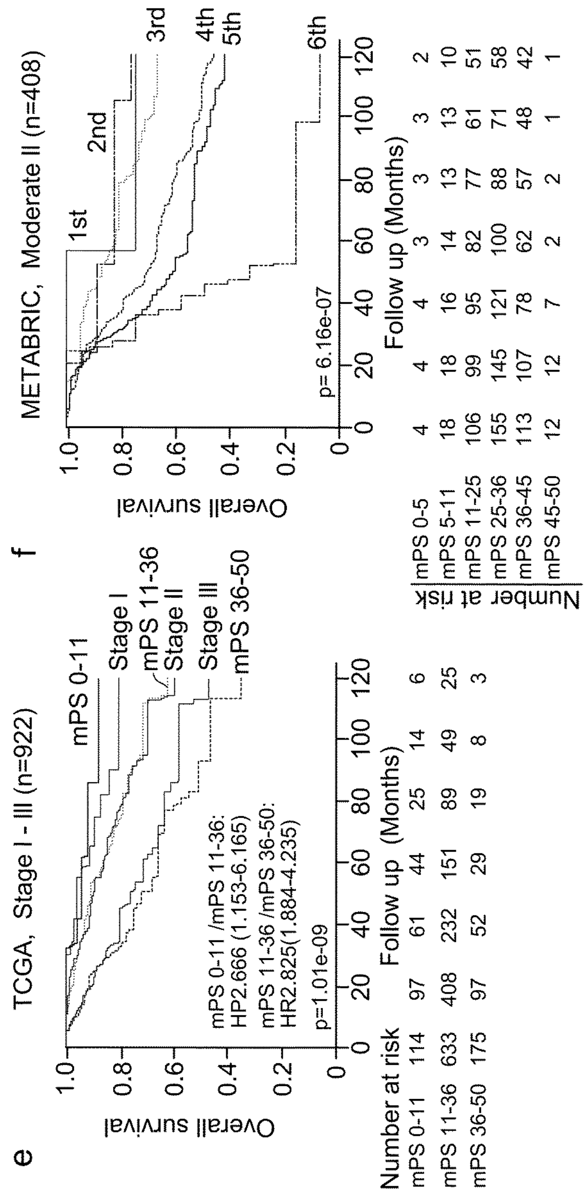
e
TCGA, Stage I - III (n=922)
Overall survival
Follow up (Months)
mPS 0-11
Stage I
mPS 11-36
Stage II
Stage III
mPS 36-50
mPS 0-11 /mPS 11-36:
HP2.666 (1.153-6.165)
mPS 11-36 /mPS 36-50:
HR2.825(1.884-4.235)
p=1.01e-09
Number at risk
mPS 0-11 114 97 61 44 25 14 6
mPS 11-36 633 408 232 151 89 49 25
mPS 36-50 175 97 52 29 19 8 3
f
METABRIC, Moderate II (n=408)
Overall survival
Follow up (Months)
1st
2nd
3rd
4th
5th
6th
p= 6.16e-07
Number at risk
mPS 0-5 4 4 4 3 3 3 2
mPS 5-11 18 18 16 14 13 13 10
mPS 11-25 106 99 95 82 77 61 51
mPS 25-36 155 145 121 100 88 71 58
mPS 36-45 113 107 78 62 57 48 42
mPS 45-50 12 12 7 2 2 1 1

[FIG. 18]

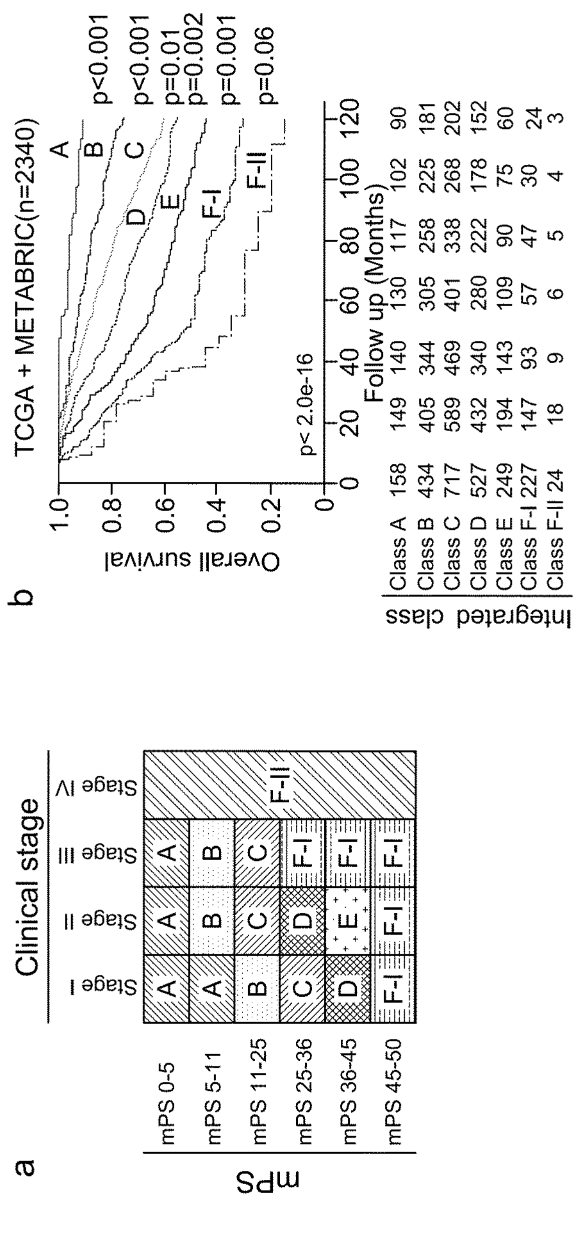
a
Clinical stage
Stage I
Stage II
Stage III
Stage IV
mPS
mPS 0-5
mPS 5-11
mPS 11-25
mPS 25-36
mPS 36-45
mPS 45-50
A
B
C
D
E
F-I
F-II
b
TCGA + METABRIC(n=2340)
Overall survival
1.0
0.8
0.6
0.4
0.2
0
p< 2.0e-16
p<0.001
p<0.001
p=0.01
p=0.002
p=0.001
p=0.06
0
20
40
60
80
100
120
Follow up (Months)
Integrated class
Class A 158 149 140 130 117 102 90
Class B 434 405 344 305 258 225 181
Class C 717 589 469 401 338 268 202
Class D 527 432 340 280 222 178 152
Class E 249 194 143 109 90 75 60
Class F-I 227 147 93 57 47 30 24
Class F-II 24 18 9 6 5 4 3

## INTERNATIONAL SEARCH REPORT

International application No.
PCT/JP2019/033085

A. CLASSIFICATION OF SUBJECT MATTER
Int.Cl. C12Q1/68(2018.01)i, G01N33/50(2006.01)i, G01N33/68(2006.01)i
According to International Patent Classification (IPC) or to both national classification and IPC

B. FIELDS SEARCHED
Minimum documentation searched (classification system followed by classification symbols)
Int.Cl. C12Q1/68, G01N33/50, G01N33/68

Documentation searched other than minimum documentation to the extent that such documents are included in the fields searched

| | |
|---|---|
| Published examined utility model applications of Japan | 1922-1996 |
| Published unexamined utility model applications of Japan | 1971-2019 |
| Registered utility model specifications of Japan | 1996-2019 |
| Published registered utility model applications of Japan | 1994-2019 |

Electronic data base consulted during the international search (name of data base and, where practicable, search terms used)
CAplus/MEDLINE/EMBASE/BIOSIS/WPIDS (STN), JSTPlus/JMEDPlus/JST7580 (JDreamIII)

C. DOCUMENTS CONSIDERED TO BE RELEVANT

| Category* | Citation of document, with indication, where appropriate, of the relevant passages | Relevant to claim No. |
|---|---|---|
| Y | PAIK, S. et al., "A multigene assay to predict recurrence of tamoxifen-treated, node-negative breast cancer", N. Engl. J. Med., 2004, vol. 351, no. 27, pp. 2817-2826, abstract, page 2818, left column, line 30 to right column, line 6, page 2819, left column, lines 9-22 | 1-24, 29-32 |
| Y | JP 2014-532428 A (GENOMIC HEALTH, INC.) 08 December 2014, claim 1, paragraphs [0133], [0134], example 2 & US 2014/0296085 A1, claim 1, paragraphs [0132], [0133], example 2 | 1-24, 29-32 |

☒ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 12 November 2019 (12.11.2019) | 19 November 2019 (19.11.2019) |

| Name and mailing address of the ISA/ | Authorized officer |
|---|---|
| Japan Patent Office<br>3-4-3, Kasumigaseki, Chiyoda-ku,<br>Tokyo 100-8915, Japan | Telephone No. |

Form PCT/ISA/210 (second sheet) (January 2015)

**INTERNATIONAL SEARCH REPORT**

International application No. PCT/JP2019/033085

C (Continuation). DOCUMENTS CONSIDERED TO BE RELEVANT

| Category* | Citation of document, with indication, where appropriate, of the relevant passages | Relevant to claim No. |
|---|---|---|
| Y | JP 2018-520366 A (MINOMIC INTERNATIONAL LTD.) 26 July 2018, claim 9 & WO 2017/011855 A1, claim 9 | 1-24, 29-32 |
| Y | JP 2018-072349 A (NESTEC S.A.) 10 May 2018, paragraphs [0239]-[0242] & US 2014/0141983 A1, paragraphs [0395]-[0398] | 1-24, 29-32 |
| Y | JP 2018-500276 A (DANA-FARBER CANCER INSTITUTE, INC.) 11 January 2018, paragraph [0252] & WO 2016/057651 A1, page 83, lines 11-33 | 1-24, 29-32 |
| Y | VAN'T VEER, L. J. et al., "Gene expression profiling predicts clinical outcome of breast cancer", Nature, 2002, vol. 415, pp. 530-536, page 530, right column, lines 20-23 | 1-24, 29-32 |
| Y | SORLIE, T. et al., "Gene expression patterns of breast carcinomas distinguish tumor subclasses with clinical implications", Proc. Natl. Acad. Sci. U. S. A., 2001, vol. 98, no. 19, pp. 10869-10874, page 10870, left column "Statistical Analysis" | 1-24, 29-32 |

Form PCT/ISA/210 (continuation of second sheet) (January 2015)

**INTERNATIONAL SEARCH REPORT**

International application No.
PCT/JP2019/033085

**Box No. II Observations where certain claims were found unsearchable (Continuation of item 2 of first sheet)**

This international search report has not been established in respect of certain claims under Article 17(2)(a) for the following reasons:

1. ☐ Claims Nos.:
because they relate to subject matter not required to be searched by this Authority, namely:

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

**Box No. III Observations where unity of invention is lacking (Continuation of item 3 of first sheet)**

This International Searching Authority found multiple inventions in this international application, as follows:
See extra sheet

1. ☐ As all required additional search fees were timely paid by the applicant, this international search report covers all searchable claims.
2. ☐ As all searchable claims could be searched without effort justifying additional fees, this Authority did not invite payment of additional fees.
3. ☐ As only some of the required additional search fees were timely paid by the applicant, this international search report covers only those claims for which fees were paid, specifically claims Nos.:

4. ☒ No required additional search fees were timely paid by the applicant. Consequently, this international search report is restricted to the invention first mentioned in the claims; it is covered by claims Nos.: 1-24, 29-32

**Remark on Protest**

☐ The additional search fees were accompanied by the applicant's protest and, where applicable, the payment of a protest fee.

☐ The additional search fees were accompanied by the applicant's protest but the applicable protest fee was not paid within the time limit specified in the invitation.

☐ No protest accompanied the payment of additional search fees.

Form PCT/ISA/210 (continuation of first sheet (2)) (January 2015)

**INTERNATIONAL SEARCH REPORT**

International application No.
PCT/JP2019/033085

<Continuation of Box No. III>

Claims are classified into the following 415 inventions.

(Invention 1) Claims 1-24 and 29-32

Claims 1-24 and 29-32 have the special technical feature of a "method for producing a discriminator comprising: step 1 for determining, by a statistic method, whether or not each biomarker is altered in regard to an event occurring in a body of a subject to be tested, on the basis of the measurements of each biomarker, with respect to a population of the biomarker derived from the subject to be tested, and extracting a biomarker group, which is determined to be altered, as a first subpopulation; step 2 for verifying each biomarker belonging to a first subpopulation and extracting a biomarker group, which is statistically predicted that the biomarker group has closer relation to the event occurring in the body, as a second subpopulation; and step 3 of calculating the weight of each biomarker belonging to the second subpopulation by a deep learning method, wherein the discriminator uses scores obtained from the measurements of each biomarker belonging to the second subpopulation and a weight of each biomarker calculated in step 3 to calculate a weighted sum of the scores of the biomarker belonging to the second subpopulation," and thus are classified as invention 1.

(Inventions 2-415) Claims 25-28

Claims 25-28 has substantial options which are composed of other genes, and are classified as inventions 2-415 on the basis of each option.

In addition, each invention in claims 25-28 shares the common technical feature of "relating to a biomarker" with the invention in claim 1.

However, said technical feature does not make a contribution over the prior art in light of the disclosure of any one among documents 1-5, and thus cannot be considered a special technical feature. Also, there are no other identical or corresponding special technical features between each invention in claims 25-28 and claim 1.

Moreover, each invention in claims 25-28 is not dependent on claim 1, and are not substantially identical or equivalent to any of the claims classified as invention 1.

Therefore, each invention in claims 25-28 cannot be classified as invention 1.

Form PCT/ISA/210 (extra sheet) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 5725274 B


### Non-patent literature cited in the description


- **SPARANO, J. A. et al.** Prospective Validation of a 21-Gene Expression Assay in Breast Cancer. *N. Engl. J. Med.,* 2015, vol. 373, 2005-2014
- **VAN 'T VEER, L. J. et al.** *Nature,* 2002, vol. 415, 530-536
- **PARKER, J. S. et al.** *J. Clin. Oncol.,* 2009, vol. 27, 1160-1167
- **MA, X. J. et al.** *Cancer Cell,* 2004, vol. 5, 607-616
- **FILIPITS, M. et al.** *Clin. Cancer Res.,* 2011, vol. 17, 6012-6020
- **CERAMI, E. et al.** *Cancer Discov.,* 2012, vol. 2, 401-404
- **ABDEL-FATAH, T. M. A. et al.** *The Lancet. Oncology,* 2016, vol. 17, 1004-1018
- **PEREIRA, B. et al.** *Nature communications,* 2016, vol. 7, 11479
- **CURTIS, C. et al.** *Nature,* 2012, vol. 486, 346-352
- **KOUROU, K. et al.** *Computational and structural biotechnology journal,* 2015, vol. 13, 8-17
- **MERMEL, C. H. et al.** *Genome Biol.,* 2011, vol. 12, R41